(19) Europäisches Patentamt
European Patent Office
Office européen des brevets

(11) Veröffentlichungsnummer: **0 378 850 B1**

(12) # EUROPÄISCHE PATENTSCHRIFT

(45) Veröffentlichungstag der Patentschrift: **06.04.94**

(51) Int. Cl.$^5$: **C07D 471/04**, A61K 31/505, //(C07D471/04,239:00,221:00)

(21) Anmeldenummer: **89123894.1**

(22) Anmeldetag: **23.12.89**

(54) **Neue substituierte Pyrido(2,3-d)pyrimidine.**

(30) Priorität: **07.01.89 DE 3900363**
**18.07.89 IT 2121589**

(43) Veröffentlichungstag der Anmeldung:
**25.07.90 Patentblatt 90/30**

(45) Bekanntmachung des Hinweises auf die Patenterteilung:
**06.04.94 Patentblatt 94/14**

(84) Benannte Vertragsstaaten:
**AT BE CH DE ES FR GB GR IT LI LU NL SE**

(56) Entgegenhaltungen:
**US-A- 4 761 419**

(73) Patentinhaber: **BAYER AG**

**D-51368 Leverkusen(DE)**

(72) Erfinder: **Hübsch, Walter, Dr.**
**Am Eckbusch 43/50**
**D-5600 Wuppertal 1(DE)**
Erfinder: **Angerbauer, Rolf, Dr.**
**Moospfad 20**
**D-5600 Wuppertal 1(DE)**
Erfinder: **Fey, Peter, Dr.**
**Ursulastrasse 14**
**D-5600 Wuppertal 1(DE)**
Erfinder: **Philipps, Thomas, Dr.**
**Silesuisstrasse 74**
**D-5000 Köln 80(DE)**
Erfinder: **Bischoff, Hilmar, Dr.**
**Am Rohm 78**
**D-5600 Wuppertal 1(DE)**
Erfinder: **Petzinna, Dieter, Dr.**
**Peter-Berten-Strasse 10**
**D-4000 Düsseldorf 12(DE)**
Erfinder: **Schmidt, Delf, Dr.**
**Am Eckbusch 55b**
**D-5600 Wuppertal 1(DE)**
Erfinder: **Thomas, Günter, Dr.**
**Via Campogallo 21/14**
**I-20020 Arese (Milano)(IT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 378 850 B1**

## Beschreibung

Die Erfindung betrifft neue substituierte Pyrido(2,3-d)pyrimidine, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl-Coenzym A Reduktase (HMG-CoA-Reduktase) sind [Mevinolin, EP-A 22 478; US 4 231 938]. Darüberhinaus sind auch bestimmte Indolderivate bzw. Pyrazolderivate Inhibitoren der HMG-CoA-Reduktase [EP-A 1 114 027; US-Patent 4 613 610].

Es wurden nun neue substituierte Pyrido(2,3-d)pyrimidine der allgemeinen Formel (I),

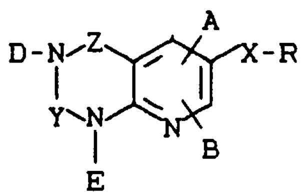

(I)

in welcher

| | |
|---|---|
| A | - für einen 5- bis 7-gliedrigen Heterocyclus steht, der bis zu 4 Heteroatome aus der Reihe Schwefel, Sauerstoff oder Stickstoff enthalten kann und der gegebenenfalls bis zu 3-fach gleich oder verschieden durch Halogen, Hydroxy, Trifluormethyl, geradkettiges oder verzweigtes Alkyl, Alkoxy oder Alkoxycarbonyl mit jeweils bis zu 8 Kohlenstoffatomen, Aryl mit 6 bis 10 Kohlenstoffatomen oder durch eine Gruppe der Formel $-NR^1R^2$ substituiert ist, |
| | worin |
| $R^1$ und $R^2$ | gleich oder verschieden sind und |
| | - Wasserstoff, Aryl oder Arylsulfonyl mit 6 bis 10 Kohlenstoffatomen, geradkettiges oder verzweigtes Alkyl oder Alkylsulfonyl mit bis zu 8 Kohlenstoffatomen bedeuten, wobei die letztgenannten Reste gegebenenfalls durch Aryl mit 6 bis 10 Kohlenstoffatamen substituiert sind, oder |
| | - eine Gruppe der Formel $-COR^3$ bedeuten, |
| | worin |
| $R^3$ | - geradkettiges oder verzweigtes Alkyl oder Alkoxy mit bis zu 8 Kohlenstoffatomen oder Phenyl bedeutet, |
| | - für Aryl mit 6 bis 10 Kohlenstoffatomen steht, das gegebenenfalls bis zu 5-fach gleich oder verschieden substituiert ist durch geradkettiges oder verzweigtes Alkyl, Alkylthio, Alkylsulfonyl, Alkoxy oder Alkoxycarbonyl mit jeweils bis zu 10 Kohlenstoffatomen, welche ihrerseits durch Trifluormethyl, Hydroxy, Alkoxy mit bis zu 6 Kohlenstoffatomen, Phenyl oder Phenoxy substituiert sein können, |
| | oder durch Aryl, Aryloxy, Arylthio oder Arylsulfonyl mit 6 bis 10 Kohlenstoffatomen, oder durch Halogen, Nitro, Cyano, Trifluormethyl, Benzyloxy oder eine Gruppe der Formel $-NR^1R^2$ substituiert ist, |
| | oder |
| | - für ein geradkettiges oder verzweigtes Alkyl mit jeweils bis zu 8 Kohlenstoffatomen steht, |
| | worin |
| $R^1$ und $R^2$ | die oben angegebene Bedeutung haben, |
| B | - für Cycloalkyl mit 3 bis 8 Kohlenstoffatomen steht, |
| | - für Trifluormethyl oder geradkettiges oder verzweigtes Alkyl mit bis zu 12 Kohlenstoffatomen steht, das gegebenenfalls durch Halogen, Hydroxy, Cyano, Azido, Trifluormethyl, Alkylthio, Alkylsulfonyl oder Alkoxy mit jeweils bis zu 8 Kohlenstoffatomen oder durch Aryl, Aryloxy oder Arylthio mit 6 bis 10 Kohlenstoffatomen substituiert ist, wobei die Arylreste gegebenenfalls bis zu 3-fach gleich oder verschieden durch Halogen, Cyano, Trifluormethyl, Trifluormethoxy, geradkettiges oder verzweigtes Alkyl, Alkoxy, Alkylthio oder Alkylsulfonyl mit jeweils bis zu 8 Kohlenstoffatomen substituiert sein können, oder durch eine Gruppe der Formel $-NR^1R^2$ oder $-COR^3$ substituiert ist, |

worin

$R^1$, $R^2$ und $R^3$ die oben angegebene Bedeutung haben,
- für Aryl mit 6 bis 10 Kohlenstoffatomen steht, das gegebenenfalls durch Halogen, Cyano, Nitro, Trifluormethyl, geradkettiges oder verzweigtes Alkyl, Alkoxy oder Alkoxycarbonyl mit jeweils bis zu 8 Kohlenstoffatomen oder Amino substituiert ist,

D - für Wasserstoff, Hydroxy oder
- Cycloalkyl mit 3 bis 8 Kohlenstoffatomen steht,
- für geradkettiges oder verzweigtes Alkyl mit jeweils bis zu 12 Kohlenstoffatomen steht, das gegebenenfalls durch Halogen, Hydroxy oder Alkoxy mit bis zu 8 Kohlenstoffatomen substituiert ist,

E - die oben angegebene Bedeutung von A hat und mit dieser gleich oder verschieden ist, oder
- für Wasserstoff oder
- Cycloalkyl mit 3 bis 8 Kohlenstoffatomen steht,
- für geradkettiges oder verzweigtes Alkyl mit bis 12 Kohlenstoffatomen steht, das gegebenenfalls durch Halogen, geradkettiges oder verzweigtes Alkenyl mit bis zu 8 Kohlenstoffatomen, Aryl mit 6 bis 10 Kohlenstoffatomen, durch einen 5- bis 7-gliedrigen Heterocyclus mit bis zu 4 Heteroatomen aus der Reihe Stickstoff, Sauerstoff oder Schwefel oder durch eine Gruppe der Formel $-NR^1R^2$, $-OR^4$, $-COR^5$ oder $S(O)_n-R^6$ substituiert ist,

worin

$R^1$ und $R^2$ die oben angegebene Bedeutung haben,

$R^4$ - Wasserstoff oder
- geradkettiges oder verzweigtes Alkyl mit bis zu 10 Kohlenstoffatomen bedeutet, das gegebenenfalls durch Hydroxy, Trialkylsilyl mit bis zu 10 Kohlenstoffatomen im Alkylteil, Halogen oder Aryl mit 6 bis 10 Kohlenstoffatomen substituiert ist,
- Trialkylsilyl mit bis zu 10 Kohlenstoffatomen oder Cycloalkyl mit 3 bis 8 Kohlenstoffatomen oder Aryl mit 6 bis 10 Kohlenstoffatomen bedeutet, welches seinerseits durch Halogen, Cyano, Nitro oder Amino substituiert sein kann, oder
- eine Gruppe der Formel $-COR^7$ bedeutet,

worin

$R^7$ - geradkettiges oder verzweigtes Alkyl mit bis zu 8 Kohlenstoffatomen, Aryl mit 6 bis 10 Kohlenstoffatomen oder die Gruppe $-NR^1R^2$ bedeutet,

worin

$R^1$ und $R^2$ die oben angegebene Bedeutung haben,

$R^5$ - Wasserstoff oder geradkettiges oder verzweigtes Alkyl mit bis zu 8 Kohlenstoffatomen bedeutet, das gegebenenfalls durch Hydroxy, Phenyl, Halogen oder Cyano substituiert ist,
- Aryl mit 6 bis 10 Kohlenstoffatomen bedeutet, welches durch Halogen, Amino, Hydroxy, Nitro oder Cyano substituiert sein kann, oder
- eine Gruppe der Formel $-NR^1R^2$ oder $-OR^4$ bedeutet,

worin

$R^1$, $R^2$ und $R^4$ die oben angegebene Bedeutung haben,

n - eine Zahl 0 oder 2 bedeutet,

$R^6$ - geradkettiges oder verzweigtes Alkyl mit bis zu 10 Kohlenstoffatomen bedeutet, das durch Halogen, Hydroxy, Phenyl oder eine Gruppe der Formel $-NR^1R^2$ substituiert sein kann,

worin

$R^1$ und $R^2$ die oben angegebene Bedeutung haben,
- Aryl mit 6 bis 10 Kohlenstoffatomen bedeutet, das durch Halogen, Hydroxy, Cyano, Nitro oder Amino substituiert sein kann, oder
- eine Gruppe der Formel $-NR^1R^2$ bedeutet, wenn n für die Zahl 2 steht,

worin

$R^1$ und $R^2$ die oben angegebene Bedeutung haben,

oder

E - für eine Gruppe der Formel $-NR^1R^2$ oder $-OR^4$ steht,

worin

$R^1$, $R^2$ und $R^4$ die oben angegebene Bedeutung haben,

Y und Z gleich oder verschieden sind und
- für eine Gruppe der Formel

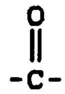 oder 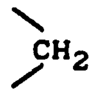

stehen,
oder

Y - für eine Thiocarbonylgruppe steht,

X - für eine Gruppe der Formel $-CH_2-CH_2-$ oder $-CH=CH-$steht,
und

R - für eine Gruppe der Formel

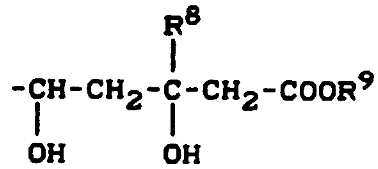 oder 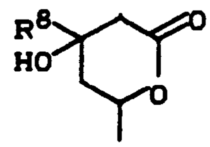

steht,
worin

$R^8$ - Wasserstoff oder geradkettiges oder verzweigtes Alkyl mit bis zu 10 Kohlenstoffatomen bedeutet
und

$R^9$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,

und deren Salze gefunden.

Bildet $R^9$ mit der Carboxygruppe einen Esterrest so ist hiermit bevorzugt ein physiologisch verträglicher Esterrest gemeint, der in vivo leicht zu einer freien Carboxylgruppe und einem entsprechenden physiologisch verträglichen Alkohol hydrolysiert wird. Hierzu, gehören beispielsweise Alkylester ($C_1$ bis $C_6$) und Aralkylester ($C_7$ bis $C_{10}$), bevorzugt ($C_1$-$C_4$)-Alkylester sowie Benzylester. Darüberhinaus seien die folgenden Esterreste genannt: Methylester, Ethylester, Propylester, Benzylester.

Steht $R^9$ für ein Kation, so ist bevorzugt ein physiologisch verträgliches Metall- oder Ammoniumkation gemeint. Bevorzugt sind hierbei Alkali- bzw, Erdalkalikationen wie beispielsweise Natrium-, Kalium-, Magnesium- oder Calciumkationen, sowie Aluminium- oder Ammoniumkationen, sowie nichttoxische substituierte Ammoniumkationen aus Aminen wie ($C_1$-$C_4$)-Dialkylamine, ($C_1$-$C_4$)-Trialkylamine, Prokain, Dibenzylamin, N,N'-Dibenzylethylendiamin, N-Benzyl-$\beta$-phenylethylamin, N-Methylmorpholin oder N-Ethylmorpholin, 1-Ephenamin, Dihydroabietylamin, N,N'-Bis-dihydroabietylethylendiamin, N-Niederalkylpiperidin und andere Amine, die zur Bildung von Salzen verwendet werden können.

Überraschenderweise zeigen die erfindungsgemäßen substituierten Pyrido(2,3-d)pyrimidine eine überlegene inhibitorische Wirkung auf die HMG-CoA-Reduktase (3-Hydroxy-3-methyl-glutaryl-Coenzym A Reduktase).

Im Rahmen der allgemeinen Formel (I) sind Verbindungen der allgemeinen Formel (Ia) und (Ib)

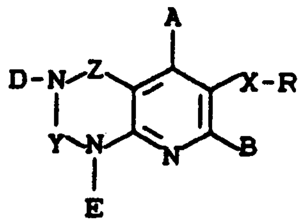

und

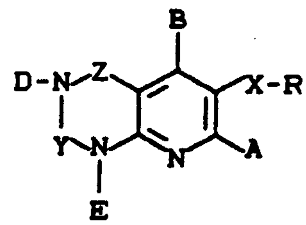

(Ia) . (Ib)

in welcher
A, B, D, E, X, Y, Z und R die oben angegebene Bedeutung haben, bevorzugt.

Bevorzugt sind Verbindungen der allgemeinen Formel (Ia) und (Ib); in welcher

A - für Thienyl, Furyl, Pyridyl oder Pyrimidyl steht, das gegebenenfalls bis zu 3-fach gleich oder verschieden durch Fluor, Chlor, Brom, Hydroxy, Trifluormethyl, geradkettiges oder verzweigtes Alkyl, Alkoxy oder Alkoxycarbonyl mit jeweils bis zu 6 Kohlenstoffatomen, Phenyl, oder durch eine Gruppe der Formel $-NR^1R^2$ substituiert ist,
worin

$R^1$ und $R^2$ gleich oder verschieden sind und
- Wasserstoff, Phenyl, Phenylsulfonyl, geradkettiges oder verzweigtes Alkyl oder Alkylsulfonyl mit bis zu 6 Kohlenstoffatomen, Benzyl oder Benzylsulfonyl bedeuten,
- eine Gruppe der Formel $-COR^3$ bedeuten,
worin

$R^3$ - geradkettiges oder verzweigtes Alkyl oder Alkoxy mit bis zu 6 Kohlenstoffatomen oder Phenyl bedeutet,
oder
- für Phenyl oder Naphthyl steht, das gegebenenfalls bis zu 4-fach gleich oder verschieden substituiert ist durch geradkettiges oder verzweigtes Alkyl, Alkoxy oder Alkoxycarbonyl mit jeweils bis zu 8 Kohlenstoffatomen, welche ihrerseits durch Trifluormethyl, Hydroxy, Alkoxy mit bis zu 4 Kohlenstoffatomen, Phenyl oder Phenoxy substituiert sein können,
oder durch Phenyl, Phenoxy, Phenylthio, Phenylsulfonyl, Fluor, Chlor, Brom, Nitro, Cyano, Trifluormethyl, Benzyloxy oder durch eine Gruppe der Formel $-NR^1R^2$ substituiert ist,
worin

$R^1$ und $R^2$ die oben angegebene Bedeutung haben,
oder
- für ein geradkettiges oder verzweigtes Alkyl mit jeweils bis zu 6 Kohlenstoffatomen steht,

B - für Cyclopropyl, Cyclobutyl, Cyclopentyl oder Cyclohexyl steht,
- für Trifluormethyl oder geradkettiges oder verzweigtes Alkyl mit bis zu 10 Kohlenstoffatomen steht, das gegebenenfalls durch Fluor, Chlor, Brom, Hydroxy, Cyano, Azido, Trifluormethyl, Methylthio, Methylsulfonyl, Alkoxy mit bis zu 6 Kohlenstoffatomen oder durch Phenyl, Phenyloxy oder Phenylthio substituiert ist, wobei die Phenylreste bis zu 2-fach gleich oder verschieden durch Fluor, Chlor, Brom, Cyano, Trifluormethyl, Trifluormethoxy, geradkettiges oder verzweigtes Alkyl, Alkoxy, Alkylthio oder Alkylsulfonyl mit jeweils bis zu 6 Kohlenstoffatomen substituiert sein können, oder durch eine Gruppe der Formel $-NR^1R^2$ oder $-COR^3$ substituiert ist,
worin

$R^1$, $R^2$ und $R^3$ die oben angegebene Bedeutung haben,
oder
- für Phenyl steht, das gegebenenfalls durch Fluor oder Chlor substituiert ist,

D - für Wasserstoff, Hydroxy oder
- Cyclopropyl, Cyclobutyl, Cyclopentyl oder Cyclohexyl steht,

- für geradkettiges oder verzweigtes Alkyl mit bis zu 10 Kohlenstoffatomen steht, das gegebenenfalls durch Fluor, Chlor, Brom, Hydroxy oder Alkoxy mit bis zu 6 Kohlenstoffatomen substituiert ist,

E — die oben angegebene Bedeutung von A hat und mit dieser gleich oder verschieden ist, oder
- für Wasserstoff oder
- Cyclopropyl, Cyclobutyl, Cyclopentyl oder Cyclohexyl steht,
- für geradkettiges oder verzweigtes Alkyl mit bis zu 10 Kohlenstoffatomen steht, das gegebenenfalls durch Fluor, Chlor, Brom, geradkettiges oder verzweigtes Alkenyl mit bis zu 6 Kohlenstoffatomen, Phenyl, Pyrimidyl, Pyrrolyl, Pyrrolidinyl, Furyl oder Thiazolyl substituiert ist, oder durch eine Gruppe der Formel $-NR^1R^2$, $-OR^4$, $-COR^5$ oder $-S(O)_n-R^6$ substituiert ist,

worin

$R^1$ und $R^2$ — die oben angegebene Bedeutung haben,

$R^4$ — Wasserstoff oder
- geradkettiges oder verzweigtes Alkyl mit bis zu 8 Kohlenstoffatomen bedeutet, das gegebenenfalls durch Hydroxy, Fluor, Chlor, Brom oder durch Phenyl substituiert ist, oder
- eine Gruppe der Formel $-COR^7$ bedeutet,

worin

$R^7$ — geradkettiges oder verzweigtes Alkyl mit bis zu 6 Kohlenstoffatomen, Phenyl oder eine Gruppe der Formel $-NR^1R^2$ bedeutet,

worin

$R^1$ und $R^2$ — die oben angegebene Bedeutung haben,

$R^5$ — Wasserstoff, geradkettiges oder verzweigtes Alkyl mit bis zu 6 Kohlenstoffatomen bedeutet, das gegebenenfalls durch Hydroxy, Phenyl, Fluor, Chlor, Brom oder Cyano substituiert ist,
- Phenyl bedeutet, welches seinerseits durch Fluor, Chlor, Brom, Amino, Hydroxy, Nitro oder Cyano substituiert sein kann, oder
- eine Gruppe der Formel $-NR^1R^2$ oder $-OR^4$ bedeutet,

worin

$R^1$, $R^2$ und $R^4$ — die oben angegebene Bedeutung haben,

n — eine Zahl 0 oder 2 bedeutet,

$R^6$ — geradkettiges oder verzweigtes Alkyl mit bis zu 8 Kohlenstoffatomen, oder
- Phenyl bedeutet, das durch Fluor, Chlor, Brom, Hydroxy, Cyano, Nitro oder Amino substituiert sein kann, oder
- eine Gruppe der Formel $-NR^1R^2$ bedeutet, wenn n für die Zahl 2 steht,

worin

$R^1$ und $R^2$ — die oben angegebene Bedeutung haben,

oder

E — für eine Gruppe der Formel $-NR^1R^2$ oder $-OR^4$ steht,

worin

$R^1$, $R^2$ und $R^4$ — die oben angegebene Bedeutung haben,

Y und Z — gleich oder verschieden sind und
- für eine Gruppe der Formel

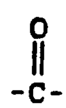 oder 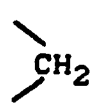

stehen,

oder

Y — für eine Thiocarbonylgruppe steht,

X — für eine Gruppe der Formel $-CH_2-CH_2-$ oder $-CH=CH-$steht

und

R — für eine Gruppe der Formel

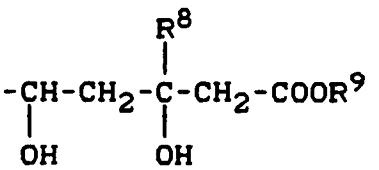 oder 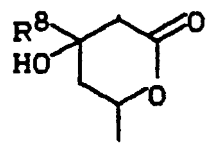

steht, worin

R⁸ - Wasserstoff oder geradkettiges oder verzweigtes Alkyl mit bis zu 8 Kohlenstoffatomen bedeutet
und

$R^9$ - Wasserstoff oder geradkettiges oder verzweigtes Alkyl mit bis zu 8 Kohlenstoffatomen oder Benzyl bedeutet, oder
- Phenyl oder ein Kation bedeutet

und deren Salze.

Besonders bevorzugt sind Verbindungen der allgemeinen Formeln (Ia) und (Ib),
in welchen

A - für Thienyl, Furyl oder Pyridyl steht, das gegebenenfalls bis zu 2-fach gleich oder verschieden durch Fluor, Chlor, Brom, Hydroxy, Trifluormethyl, Methyl, Ethyl, Methoxy, Ethoxy oder Phenyl, oder durch eine Gruppe der Formel $-NR^1R^2$ substituiert ist,
worin

$R^1$ und $R^2$ gleich oder verschieden sind und
- Wasserstoff, Phenyl, geradkettiges oder verzweigtes Alkyl mit bis zu 4 Kohlenstoffatomen bedeuten,
- für Phenyl steht, das gegebenenfalls bis zu 3-fach gleich oder verschieden substituiert ist durch geradkettiges oder verzweigtes Alkyl oder Alkoxy mit jeweils bis zu 6 Kohlenstoffatomen, welche ihrerseits durch Trifluormethyl, Hydroxy, Methoxy, Ethoxy, Propoxy, Phenyl oder Phenoxy substituiert sein können,
oder durch Phenyl, Phenoxy, Fluor oder Chlor substituiert ist,
oder
- für ein geradkettiges oder verzweigtes Alkyl mit jeweils bis zu 4 Kohlenstoffatomen steht,

B - für Cyclopropyl, Cyclobutyl, Cyclopentyl oder Cyclohexyl steht,
- für Methyl, Ethyl, Propyl, Isopropyl, Butyl, tert.-Butyl oder Trifluormethyl steht,
- für Phenyl steht, das gegebenenfalls durch Fluor substituiert ist,

D - für Wasserstoff oder
- für Cyclopropyl, Cyclopentyl oder Cyclohexyl steht,
- für Methyl, Ethyl, Propyl, Isopropyl, Butyl oder tert.Butyl steht,

E die oben angegebene Bedeutung von A hat und mit dieser gleich oder verschieden ist,
oder
- für Wasserstoff, oder
- für Cyclopropyl, Cyclopentyl oder Cyclohexyl steht,
- für geradkettiges oder verzweigtes Alkyl mit bis zu 8 Kohlenstoffatomen steht, das gegebenenfalls durch Fluor, Chlor, geradkettiges oder verzweigtes Alkenyl mit bis zu 4 Kohlenstoffatomen, Phenyl oder Furyl substituiert ist, oder
- durch eine Gruppe der Formel $-NR^1R^2$ oder $-OR^4$ substituiert ist,
worin

$R^1$ und $R^2$ die oben angegebene Bedeutung haben,

$R^4$ - Wasserstoff oder
- geradkettiges oder verzweigtes Alkyl mit bis zu 6 Kohlenstoffatomen bedeutet, das gegebenenfalls durch Hydroxy oder durch Phenyl substituiert ist,
oder

E - für eine Gruppe der Formel $-NR^1R^2$ steht,
worin

$R^1$ und $R^2$ die oben angegebene Bedeutung haben,

Y und Z gleich oder verschieden sind und
- für eine Gruppe der Formel

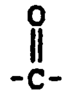 oder 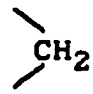

stehen,

oder

Y - für eine Thiocarbonylgruppe steht,

X - für eine Gruppe -CH = CH- steht

und

R - für eine Gruppe der Formel

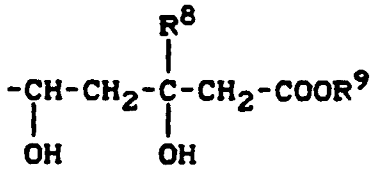 oder 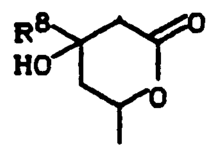

steht, worin

$R^8$ - Wasserstoff, Methyl, Ethyl, Propyl, Isopropyl, Butyl, Isobutyl oder tert.Butyl bedeutet

und

$R^9$ - Wasserstoff, Methyl, Ethyl, Propyl, Isopropyl, Butyl, Isobutyl, tert.Butyl oder Benzyl bedeutet, oder

- ein Natrium-, Kalium-, Calcium-, Magnesium- oder Ammoniumion bedeutet

und deren Salze.

Die erfindungsgemäßen substituierten Pyrido(2,3-d)pyrimidine der allgemeinen Formel (I) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der Gruppe X bzw. des Restes R ergeben sich unterschiedliche Stereoisomere, die im folgenden näher erläutert werden sollten:

a) Steht die Gruppe -X- für eine Gruppe der Formel -CH = CH- , so können die erfindungsgemäßen Verbindungen in zwei stereoisomeren Formen existieren, die an der Doppelbindung E-konfiguriert (II) oder Z-konfiguriert (III) sein können:

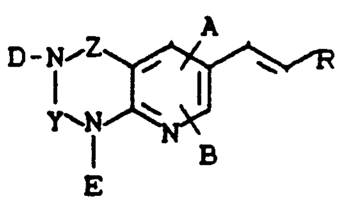

(II) E-Form

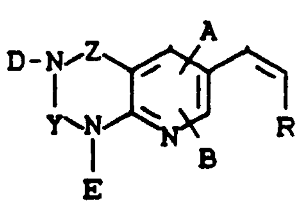

(III) Z-Form

(A, B, D, E, Y, Z und R haben die oben angegebene Bedeutung).

Bevorzugt sind diejenigen Verbindungen der allgemeinen Formel (I) die E-konfiguriert sind (II).

b) Steht der Rest -R- für eine Gruppe der Formel

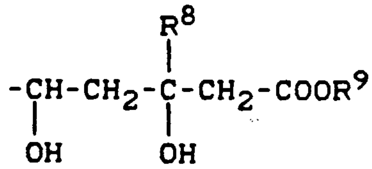

so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

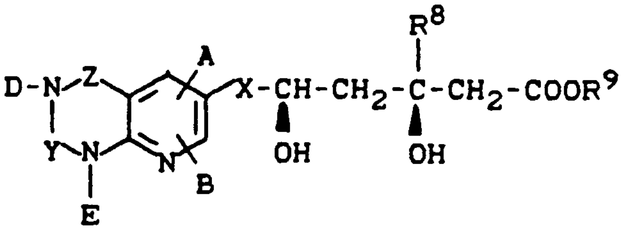

Erythro-Form (IV)

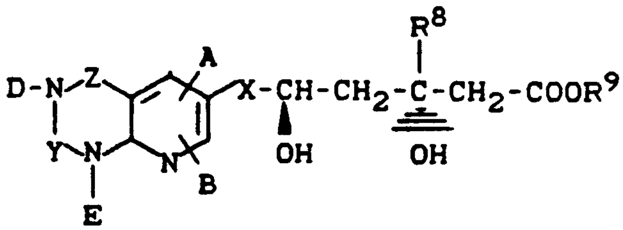

Threo-Form (V)

Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).
Bevorzugt sind hierbei die Erythro-konfigurierten Isomeren, besonders bevorzugt das 3R,5S-Isomere sowie das 3R,5S-3S,5R-Racemat.
c) Steht der Rest -R- für eine Gruppe der Formel

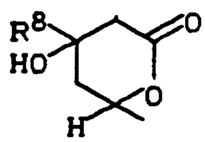

so besitzen die substituierten Pyrido(2,3-d)pyrimidine mindestens zwei asymmetrische Kohlenstoffatome, nämlich das Kohlenstoffatom an dem die Hydroxygruppe gebunden ist, und das Kohlenstoffatom an welchem der Rest der Formel

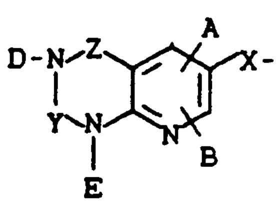

gebunden ist. Je nach der Stellung der Hydroxygruppe zur freien Valenz am Lactonring können die substituierten Pyrido(2,3-d)pyrimidine als cis-Lactone (VI) oder als trans-Lactone (VII) vorliegen.

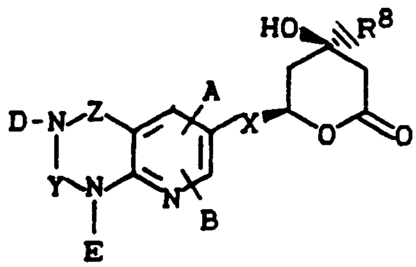

cis-Lacton (VI)

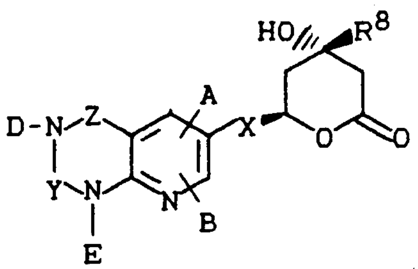

trans-Lacton (VII)

Sowohl vom cis-Lacton aus als auch vom trans-Lacton existieren wiederum jeweils zwei Isomere nämlich das 4R,6R-Isomere bzw. das 4S,6S-Isomeren (cis-Lacton), sowie das 4R,6S-Isomere bzw. 4S,6R-Isomere (trans-Lacton). Bevorzugte Isomeren sind die trans-Lactone. Besonders bevorzugt ist hierbei das 4R,6S-Isomere (trans) sowie das 4R,6S-4S,6R-Racemat.

Beispielsweise seien die folgenden isomeren Formen der substituierten Pyrido(2,3-d)pyrimidine genannt:

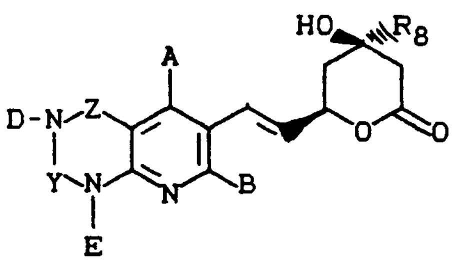

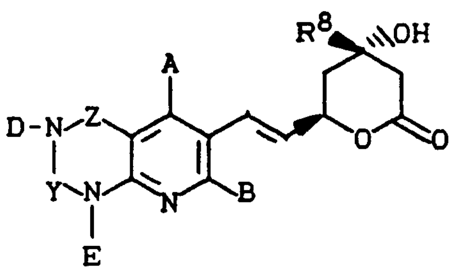

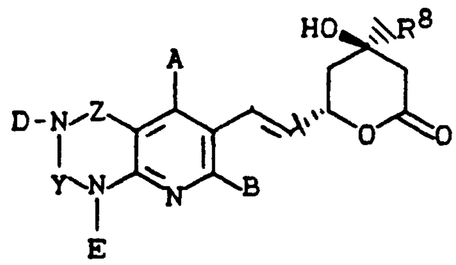

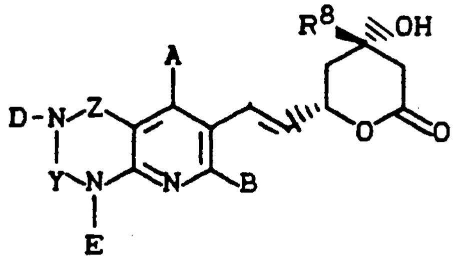

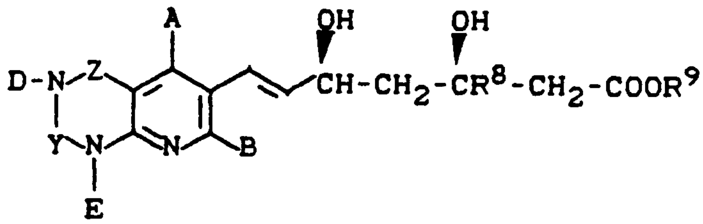

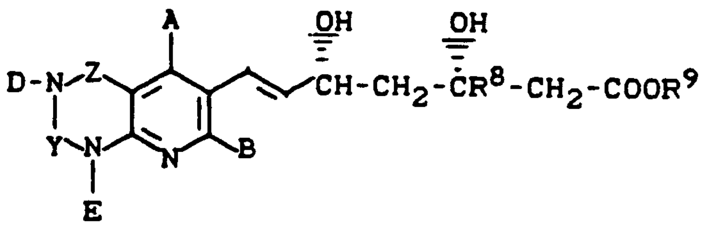

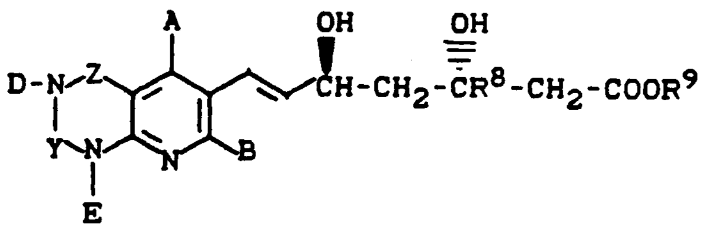

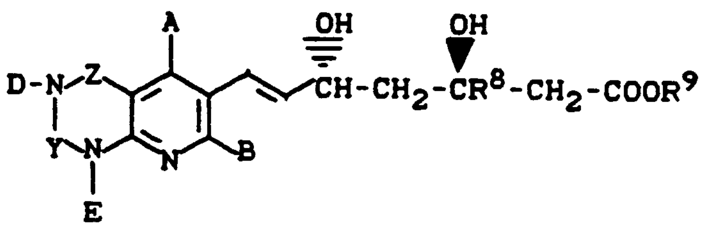

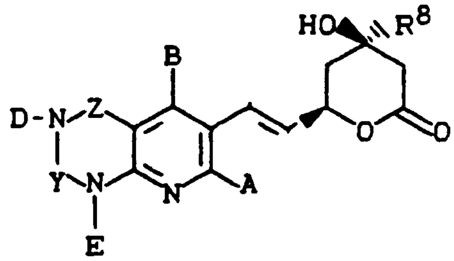

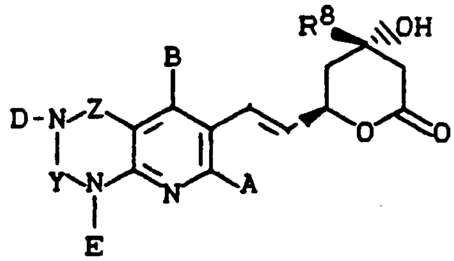

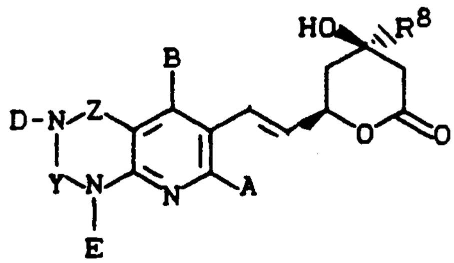

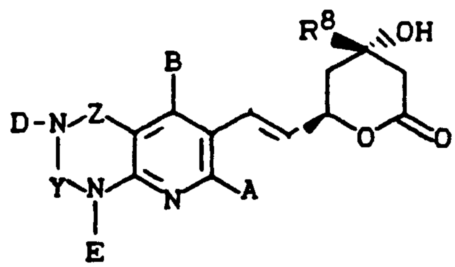

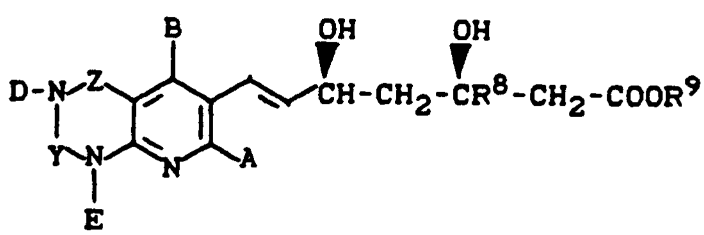

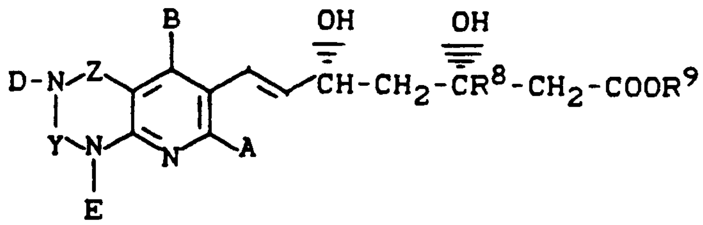

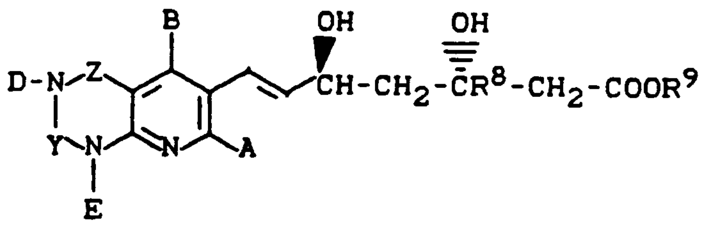

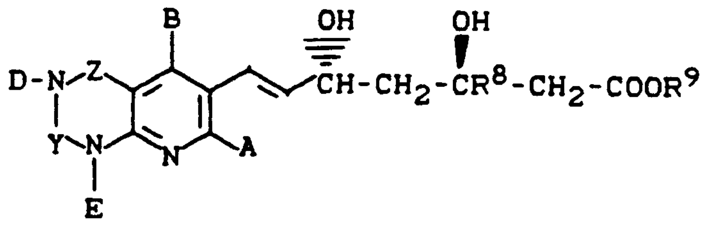

Außerdem wurde ein Verfahren zur Herstellung der substituierten Pyrido(2,3-d)pyrimidine der allgemeinen Formel (I)

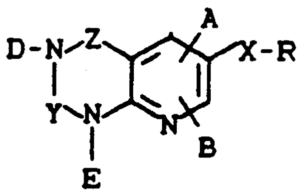

(I)

in welcher
A, B, D, E, X, Y, Z und R die oben angegebene Bedeutung haben,
gefunden,
das dadurch gekennzeichnet ist, daß man
Ketone der allgemeinen Formel (VIII)

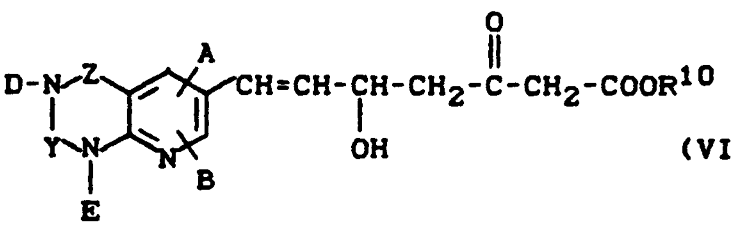

(VIII)

in welcher
A, B, D, E, Y und Z die oben angegebene Bedeutung haben,
und

$R^{10}$ - für Alkyl mit bis zu 6 Kohlenstoffatomen steht,

reduziert,

im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X = -CH=CH-) nach üblichen Methoden hydriert,
und gegebenenfalls Isomeren trennt.

Das erfindungsgemäße Verfahren kann durch das folgende Formelschema erläutert werden:

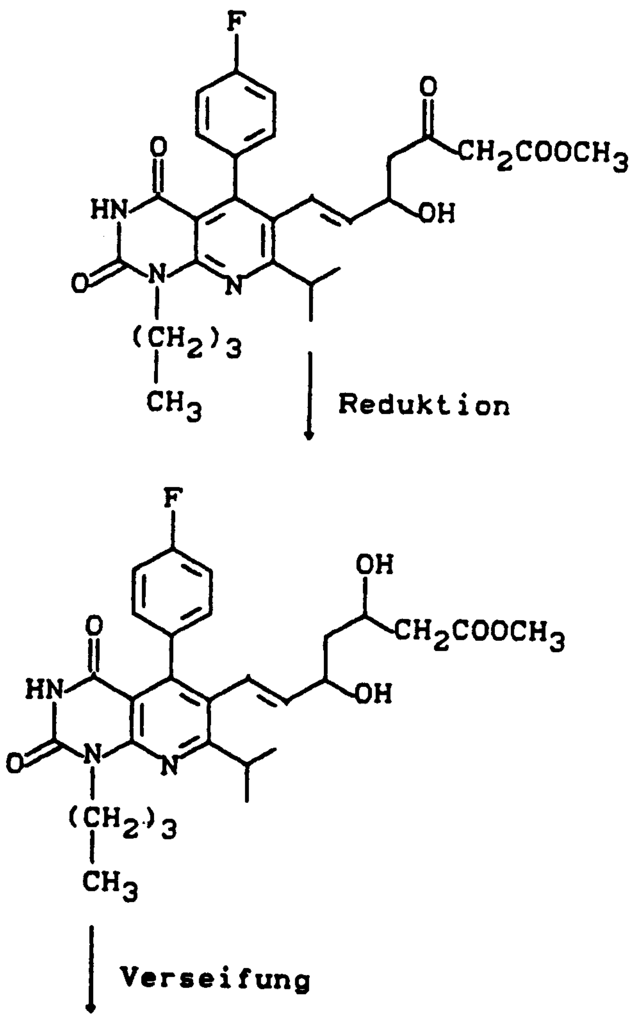

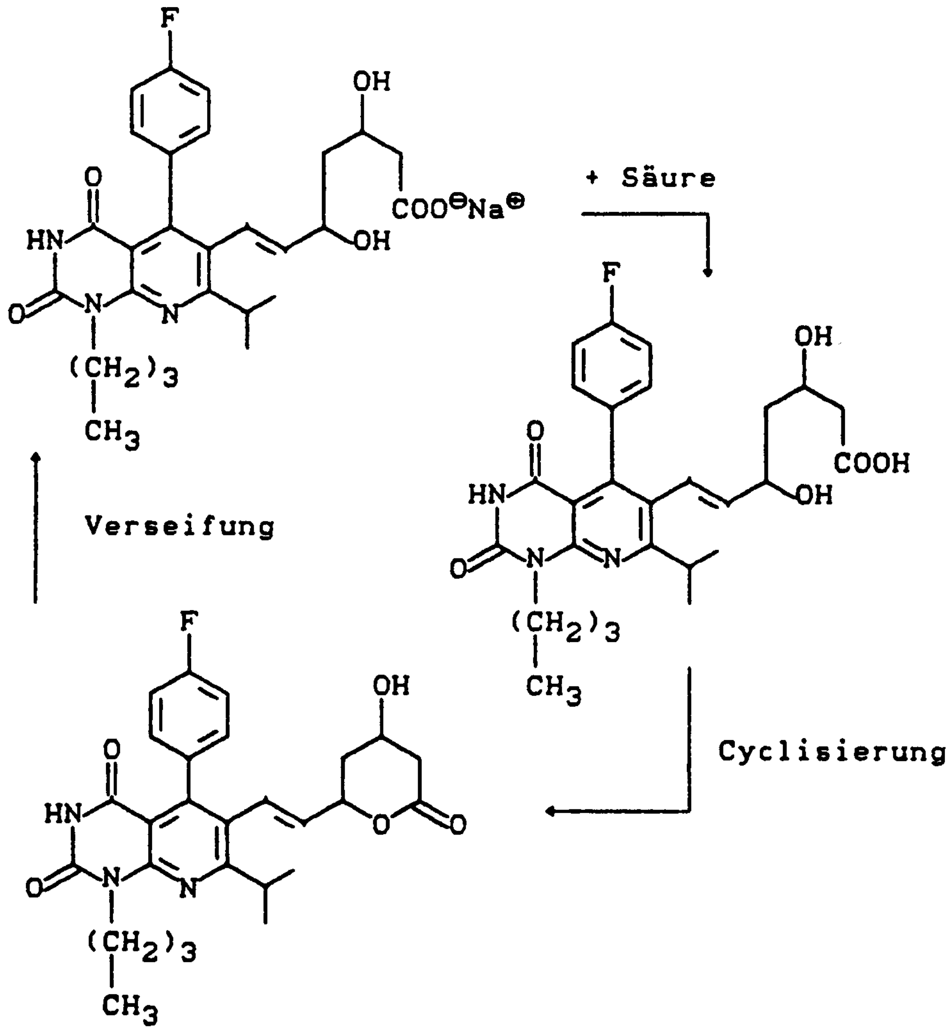


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-borate, Natrium-trialkyl-hydrido-boranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran und Methanol durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden. Hierzu besonders geeignet ist die Verwendung von Natriumborhydrid als Reduktionsmittel, in Anwesenheit von Triethylboran und Methanol in inerten Lösemitteln wie vorzugsweise Ethern.

Die Reduktion erfolgt im allgemeinen in einem Temperaturbereich von -80 °C bis +30 °C, bevorzugt von -78 °C bis 0 °C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (III) unter solchen Bedingungen durchgeführt werden, unter denen sowohl die Carbonylgruppe als auch die Doppelbindung reduziert wird.

Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ic)

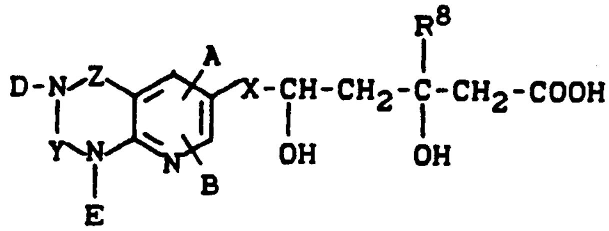

(Ic)

in welcher

A, B, D, E, X, Y, Z und $R^8$ die oben angegebene Bedeutung haben.

Die Carbonsäureester im Rahmen der allgemeinen Formel (I) entsprechen der Formel (Id)

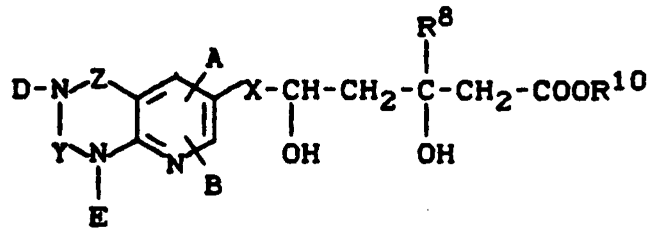

(Id)

in welcher

A, B, D, E, X, Y, Z und $R^8$ die oben angegebene Bedeutung haben,

und

$R^{10}$ - für Alkyl mit bis zu 6 Kohlenstoffatomen steht.

Die Salze der erfindungsgemäßen Verbindungen im Rahmen der allgemeinen Formel (I) entsprechen der Formel (Ie)

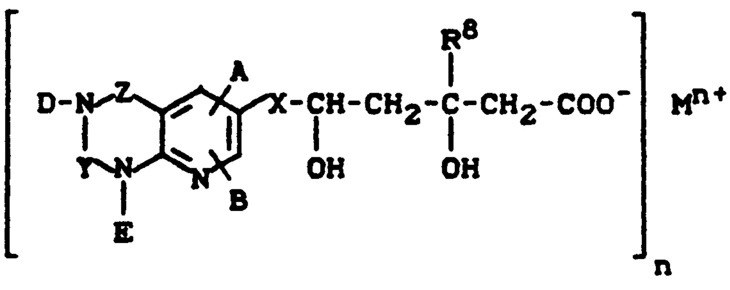

(Ie)

in welcher

A, B, D, E, X, Y, Z und $R^8$ die oben angegebene Bedeutung haben,

und

$M^{n+}$ für ein Kation steht, wobei n die Wertigkeit angibt.

Die Lactone im Rahmen der allgemeinen Formel (I) entsprechen der Formel (If)

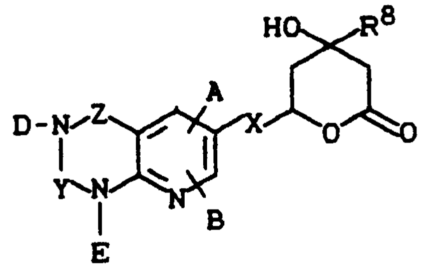

(If)

in welcher
A, B, D, E, X, Y, Z und $R^8$ die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen Lanorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Ebenso ist es möglich, Gemische der genannten Lösemittel einzusetzen. Besonders bevorzugt werden Alkohole wie Methanol, Ethanol, Propanol oder Isopropanol oder ein Gemisch von Tetrahydrofuran und Wasser verwendet.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +200°C, bevorzugt von -25°C bis +110°C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei

bevorzugt Carbodiimide verwendet. Als Carbodiimide werden bevorzugt N,N′-Dicyclohexylcarbodiimid-Paratoluolsulfonat, N-Cyclohexyl-N′-[2-(N″-methylmorpholinium)ethyl]carbodiimid oder N-(3-Dimethylamino-propyl)-N′-ethylcarbodiimid-Hydrochlorideingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0 °C bis +80 °C, bevorzugt von +10 °C bis +50 °C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Ester. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ig)

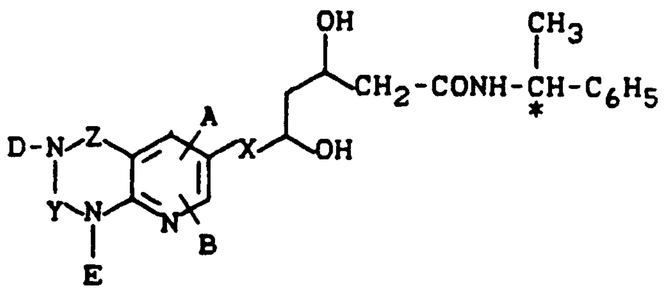

(Ig)

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden:

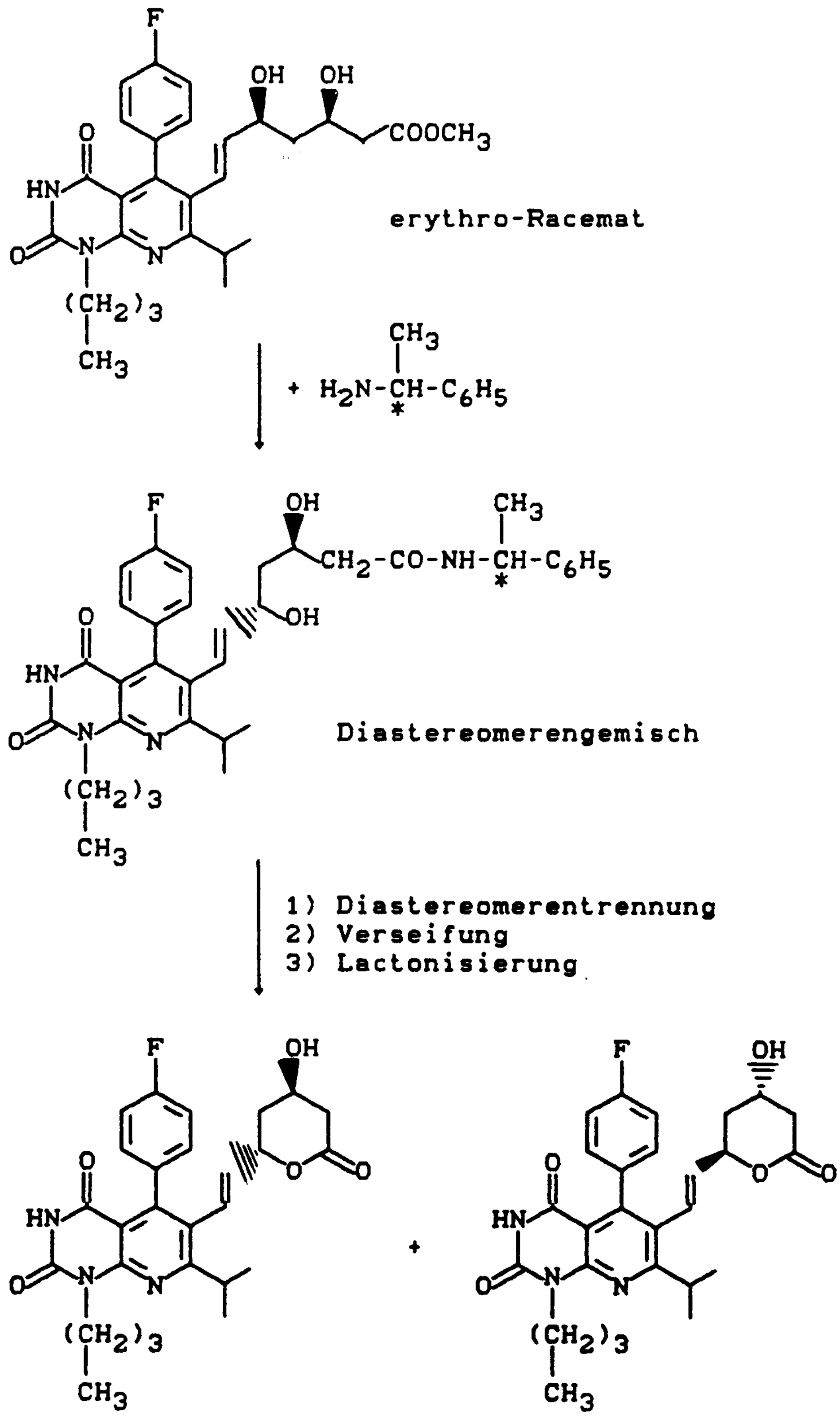


Die als Ausgangsstoffe eingesetzten Ketone (VIII) sind neu.

Es wurde ein Verfahren zur Herstellung der erfindungsgemäßen Ketone der allgemeinen Formel (VIII)

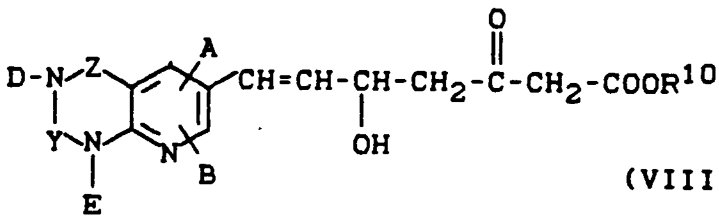

(VIII)

in welcher
A, B, D, E, Y, Z und $R^{10}$ die oben angegebene Bedeutung haben,
gefunden, das dadurch gekennzeichnet ist, daß man
Aldehyde der allgemeinen Formel (IX)

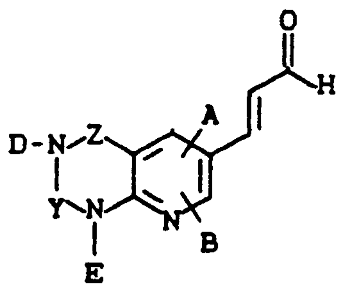

(IX)

in welcher
A, B, D, E, Y und Z die oben angegebene Bedeutung haben,
in inerten Lösemitteln mit Acetessigester der allgemeinen Formel (X)

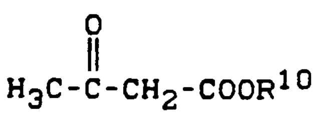

(X)

in welcher
$R^{10}$ die oben angegebene Bedeutung hat,
in Anwesenheit von Basen umsetzt.

Das erfindungsgemäße Verfahren kann beispielsweise durch folgendes Formelschema erläutert werden:

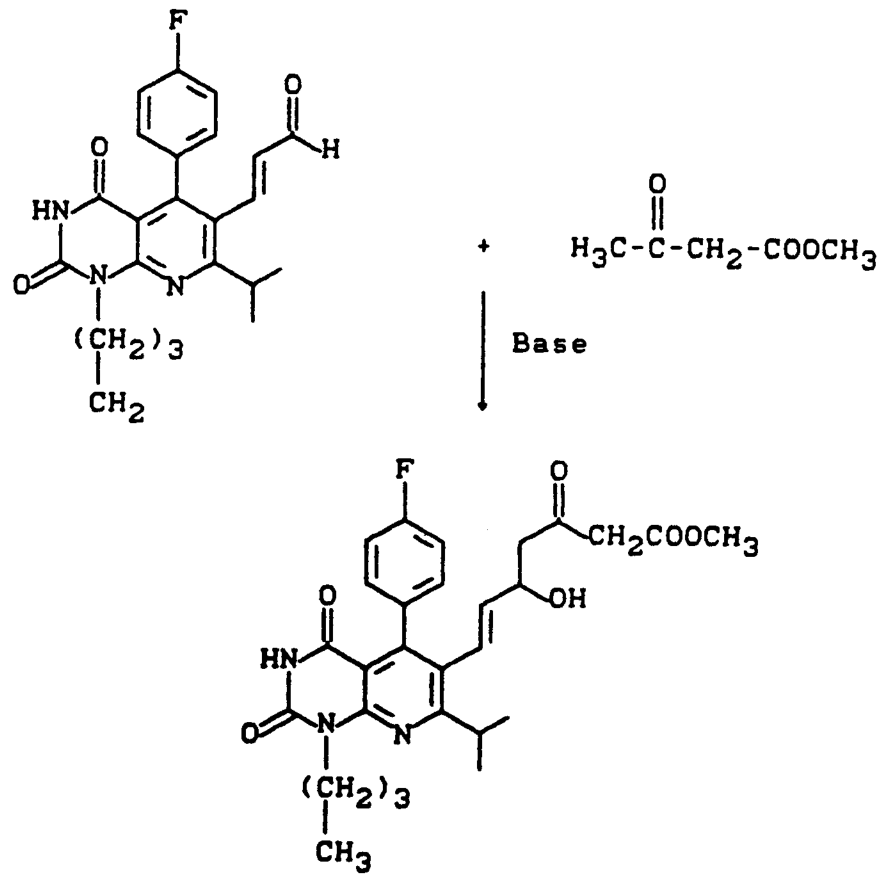


Als Basen kommen hierbei die üblichen stark basischen Verbindungen in Frage. Hierzu gehören bevorzugt lithiumorganische Verbindungen wie beispielsweise N-Butyllithium, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Eventuell sind Zusätze von Metallhalogeniden wie z.B. Magnesiumchlorid, Zinkchlorid oder Zinkbromid vorteilhaft. Besonders bevorzugt ist der Zusatz von Zinkhalogeniden.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erdölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80 °C bis +50 °C, bevorzugt von -20 °C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 3, bevorzugt von 1 bis 2 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:
Acetessigsäuremethylester, Acetessigsäureethylester, Acetessigsäurepropylester, Acetessigsäureisopropylester.

Die Herstellung der als Ausgangsstoffe eingesetzten Aldehyde der allgemeinen Formel (IX) soll im folgenden beispielhaft für die 1,2,3,4-Tetrahydro-pyrido(2,3-d)pyrimidine des Typs (Ia) erläutert werden.

[A]

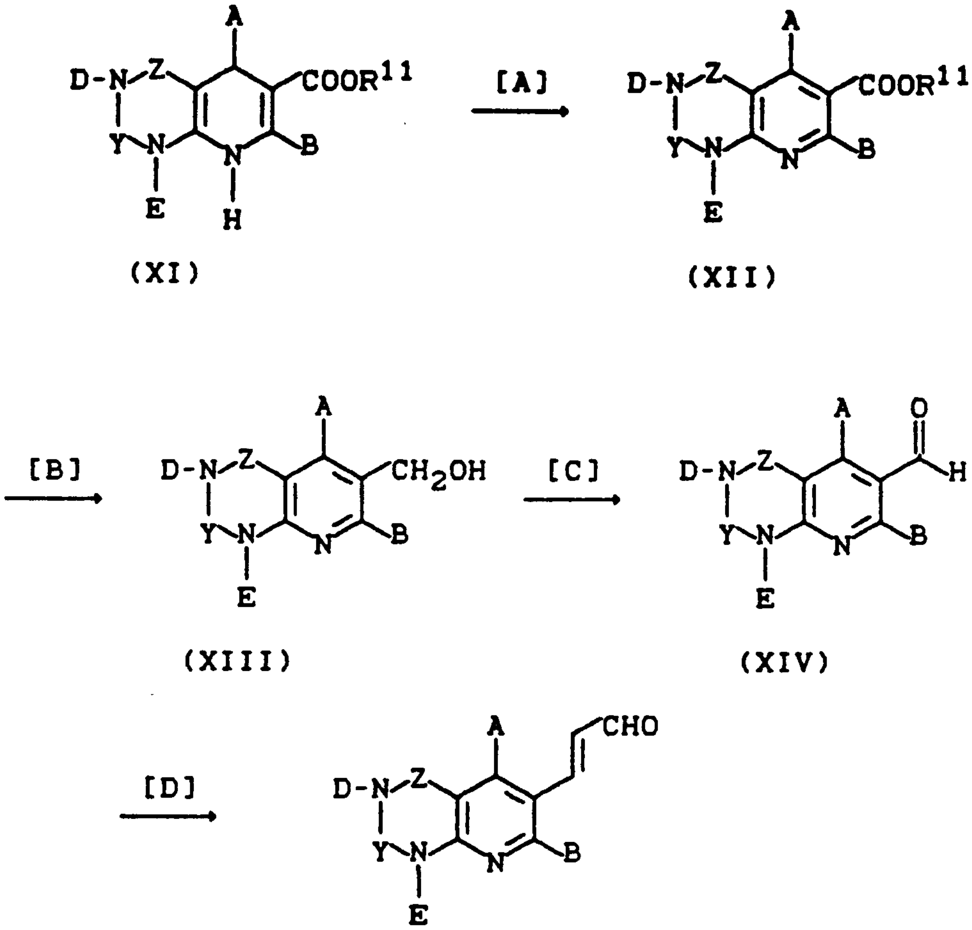


(XI) (XII) (XIII) (XIV) (IX)

Hierbei werden gemäß im ersten Schritt [A] die 1,2,3,4,5,8-Hexahydro-pyrido(2,3-d)pyrimidine der allgemeinen Formel (XI), in welcher $R^{11}$ für einen Alkylrest mit bis zu 4 Kohlenstoffatomen steht, in geeigneten Lösemitteln, mit geeigneten Oxidationsmitteln oxidiert. Bevorzugt werden 1,2,3,4-Hexahydro-pyrido(2,3-d)-pyrimidine in Chlorkohlenwasserstoffen wie beispielsweise Methylenchlorid, mit 2,3-Dichlor-4,5-dicyano-p-benzochinon bei Raumtemperatur, oder mit Chromtrioxid in Eisessig bei erhöhten Temperaturen, bevorzugt unter Rückflußtemperatur, zu den 1,2,3,4-Tetrahydro-pyrido(2,3-d)pyrimidinen (XII) oxidiert. Im zweiten Schritt [B] werden die 1,2,3,4-Tetrahydro-pyrido(2,3-d)pyrimidine (XII) in inerten Lösemitteln wie Ethern, beispielsweise Diethylether, Tetrahydrofuran oder Dioxan, oder in Kohlenwasserstoffen beispielsweise Benzol oder Toluol, vorzugsweise in Tetrahydrofuran oder Toluol, mit Metallhydriden als Reduktionsmittel, beispielsweise Lithiumaluminiumhydrid, Natriumcyanoborhydrid, Natriumaluminiumhydrid, Diisobutylaluminiumhydrid oder Natrium-bis-(2-methoxyethoxy)dihydroaluminat, in Temperaturbereichen von -70°C bis +100°C, vorzugsweise von -70°C bis Raumtemperatur, bzw. von Raumtemperatur bis 70°C je nach verwendetem Reduktionsmittel zu den Hydroxymethylverbindungen (XIII) reduziert. Vorzugsweise wird die Reduktion mit Diisobutylaluminiumhydrid in Tetrahydrofuran in einem Temperaturbereich von -78°C bis Raumtemperatur durchgeführt. Im dritten Schritt [C] werden die Hydroxymethylverbindungen (XIII) nach üblichen Methoden zu den Aldehyden (XIV) oxidiert. Die Oxidation kann beispielsweise mit Pyridiniumchlorochromat, gegebenenfalls in Anwesenheit von Aluminiumoxid, in inerten Lösemitteln wie Chlorkohlenwasserstoffen oder Ethern, vorzugsweise Methylenchlorid oder Tetrahydrofuran, bei Raumtemperatur oder mit Trifluoressigsäureanhydrid und Dimethylsulfoxid (Swern-Oxidation) oder aber nach anderen für die Oxidation von Hydroxymethylverbindungen zu Aldehyden üblichen Methoden erfolgen. Im vierten Schritt [D] werden die Aldehyde (XIV) durch Umsetzen mit Diethyl-2-(cyclohexylamino)-vinyl-phosphonat oder mit 1,3-Dioxan2-yl-methyl-triphenylphosphonium-bromid in inerten Lösemitteln wie Ethern oder Dimethylformamid, bevorzugt in Tetrahydrofuran in Anwesenheit von Natriumhydrid oder Natriummethanolat, in einem Temperaturbereich von -20°C bis +30°C, bevorzugt von -5°C bis Raumtemperatur in die Verbindungen (IX) überführt.

Die Verbindungen der allgemeinen Formel (IX), in welcher D die oben angegebene Bedeutung hat, aber nicht für Wasserstoff steht, können hergestellt werden,
indem man
Verbindungen der allgemeinen Formel (IXa)

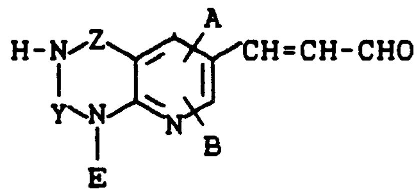

(IXa)

in welcher
A, B, E, Y und Z die oben angegebene Bedeutung haben,
zur Einführung von D, mit Alkylhalogeniden, vorzugsweise Alkyliodid, beispielsweise Methyljodid, in Anwesenheit von den oben aufgeführten Basen, vorzugsweise Natriumhydrid oder mit aktivierten Olefinen, wie beispielsweise Acrylnitril in den oben erwähnten inerten Lösemitteln, vorzugsweise Tetrahydrofuran alkyliert.

Die als Ausgangsstoffe eingesetzten Verbindungen der allgemeinen Formel (XI) sind an sich bekannnt oder können nach üblichen Methoden hergestellt werden [vgl. Ger. Offen. DE 27 38 153, US 4 596 805; V. Papesch, E.F. Schroeder, J. Org. Chem. 13, 1879 (1951); T. Kishikawe, H. Yuhi, Chem. Pharm. Bull. Band 14, 1365-1370 (1966)].

Die Ausgangsverbindungen der allgemeinen Formeln (XII), (XIII) und (XIV) sind an sich bekannt oder können nach üblichen Methoden hergestellt werden [Chem. Ber. 101 (2), 512 - 521; J. Heterocycl. Chem., 22 (2), 345 - 347; Khim. Geterosikl. Soedin., (6), 834 - 837; Farmaco, Ed. Sci. 37 (4), 247 - 258].

Die erfindungsgemäßen Verbindungen der allgemeinen Formel (I) besitzen wertvolle pharmakologische Eigenschaften und können in Arzneimitteln eingesetzt werden. Insbesondere sind sie Inhibitoren der 3-Hydroxy-3-methylglutaryl-Coenzym A (HMG-CoA) Reduktase und infolge dessen Inhibitoren der Cholesterolbiosynthese. Sie können deshalb zur Behandlung von Hyperlipoproteinämie, Lipoproteinämie oder Atheriosklerose eingesetzt werden. Die erfindungsgemäßen Wirkstoffe bewirken außerdem eine Senkung des Cholesteringehaltes im Blut.

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Colestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat, 0,03 m Ethylendiamintetraessigsäure, 0,002 m Dithiothreit (SPE)-Puffer pH 7,2, homogenisiert. Anschließend wurde 15 Minuten bei 15 000 g zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumes SPE-Puffer aufgenommen, homogenisiert und bei -78°C eingefroren und gelagert (= Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase 35 µMol $K_xH_y$-Phosphat pH 7,2, 20 µl Enzympräparation und 56 nMol $3_x$-Hydroxy-3-methyl-glutaryl-Coenzym A (Glutaryl-3-$^{14}$C) 100 000 dpm.

Nach Inkubation von 60 Minuten bei 37°C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. $IC_{50}$-Werte wurden durch Auftrag der prozentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 1 gesetzt und mit

dem simultan bestimmten $IC_{50}$-Wert der Testverbindung verglichen.

| Relative in vitro-Aktivitäten | |
|---|---|
| Beispiel-Nr. | relative Aktivität (Mevinolin = 1) |
| 8 | 2 |
| 16 | 6 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchem die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

A) AUSGANGSPRODUKTE

A 1

E,Z-2-Ethoxycarbonyl-1-(4-fluorphenyl)-4-methyl-pent-1-en-3-on

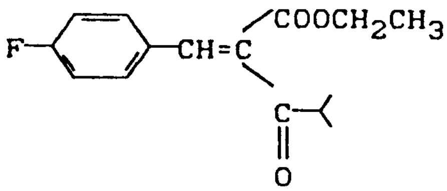

Zu 554 g (3,5 mol) Isobutyrylessigsäureethylester und 434 9 (3,5 mol) 4-Fluorbenzaldehyd in 1,8 l Isopropanol gibt man eine Lösung von 20 ml (0,2 mol) Piperidin und 12 ml (0,21 mmol) Essigsäure in 200 ml Isopropanol. Man rührt 1 Tag bei Raumtemperatur, engt im Vakuum ein und destilliert den Rückstand im Hochvakuum.
Ausbeute: 796 g (86% der Theorie) gelbliches Öl
Kp.; 135 - 140 °C (0.2 mbar)

A 2

1-Butyl-6-ethoxycarbonyl-5-(4-fluorphenyl)-7-isopropyl-2,4-dioxo-1,2,3,4,5,8-hexahydro-pyrido(2,3-d)-pyrimidin

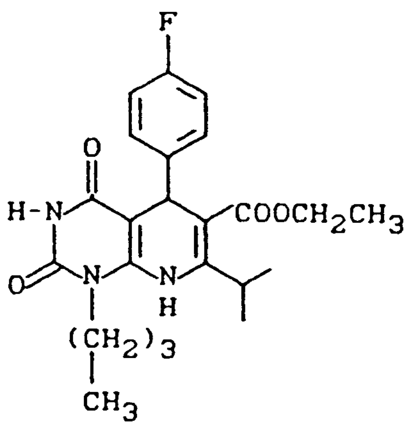

91,6 g (0,5 mol) 6-Amino-1-butyl-uracil und 145,4 g (0,55 mol) der Verbindung aus A 1 werden in 750 ml Isopropanol über Nacht am Rückfluß gekocht. Man gibt noch 39,6 g (0,15 mol) der Verbindung aus A 1 zu und kocht einen weiteren Tag. Der ausgefallene Niederschlag wird abgesaugt und mit wenig Isopropanol nachgewaschen.
Ausbeute: 143,9 g (67% der Theorie) farblose Kristalle
Schmp.: 214 °C (aus Methanol)

A 3

1-Butyl-6-ethoxycarbonyl-5-(4-fluorphenyl)-7-isopropyl-2,4-dioxo-1,2,3,4-tetrahydro-pyrido(2,3-d)pyrimidin

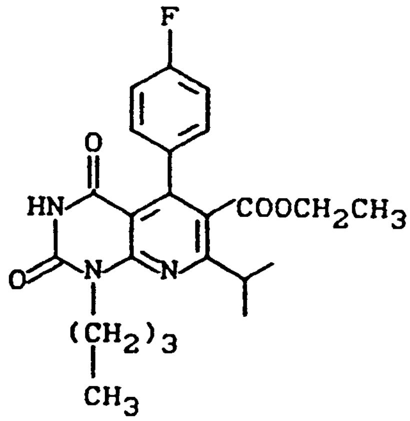

Zu einer Mischung von 21,5 g (50 mmol) der Verbindung aus A 2 in 800 ml Dichlormethan gibt man 11,35 g (50 mmol) 2,3-Dichlor-4,5-dicyano-benzochinon und rührt 1 h bei Raumtemperatur. Man filtriert von einem beigen Niederschlag ab, wäscht das Filtrat viermal mit Wasser, trocknet die organische Phase über Natriumsulfat, filtriert über eine kurze Schicht Aktivkohle und engt das Filtrat im Vakuum ein. Der Rückstand wird aus Ether/Petrolether kristallisiert.
Ausbeute: 20,1 g (94% der Theorie) farblose Kristalle
Schmp.: 129 ° C

A 4

1-Butyl-5-(4-fluorphenyl)-6-hydroxymethyl-7-isopropyl-2,4-dioxo-1,2,3,4-tetrahydro-pyrido(2,3-d)pyrimidin

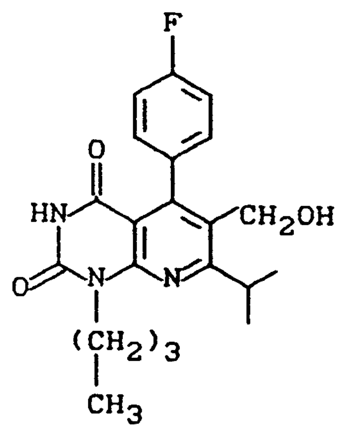

Unter Argon gibt man zu einer Suspension von 19,2 g (45 mmol) der Verbindung aus A 3 in 400 ml Toluol bei -75 ° C langsam 135 ml einer 1 molaren Lösung von Diisobutylaluminiumhydrid in Toluol, was zu einer klaren Lösung führt. Nach 1 h gibt man bei derselben Temperatur noch 30 ml Dibutylaluminiumhydrid-Lösung zu, rührt eine weitere Stunde und laßt dann auf Raumtemperatur erwärmen, wobei ab -30 ° C vorsichtig mit 400 ml Wasser und 200 ml Essigester versetzt wird. Man saugt über Kieselgur ab und wäscht mit Essigester nach. Nach Phasentrennung extrahiert man die wäßrige Phase mit Essigester, wäscht die vereinigten organischen Phasen mit Natriumchloridlösung, trocknet über Natriumsulfat und engt im Vakuum ein. Der Rückstand wird in einer Säule (Ø 6 cm) an 500 g Kieselgel 230-400 mesh mit Petrolether/Essigester (2:1) chromatographiert.
Ausbeute: 11.8 g (68% der Theorie) farblose Kristalle
Schmp.: 99 ° C (aus Ether/Petrolether)

A 5

1-Butyl-5-(4-fluorphenyl)-6-formyl-7-isopropyl-2,4-dioxo-1,2,3,4-tetrahydro-pyrido(2,3-d)pyrimidin

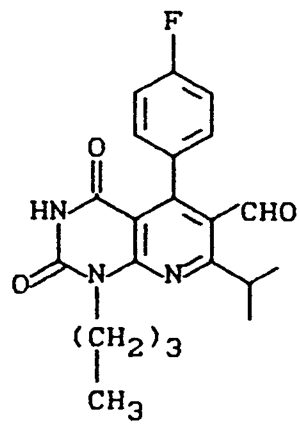

8,5 g (22 mmol) der Verbindung aus A 4 werden in 220 ml Dichlormethan gelöst, mit 4,5 g neutralem Aluminiumoxid und 9,5 g (44 mmol) Pyridiniumchlorochromat versetzt und 1 h bei Raumtemperatur gerührt. Man filtriert über ein Kieselgelbett, ohne trocken zu saugen, wäscht mit Dichlormethan nach und engt das Filtrat im Vakuum zur Trockne ein. Der Rückstand wird an 150 g Kieselgel 230-400 mesh in einer Säule (Ø 4 cm) mit Petrolether/Essigester 2:1 chromatographiert.
Ausbeute: 7,6 g (90% der Theorie) farbloser Feststoff
Schmp.: 179 °C

A 6

(E)-3-[1-Butyl-5-(4-fluorphenyl)-7-isopropyl-2,4-dioxo-1,2,3,4-tetrahydro-pyrido(2,3-d)pyrimidin-6-yl]prop-2-enal

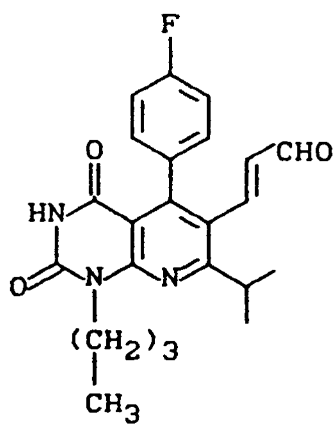

Zu einer Suspension von 0,25 g (8,4 mmol) 80%igem Natriumhydrid in 15 ml wasserfreiem Tetrahydrofuran tropft man innerhalb von 10 min bei 0 - 5 °C unter Argon eine Lösung von 1,15 g (4,4 mol) Diethyl-2-(cyclohexylamino)-vinyl-phosphonat in 15 ml Tetrahydrofuran. Es wird 15 min bei 0 °C gerührt, innerhalb von 20 min eine Lösung von 1,5 g (4 mmol) der Verbindung aus A 5 in 15 ml Tetrahydrofuran bei 0 °C zugetropft, 1 h bei Raumtemperatur und 20 min unter Rückfluß gerührt. Man kühlt ab, versetzt vorsichtig mit 50 ml Wasser, extrahiert zweimal mit Essigester, wäscht die vereinigten organischen Phasen mit Kochsalzlösung und engt im Vakuum ein. Der Rückstand wird mit einer Mischung aus 25 ml Toluol, 35 ml Wasser und 2,6 g (36 mmol) Oxalsäuredihydrat 1 h am Rückfluß gekocht. Die Phasen werden getrennt, die wäßrige Phase mit Essigester extrahiert, die vereinigten organischen Phasen mit Kochsalzlösung gewaschen, über Magnesiumsulfat getrocknet und im Vakuum eingeengt. Den Rückstand kristallisiert man aus Ether/Petrolether.
Ausbeute: 1,0 g (61% der Theorie) gelblicher Feststoff
Schmp.: 162 °C

A 7

Methyl-(E)-7-[1-butyl-5-(4-fluorphenyl)-7-isopropyl-2,4-dioxo-1,2,3,4-tetrahydro-pyrido(2,3-d)pyrimidin-6-yl]-5-hydroxy-3-oxo-hept-6-enoat

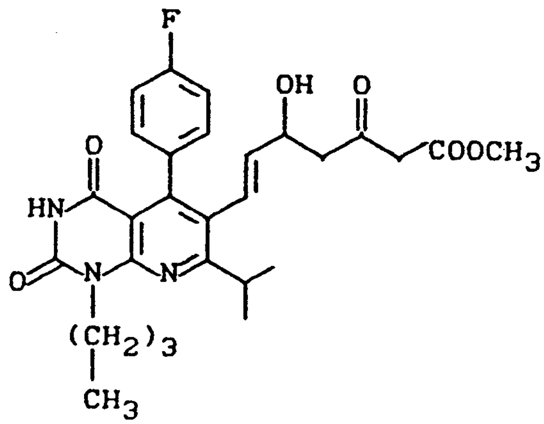

Zu einer Suspension von 0,2 g (6,6 mmol) 80%igem Natriumhydrid in 8 ml wasserfreiem Tetrahydrofuran gibt man bei 0 -5 °C tropfenweise 0,7 g (6 mmol) Acetessigsäuremethylester. Nach 15 min tropft man innerhalb von 10 min 4,9 ml (8 mmol) 15%iges Butyllithium in Hexan zu und hält weitere 15 min bei 0 °C. Dann werden 0,82 g (2 mmol) der Verbindung aus A 6 und 10 ml Tetrahydrofuran zugegeben und 1 h bei 0 - 5 °C gerührt. Anschließend versetzt man vorsichtig mit 1,2 g (20 mmol) Essigsäure in 20 ml Wasser, extrahiert dreimal mit Essigester, wäscht die organische Phase mit Kochsalzlösung, trocknet über Natriumsulfat und engt im Vakuum ein. Der Rückstand wird an 40 g Kieselgel 230-400 mesh (Ø 3 cm) mit Petrolether/Essigester (2:1) bis (1:3) chromatographiert.
Ausbeute: 0,14 g (13% der Theorie) gelbes Öl.
$R_f$ = 0,3 (Petrolether/Essigester 1:1)

A 8

6-Ethoxycarbonyl-5-(4-fluorphenyl)-7-isopropyl-1-methyl-2,4-dioxo-1,2,3,4,5,8-hexahydro-pyrido(2,3-d)-pyrimidin

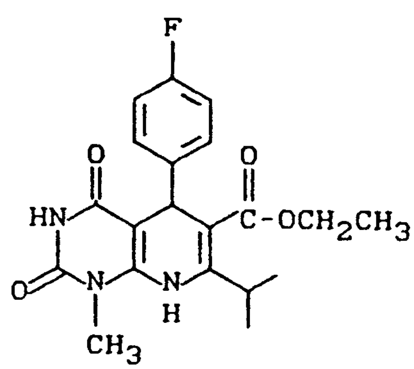

39,5 g (0,28 mol) 6-Amino-1-methyl-uracil und 147,8 g (0,56 mol) der Verbindung aus A 1 werden 4 h auf 180 °C erhitzt. Dann gibt man weitere 73,9 g (0,28 mol) der Verbindung aus A 1 zu und erhitzt nochmals 4 h. Nach dem Abkühlen wird mit 400 ml Methanol ausgerührt und abgesaugt.

Ausbeute: 78,2 g (72% der Theorie) gelblicher Feststoff
Schmp.: 277 °C

A 9

6-Ethoxycarbonyl-5-(4-fluorphenyl)-7-isopropyl-1-methyl-2,4-dioxo-1,2,3,4-tetrahydro-pyrido(2,3-d)pyrimidin

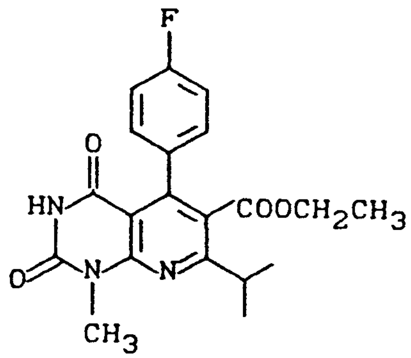

Zu einer Suspension von 19,4 g (50 mmol) der Verbindung aus A 8 in 1 l Dichlormethan gibt man 11,35 g (50 mmol) 2,3-Dichlor-4,5-dicyano-benzochinon und rührt 2 h bei Raumtemperatur. Es wird dreimal mit Wasser gewaschen, die organische Phase mit Natriumsulfat und Aktivkohle behandelt, filtriert und zur Trockne eingeengt. Es bleiben 17,5 g (91% der Theorie) farbloser Feststoff.
Schmp.: 214 °C

A 10 und A 11

5-(4-Fluorphenyl)-6-hydroxymethyl-7-isopropyl-1-methyl-2,4-dioxo-1,2,3,4-tetrahydro-pyrido(2,3-d)pyrimidin

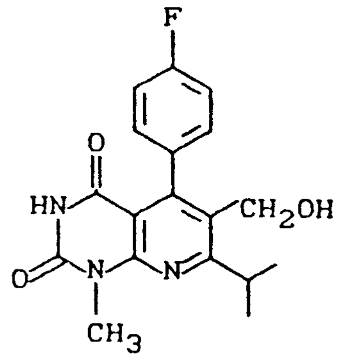

(10)

5-(4-Fluorphenyl)-6-hydroxymethyl-7-isopropyl-1-methyl-4-oxo-1,2,3,4-tetrahydro-pyrido(2,3-d)pyrimidin

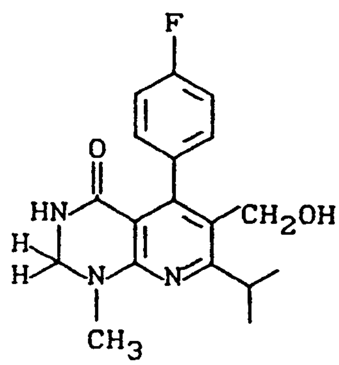

(11)

Aus 13,5 g (35 mmol) der Verbindung aus A 9 und 85,5 ml (128 mmol) einer 1,5 M Lösung von Diisobutylaluminiumhydrid in Toluol erhält man analog dem Verfahren aus A 4 ein Produktgemisch aus den oben angegebenen Verbindungen 10 und 11, das in einer Säule (Ø 6 cm) an 400 g Kieselgel 230 - 400 mesh mit einem Gradienten aus Petrolether/Essigester (2:1) bis (1:3) getrennt wird.

| | |
|---|---|
| $R_f$: | (Petrolether/Essigester, 1:1)A 10 = 0,5 |
| | A 11 = 0,2 |
| Ausbeute: | A 10 = 2,4 g (20% der Theorie) farbloser Feststoff |
| | A 11 = 1,9 g (17% der Theorie) farbloser Feststoff |
| Fp.: | A 10 = 208 °C |
| | A 11 = 197 °C |

A 12

5-(4-Fluorphenyl)-6-formyl-7-isopropyl-1-methyl-2,4-dioxo-1,2,3,4-tetrahydro-pyrido(2,3-d)pyrimidin

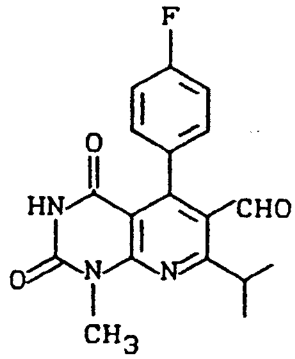

Analog dem Verfahren für A 5 erhält man aus 2,1 g (6 mmol) der Verbindung aus A 10 in 320 ml Dichlormethan mit 2,4 g Aluminiumoxid und 5,2 g (24 mmol) Pyridiniumchlorochromat 1,4 g (68% der Theorie) der Titelverbindung als farblose Kristalle.
Schmp.: 217 °C

A 13

(E)-3-5-(4-Fluorphenyl)-7-isopropyl-1-methyl-2,4-dioxo-1,2,3,4-tetrahydro-pyrido(2,3-d)pyrimidin-6-yl]prop-2-enal

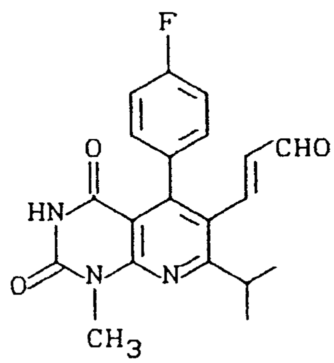

Analog der Vorschrift für A 6 werden 0,25 g (8,4 mmol) 80%iges Natriumhydrid, 1,2 g (4,2 mol) Diethyl-2-(cyclohexylamino)-vinyl-phosphonat und 1,2 g (3,5 mmol) der Verbindung aus A 12 in insgesamt 36 ml wasserfreiem Tetrahydrofuran umgesetzt, wobei 1 h am Rückfluß gekocht wird.
Ausbeute: 1,2 g (93% der Theorie) gelbliche Kristalle
Schmp.: 202 °C

A 14

5-(4-Fluorphenyl)-6-formyl-7-isopropyl-1-methyl-4-oxo-1,2,3,4-tetrahydro-pyrido(2,3-d)pyrimidin

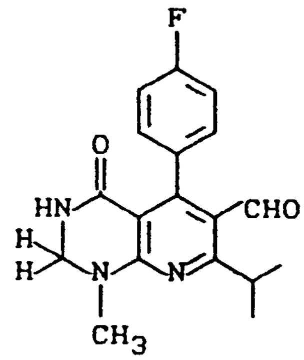

Analog der Vorschrift für A 5 erhält man aus 1,72 g (5 mmol) der Verbindung aus A 11 ein Gemisch, das durch Säulenchromatographie an Kieselgel mit Dichlormethan/Methanol 40:1 getrennt wird.
Ausbeute: 0,83 g (51% der Theorie) farblose Kristalle Schmp.: 211 °C aus Dichlormethan/Ether
$R_f$ = 0,33 (Chloroform/Methanol 20:1)
Als Nebenprodukt werden 0,55 g (34% der Theorie) 5-(4-Fluorphenyl)-6-formyl-7-isopropyl-1-methyl-4-oxo-1,4-dihydro-pyrido(2,3-d)pyrimidin als farbloser Schaum erhalten.
$R_f$ = 0,24 (Chloroform/Methanol 20:1)
$^1$H-NMR ($CDCl_3$): $\delta$ = 6,95 - 7,1 (m, 4H); 6,2 (d, 1H); 5,18 (dd, 1H); 4,25 (m, 1H); 4,07 (m, 1H); 3,75 (s, 3H); 3,6 (b, 1H); 3,29 (m, 1H); 3,12 (s, 3H); 2,4 (b, 1H); 2,4 (m, 2H); 1,15 -1,45 (m, 2H); 1,15 (m, 6H).

A 15

E/Z-1-(4-Fluorphenyl)-2-methoxycarbonyl-4-methyl-pent-1-en-3-on

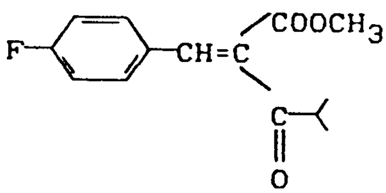

Analog der Vorschrift für A 1 erhält man aus 496,5 g (4 mol) 4-Fluorbenzaldehyd und 576,7 g (4 mol) Isobutyrylessigsäuremethylester in 1 l Isopropanol mit 22,5 ml Piperidin und 13,5 ml Eisessig 840,7 g (84% der Theorie) der Titelverbindung als gelbliches Öl.
Sdp.: 150 - 152 °C (4 mbar)

A 16

(E)-3-[1-Ethyl-5-(4-fluorphenyl)-7-isopropyl-2,4-dioxo-1,2,3,4-tetrahydro-pyrido(2,3-d)pyrimidin-6-yl]prop-2-enal

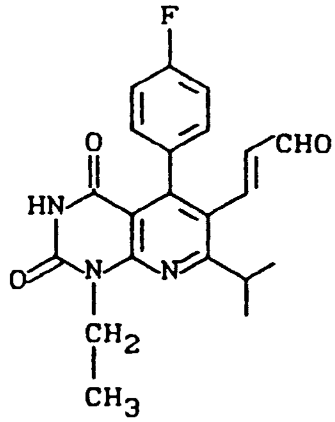

Ausgehend von der Verbindung aus A 15 und 6-Amino-1-ethyl-uracil wird die Verbindung analog den Vorschriften für A 8, A 9, A 4, A 12 und A 13 als gelbliche Kristalle erhalten.
Schmp.: 208 °C

A17

E-3-[1-Ethyl-5-(4-fluorphenyl)-7-isopropyl-3-methyl-2,4-dioxo-1,2,3,4-tetrahydro-pyrido(2,3-d)pyrimidin-6-yl]-prop-2-enal

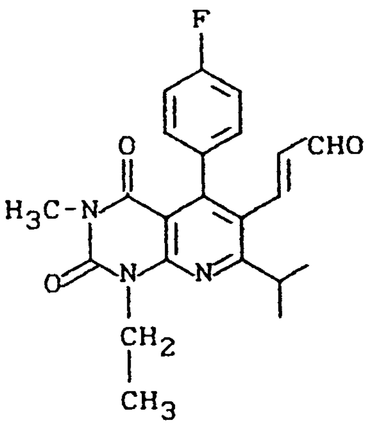

Zu einer Suspension von 0,18 g (6 mmol) 80%igem Natriumhydrid in 10 ml wasserfreiem Tetrahydrofuran tropft man unter Argon und Eiskühlung eine Lösung von 1,9 g (5 mmol) der Verbindung aus A 16 in 15 ml Tetrahydrofuran. Nach 20 min gibt man 1,1 ml (18 mmol) Methyliodid zu und rührt über Nacht bei 50 °C. Es wird vorsichtig mit 50 ml Wasser versetzt, zweimal mit Essigester extrahiert, die vereinigten organischen Phasen mit Kochsalzlösung gewaschen, über Natriumsulfat getrocknet und zur Trockne eingeengt. Säulenchromatographie (Ø 3 cm) an 50 g Kieselgel (230-400 mesh) mit Petrolether/Essigester (5:1) ergibt 1,0 g (51% der Theorie) gelblichen amorphen Feststoff.
UV (MeOH): λ max. = 304 nm

A 18

5-(4-Fluorphenyl)-7-isopropyl-6-methoxycarbonyl-2,4-dioxo-1,2,3,4,5,8-hexahydro-pyrido(2,3-d)pyrimidin

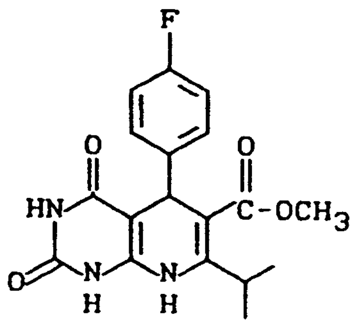

12,7 g (0,1 mol) 6-Amino-pyrimidin-2,4(1H,3H)-dion und 50 g (0,2 mol) der Verbindung aus A 15 werden in 500 ml Dimethylformamid über Nacht bei 140 °C gerührt. Man filtriert heiß von wenig Niederschlag ab, gießt auf 1 l Eiswasser und extrahiert dreimal mit Essigester. Die organischen Phasen werden im Vakuum eingeengt und der Rückstand in Methanol ausgerührt.
Ausbeute: 22,8 g (64% der Theorie) gelbliche Kristalle
Schmp.: 274 °C

A 19

5-(4-Fluorphenyl)-6-hydroxymethyl-7-isopropyl-2,4-dioxo-1,2,3,4-tetrahydro-pyrido(2,3-d)pyrimidin

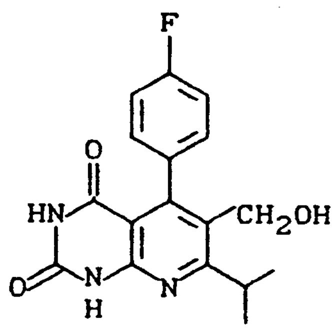

Analog der Vorschrift für A 9 und A 4, wobei hier insgesamt 6,5 mol-Equivalent an Diisobutylaluminiumhydrid verwendet werden, wird die Titelverbindung hergestellt.
Ausbeute: 1,4 g (20% der Theorie) farblose Kristalle
Schmp.: 194 °C (Semimethanolat)

A 20

5-(4-Fluorphenyl)-6-formyl-7-isopropyl-2,4-dioxo-1,2,3,4-tetrahydro-pyrido(2,3-d)pyrimidin

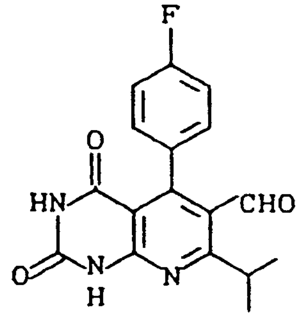

4,94 g (14,3 mmol) der Verbindung aus A 19, 3,1 g neutrales Aluminiumoxid und 6,45 g (30 mmol) Pyridiniumchlorochromat werden analog A 5 umgesetzt.
Ausbeute: 3,25 g (69% der Theorie) gelblicher Feststoff
Schmp.: 232 °C (aus Ether)

A 21

(E)-3-[5-(4-Fluorphenyl)-7-isopropyl-2,4-dioxo-1,2,3,4-tetrahydro-pyrido(2,3-d)pyrimidin-6-yl]prop-2-enal

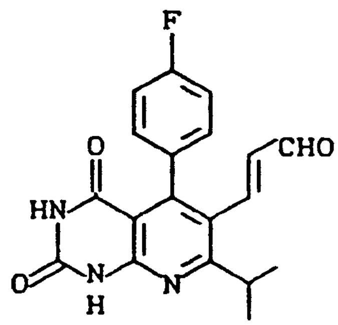

Analog dem Verfahren für A 6 wird aus 0,9 g (30 mmol) 80%igem Natriumhydrid, 2,8 g (10,8 mmol) Diethyl-2-(cyclohexylamino)-vinyl-phosphonat und 2,95 g (9 mmol) der Verbindung aus A 26 die Titelverbindung hergestellt.
Ausbeute: 1,0 g (31% der Theorie) gelbliche Kristalle
Schmp.: 255 °C

A 22

Methyl-(E)-7-[5-(4-fluorphenyl)-7-isopropyl-2,4-dioxo-1,2,3,4-tetrahydro-pyrido(2,3-d)pyrimidin-6-yl]-5-hydroxy-3-oxo-hept-6-enoat

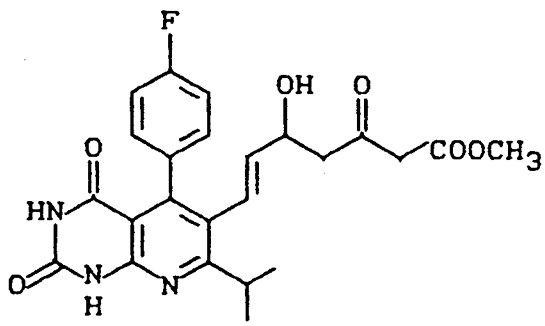

0,28 g (9,3 mmol) 80%iges Natriumhydrid, 0,97 g (8,4 mmol) Acetessigsäuremethylester, 8,6 ml (14 mmol) 15%iges Butyllithium und 0,99 g (2,8 mmol) der Verbindung aus A 21 werden analog der Vorschrift für A 7 umgesetzt.
Ausbeute: 1,4 g Rohprodukt

A 23

E, 2-1-Cyclopropyl-3-(4-fluorphenyl)-2-methoxycarbonyl-prop-2-en-1-on

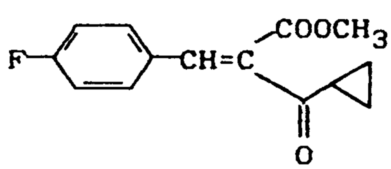

Analog A 1 erhält man aus 282,4 g (2 mol) 3-Cyclopropyl-3-oxo-propionsäuremethyl-ester [W.F. Berkowitz, A.A. Ozorio, J. Org. Chem. 36, 3787-92 (1971)] und 260,4 g (2,1 mol) 4-Fluorbenzaldehyd 43,2 g (87 %) der Titelverbindung vom Kp. (140-48 °C/0,2 mbar).

A 24

(E)-3-[7-Cyclopropyl-1-ethyl-5-(4-fluorphenyl)-2,4-dioxo-1,2,3,4-tetrahydro-pyrido(2,3-d)pyrimidin-6-yl]prop-2-enal

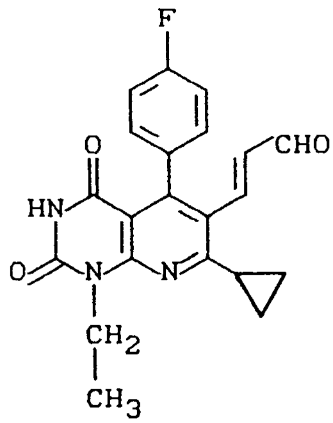

Ausgehend von 6-Amino-1-ethyl-uracil und der Verbindung aus B 14 wird analog zu den Vorschriften der A 18, A 9, A 4, A5 und A 13 die Titelverbindung hergestellt.
Schmelzpunkt: 236 ° C
Die in Tabelle aufgeführten Verbindungen wurden in Analogie zu den Vorschriften der A 18, A 9, A 4 und A 5 ausgehend von den Verbindungen der A 1 oder A 15 hergestellt:

Tabelle 2:

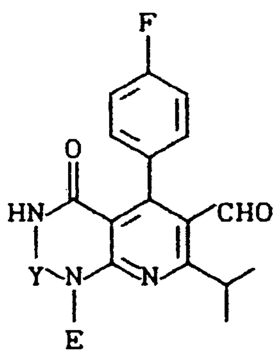

| Beispiel-Nr. | Y | E | Schmp. (° C) |
|---|---|---|---|
| A 25 | 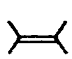 | 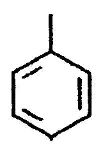 | 253° C |
| A 26 | 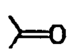 | $-(CH_2)_2CH_2$ | 156° C |
| A 27 | 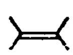 | $-CH_2CH_3$ | 217° C |

A 28

E-3-[7-Cyclopropyl-1-ethyl-5(4)-fluorphenyl)-3-methyl-2,4-dioxo-1,2,3,4-tetrahydro-pyrido(2 3-d)pyrimidin-6-yl]prop-2-enal

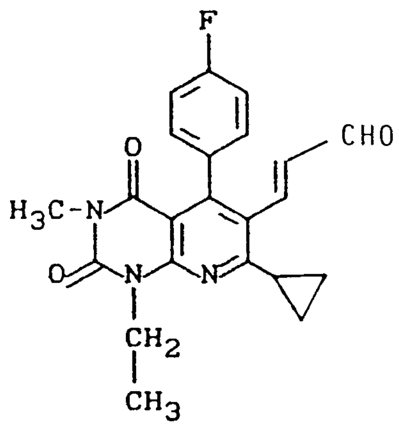

Die Verbindung wird analog der Vorschrift für A 17 aus der Verbindung des A 24 hergestellt.
Ausbeute: 56 % der Theorie, Schmelzpunkt 147 ° C.

A 29

1-Allyl-5-(4-fluorphenyl)-6-formyl-7-isopropyl-2,4-dioxo-1,2,3,4-tetrahydro-pyrido(2,3-d)pyrimidin

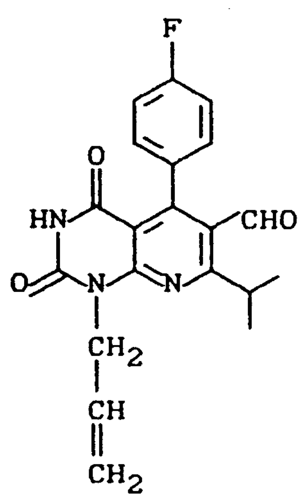

0,2 g (6,5 mmol) 80 %iges Natriumhydrid werden unter Eiskühlung zu einer Suspension von 2,13 g (6,5 mmol) der Verbindung aus A 20 in 20 ml Dimethylformamid gegeben. Es wird 10 min bei Raumtemperatur gerührt bis alles gelöst ist.

Dann tropft man 0,79 g (6,5 mmol) Allylbromid zu und rührt 1h bei Raumtempeatur. Man gießt auf 50 ml Eiswasser und extrahiert zweimal mit 50 ml Essigester. Die organischen Phasen werden mit gesättigter Kochsalzlösung gewaschen, über Natriumsulfat getrocknet und eingeengt. Den Rückstand chromatographiert man an 60 g Kieselgel (230-400 mesh), Säulendurchmesser 4 cm mit Petrolether/Essigester (2:1).
Ausbeute: 0,95 g (40 %) farblose Kristalle vom Schmelzpunkt 154 ° C (aus Ether, Petrolether).

A 30

5-(4-Fluorphenyl)-6-formyl-1,6-diisopropyl-2,4-dioxo-1,2,3,4-tetrahydro(2,3-d)pyrimidin

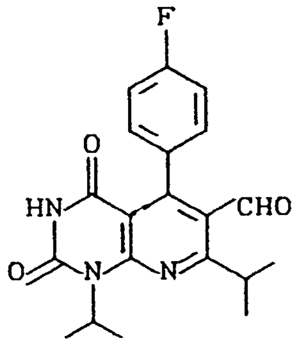

Die Verbindung erhält man aus der Verbindung des A 20 und Isopropyliodid analog dem Verfahren für A 24 , wobei 18h bei 50 ° C gehalten wird.
Ausbeute: 36 % der Theorie, Schmelzpunkt 197 ° C.

A 31

1-Benzyl-5-(4-fluorphenyl)-6-formyl-7-isopropyl-2,4-dioxo-1,2,3,4-tetrahydro(2,3-d)pyrimidin

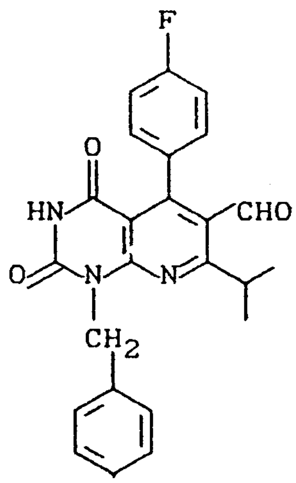

Analog der Vorschrift von A 29 aus der Verbindung aus A 20 und Benzylbromid.
Ausbeute: 37 % der Theorie, Schmelzpunkt 237 ° C.

A 32

1,7-Dibenzyl-5-(4-fluorphenyl)-6-formyl-2,4-dioxo-1,2,3,4-tetrahydro(2,3-d)pyrimidin

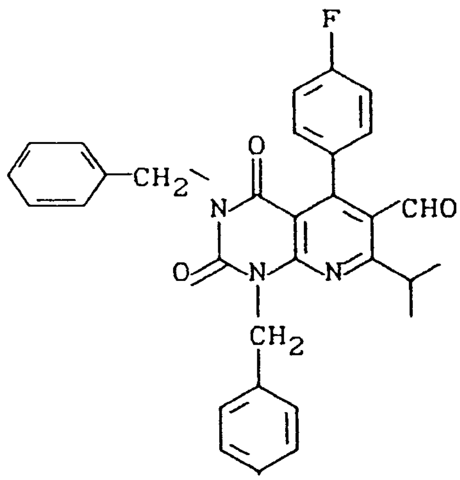

Die Verbindung erhält man bei der Herstellung von A 31 als zweites Produkt. Sie wird durch Kieselgelchromatographie mit Petrolether/Essigester isoliert.
Ausbeute: 24 % der Theorie, amorpher farbl. Feststoff
$^1$H-NMR ($CDCl_3$): $\delta$ = 1,3 (d, 6H), 3,95 (hept. 1H), 5,13 (s, 2H), 5,67 (s, 2H), 7,15-7,5 (m, 14 H), 9,6 (s, 1H).

A 33

4-Fluorbenzoylessigsäureethylester

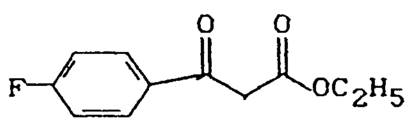

In einen Liter Diethylether p.a. werden 21,7 g (0,72 mol) Natriumhdyrid (80 %ig, 20 % Mineralöl) eingewogen und anschließend 85,5 g (127 ml, 0,72 mol) Kohlensäurediethylester zugegeben. In dieser Lösung wird in der Siedehitze über einen Zeitraum von 4 Stunden eine Lösung von 100 g (0,72 mol) 4-Fluoracetophenon in 300 ml Diethylether zugetropft (kräftiger, mechanischer Rührer erforderlich; es bildet sich ein zäher Brei!). Danach wird eine weitere Stunde am Rückfluß gekocht, dann auf ca. 5°C abgekühlt und bei dieser Temperatur unter $N_2$ zunächst eine Lösung aus 50 ml Essigsäure und 100 ml $Et_2O$ zugetropft. Anschließend etwa 500 ml $H_2O$ zugetropft und die organische Phase abgetrennt. Die Wasserphase wird nochmals mit $Et_2O$ extrahiert (2 x 400 ml), die vereinigten etherischen Phasen mit $NaHCO_3$-Lösung gewaschen, über $MgSO_4$ getrocknet und eingeengt. Der Rückstand wird über eine kurze Vigreux-Kolonne destilliert.
Ausbeute: 93 g (60 %) Kp 0,4 mm 99-102°C.

A 34

2-(4-Fluorbenzoyl)-4-methyl-pent-2-en-carbonsäureethylester

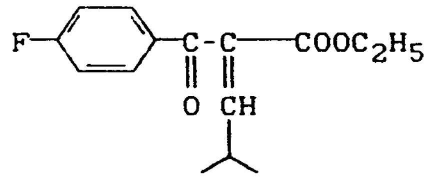

Eine Lösung von 210 g (1 mol) 4-Fluorbenzoylessigsäureethylester und 144 g (2 mol) 2-Methylpropanol werden in 100 ml Isopropanol mit 7 ml Pyridin und 5 ml Essigsäure über Nacht bei 50° gerührt. Nach vollständiger Umsetzung wird der Ansatz bei ca. 15 Torr eingeengt und das Rohprodukt (270 g ~ 85 % der Theorie) ohne weitere Reinigung weiter umgesetzt.

A 35

1-Ethyl-7-(4-fluorphenyl)-6-formyl-5-isopropyl-2,4-dioxo-1,2,3,4-tetrahydro(2,3-d)pyrimidin

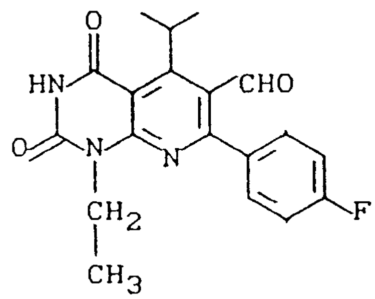

Ausgehend von der Verbindung aus A 34 mit 6-Amino-1-Ethyl-uracil wird analog zu den Reaktionen aus A 18, 3, 4 und 5 die Titelverbindung hergestellt.
Schmelzpunkt: 216°C

B) ENDPRODUKTE

B 1

Methyl-erythro-(E)-7-[1-butyl-5-(4-fluorphenyl)-7-isopropyl-2,4-dioxo-1,2,3,4-tetrahydro-pyrido(2,3-d)-pyrimidin-6-yl]-3,5-dihydroxy-hept-6-enoat

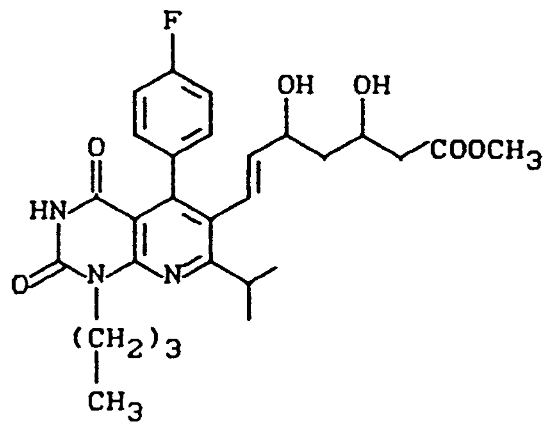

Zu einer Lösung von 126 mg (0,24 mmol) der Verbindung aus A 7 in 4 ml wasserfreiem Tetrahydrofuran gibt man 0,29 ml einer 1 molaren Lösung von Triethylboran in Tetrahydrofuran und bläst 5 min Luft durch die Lösung. Bei -78 °C gibt man 11,4 mg (0,3 mmol) Natriumborhydrid zu, dann tropft man 0,5 ml Methanol zu und hält 1 h bei -78 °C bis -75 °C. Man läßt auf Raumtemperatur erwärmen, wobei ab -30 °C 1 ml 30%iges Wasserstoffperoxid und 20 ml Wasser zugesetzt werden. Es wird viermal mit Essigester extrahiert, die vereinigten organischen Phasen mit Kochsalzlösung gewaschen, über Natriumsulfat getrocknet und eingeengt. Der Rückstand wird an 15 g Kieselgel (230 - 400 mesh) in einer Säule (Ø 2 cm) mit Petrolether/Essigester (1:1) chromatographiert.
Ausbeute: 52 mg (41% der Theorie) farbloser Feststoff
Schmp.: 171 °C

B 2

Erythro-(E)-7-[1-butyl-5-(4-fluorphenyl)-7-isopropyl-2,4-dioxo-1,2,3,4-tetrahydro-pyrido(2,3-d)pyrimidin-6-yl]-3,5-dihydroxy-hept-6-en-carbonsäure-Natriumsalz

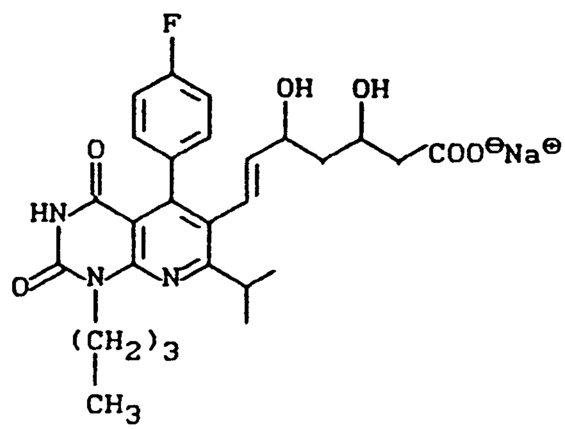

Eine Lösung von 27 mg (0,05 mmol) der Verbindung aus B 1 in 1 ml Tetrahydrofuran wird mit 0,5 ml (0,05 mmol) 0,1 molarer Natronlauge 2 h bei Raumtemperatur gerührt. Es wird eingeengt und über Phosphorpentoxid im Vakuum getrocknet.
Ausbeute: 22 mg (82% der Theorie) farbloser Feststoff.

FAB-MS: 536 (M + H), 558 (M + Na)

B 3

Methyl-erythro-(E)-7-[5-(4-fluorphenyl)-7-isopropyl-1-methyl-2,4-dioxo-1,2,3,4-tetrahydro-pyrido(2,3-d)-pyrimidin-6-yl]-3,5-dihydroxy-hept-6-enoat

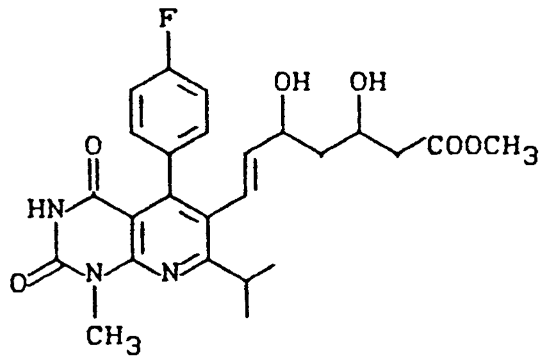

Analog der Vorschriften für A 7 und B 1 erhält man aus 1,1 g (3 mmol) der Verbindung aus A 13 140 mg (10% der Theorie) der Titelverbindung als farblose Kristalle.
Schmp.: 168 ° C (aus Ether/Petrolether)

B 4

Methyl-erythro-(E)-7-[5-(4-fluorphenyl)-7-isopropyl-1-methyl-4-oxo-1,2,3,4-tetrahydro-pyrido(2,3-d)pyrimidin-6-yl]-3,5-dihydroxy-hept-6-enoat

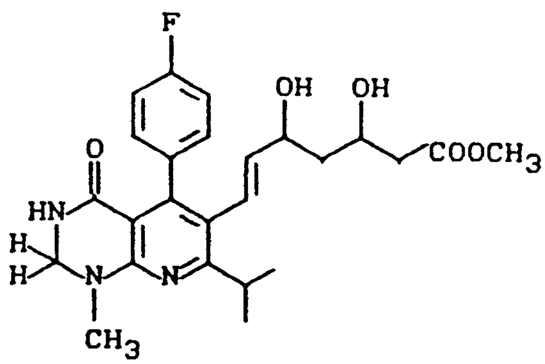

Analog den Vorschriften für A 13, A 7 und B 1 aus 0,82 g (2,5 mmol) der Verbindung aus A 14 115 mg (10% der Theorie) farbloser Kristalle erhalten.
Schmp.: 139 ° C (Dichlormethan/Ether)

B 5

Methyl-erythro-(E)-7-[1-ethyl-5-(4-fluorphenyl)-7-isopropyl-2,4-dioxo-1,2,3,4-tetrahydro-pyrido(2,3-d)-pyrimidin-6-yl]-3,5-dihydroxy-hept-6-enoat

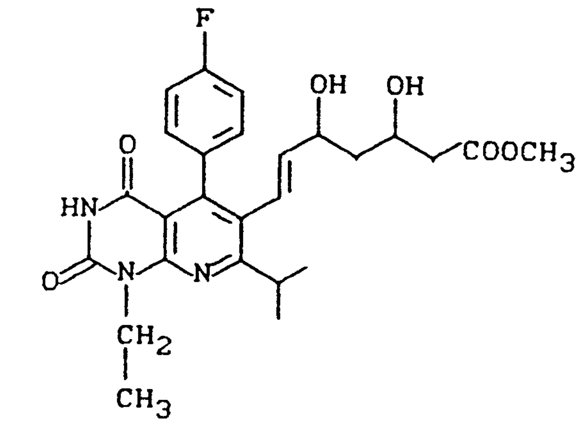

Analog den Vorschriften für A 7 und B 1 erhält man aus 1,15 g (3 mmol) der Verbindung aus A 16 (33% der Theorie) der Titelverbindung als farblosen Feststoff.
Schmp.: 155 °C

B 6

Methyl-erythro-(E)-7-[1-ethyl-5-(4-fluorphenyl)-7-isopropyl-3-methyl-2,4-dioxo-1,2,3,4-tetrahydro-pyrido(2,3-d)pyrimidin-6-yl]-3,5-dihydroxy-hept-6-enoat

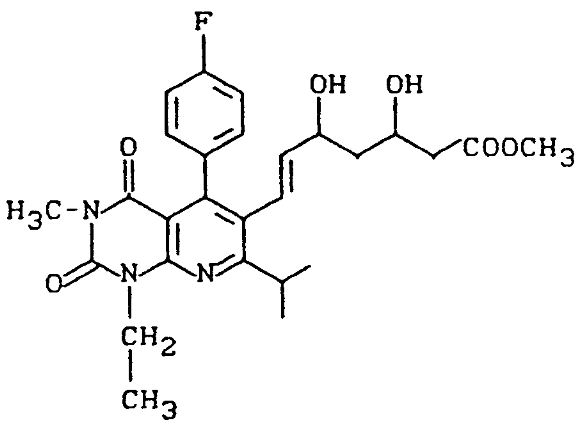

Analog der Vorschriften für A 7 und B 1 erhält man aus 1,0 g (2,5 mmol) der Verbindung aus A 17 0,38 g (30% der Theorie) der Titelverbindung als farblose Kristalle.
Schmp.: 123 °C aus Ether

B 7

Methyl-erythro-(E)-7-[5-(4-fluorphenyl)-7-isopropyl-2,4-dioxo-1,2,3,4-tetrahydro-pyrido(2,3-d)pyrimidin-6-yl]-3,5-dihydroxy-hept-6-enoat

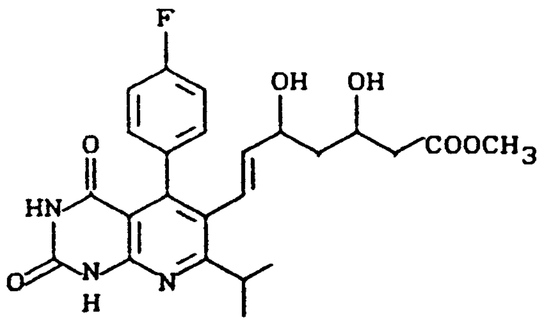

Die Titelverbindung wird analog der Vorschrift für B 1 aus 1,4 g (2,8 mmol) des Produktes aus A 22 erhalten.
Ausbeute: 190 mg (14% der Theorie) farbloser Feststoff Schmp.: 148 °C
Analog der Vorschrift für B 2 wurden aus den Verbindungen der B 3, B 4, B 5, B 6, B 7, A 27, B 18 und B 2 die in dem Tabelle aufgeführten Beispiele hergestellt.

Tabelle 1:

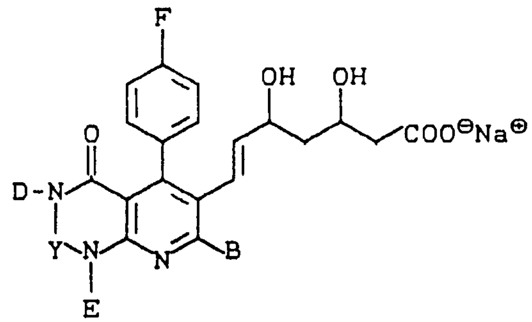

| Bsp | B | D | E | Y | Schmp. (° C) | FAB-MS |
|---|---|---|---|---|---|---|
| B 8 | 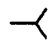 | H | $CH_3$ | 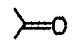 | 179 (Zers.) | 494 (M+H); 508 (M+Na) |
| B 9 | 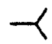 | H | $CH_3$ | 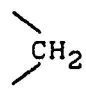 | 183 (Zers.) | 480 (M+H); 502 (M+Na) |
| B 10 | 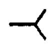 | H | $CH_2CH_3$ | 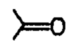 | 122 (Zers.) | 508 (M+H); 530 (M+Na) |
| B 11 | 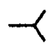 | $CH_3$ | $CH_2CH_3$ | 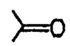 | 117 (Zers.) | 522 (M+H); 544 (M+Na) |
| B 12 | 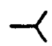 | H | H | 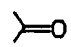 | 203 (Zers.) | 480 (M+H); 502 (M+Na) |
| B 13 | 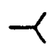 | H | $-CH_2CH_3$ | 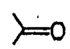 | 184° (Zers.) | 506 (M+H); 528 (M+Na) |
| B 14 | 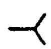 | $CH_3$ | $-CH_2CH_3$ | 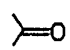 | 148° (Zers.) | 520 (M+H); 542 (M+Na) |
| B 15 | 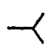 | H | $-CH_2-$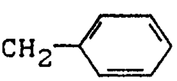 | 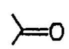 | amorph | 570 (M+H); 592 (M+Na) |

B 16

trans-6-{2-[1-ethyl-5-(4-fluorphenyl)-7-isopropyl-3-methyl-2,4-dioxo-1,2,3,4-tetrahydro-pyrido(2,3-d)pyrimidin-6-yl]-ethenyl}-4-hydroxy-3,4,5,6-tetrahydro-2H-pyran-2-one

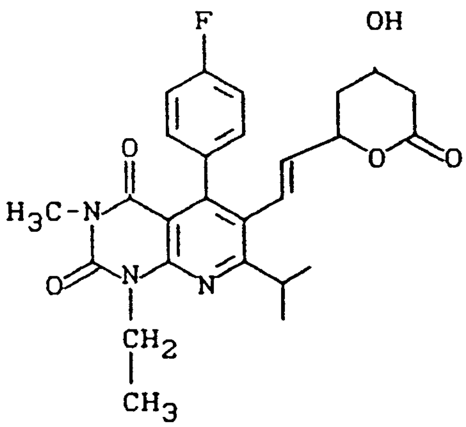

208 mg (0,4 mmol) der Verbindung aus B 11 werden in 10 ml Wasser gelöst, 0,4 ml (0,4 mmol) 1 N Salzsäure wird zugefügt und fünfmal mit Dichlormethan extrahiert. Die vereinigten organischen Phasen trocknet man über Magnesiumsulfat, engt im Vakuum ein und kocht den Rückstand in 15 ml Toluol 18 h am Wasserabscheider. Nach dem Einengen chromatographiert man an 20 g Kieselgel (230-400 mesh) in einer Säule (Durchmesser 2 cm) mit Petrolether/Essigester (1:1).
Ausbeute: 140 mg (73% der Theorie) amorpher farbloser Feststoff
$^1$H-NMR ($CDCl_3$): $\delta$ = 1,25 - 1,7 (m, 11H, CH($\underline{CH_3}$)$_2$, $CH_2$-$\underline{CH_3}$, O-CH-$\underline{CH_2}$-C(OH)); 2,03 (d, 1H, OH); 2,5 - 2,7 (m, 2H, $\underline{CH_2}$-C=O); 3,8 (s, 1H, N-$CH_3$); 3,9 (m, 1H, $\underline{CH}$($CH_3$)$_2$); 4,2 (m, 1H, $\underline{CH}$-O); 4,5 (q, 2H, N-$\underline{CH_2}$); 5,1 (m, 1H, $\underline{CH}$-O); 5,35 (dd, 1H, Olefin-H); 6,3 (d, 1H, Olefin-H); 6,95 - 7,15 (m, 4H, Aromaten-H).

B 17

Methyl-erythro-(E)-7[7-cyclopropyl-1-ethyl-5-(4-fluorphenyl)-2,4-dioxo-1,2,3,4-tetrahydropyrido(2,3-d)-pyrimidin-6-yl]-3,5-dihydroxy-hept-6-enoat

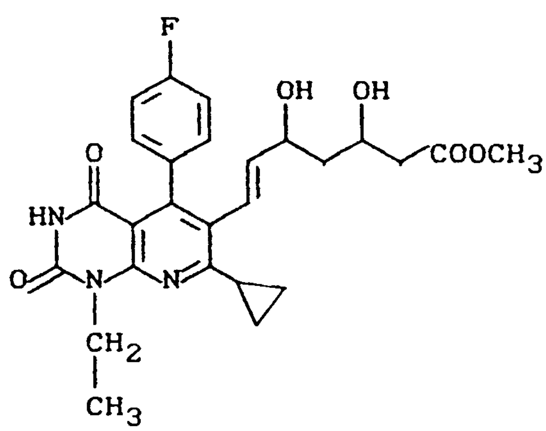

Die Titelverbindung wird in Analogie zu den Vorschriften der A 7 und B 1 aus der Verbindung des A 24 erhalten.
Ausbeute: 32 % der Theorie, Schmelzpunkt 123 °C (Dichlormethan, Petrolether).

Die in der Tabelle aufgeführten Verbindungen wurden in Analogie zu den Vorschriften der A 7, B 1 und A 13 hergestellt.

Tabelle 3:

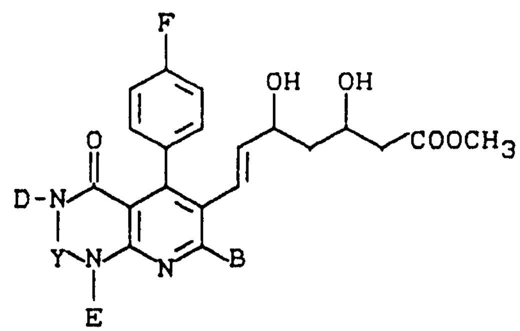

| Bsp. Nr. | B | D | E | Y | Schmp. (°C) |
|---|---|---|---|---|---|
| B 18 | 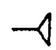 | $CH_3$ | $-C_2H_5$ | 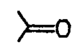 | amorph |
| B 19 | 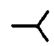 | H | $-CH_2-CH=CH_2$ | 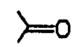 | 167 |
| B 20 | 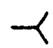 | H | 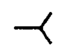 | 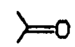 | 137 |
| B 21 | 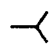 | H | $-CH_2-C_6H_5$ | 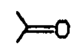 | 177 |
| B 22 | 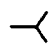 | $CH_2-C_6H_5$ | $-CH_2-C_6H_5$ | 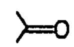 | 82 |
| B 23 | 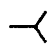 | H | $-C_6H_5$ | 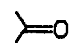 | 194 |
| B 24 | 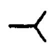 | H | $-CH_2-CH_2-CH_3$ | 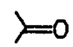 | 153 |
| B 25 | 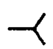 | H | $-C_2H_5$ | 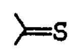 | amorph* |

* Bei der Herstellung von B 25 wird nicht mit Wasserstoffperoxid gearbeitet, sondern das Rohprodukt in der 200-fachen Menge Methanol 3h bei Raumtemperatur gerührt.

B 26

Methyl-erythro-(E)-7[1-ethyl-7-(4-fluorphenyl-5-isopropyl-2,4-Dioxo-1,2,3,4-tetrahydropyrido(2,3-d)pyrimidin-6-yl]-3,5-dihydroxy-hept-6-enoat

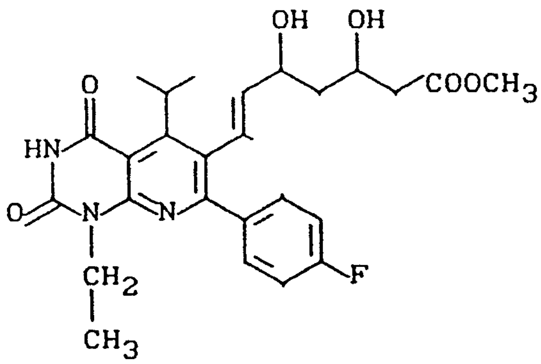

Die Titelverbindung wird analog zu den Vorschriften der A 7 und B 1 aus der Verbindung des B 25 erhalten. Ausbeute: 30 % der Theorie, Schmelzpunkt: 157 ° C.

Pharmakologisches Beispiel

Die Serum-Cholesterin-senkende Wirkung der erfindungsgemäßen Verbindungen auf die Blutcholesterinwerte von Hunden wurde in mehrwöchigen Fütterungsexperimenten gefunden. Dazu wurde die zu untersuchende Substanz über einen mehrwöchigen Zeitraum einmal täglich in einer Kapsel gesunden Beagle Hunden zusammen mit dem Futter p.o. gegeben. Dem Futter war außerdem während der gesamten Versuchsperiode, d.h. vor, während und nach der Applikationsperiode der zu untersuchenden Substanz Colestyramin (4 g/100 g Futter) als Gallensäuresequestrant beigemischt.

Zweimal wöchentlich wurde den Hunden vernöses Blut abgenommen und das Serumcholesterin mit einem handelsüblichen Testkit enzymatisch bestimmt. Die Serumcholesterinwerte während der Applikationsperiode wurden mit den Serumcholesterinwerten vor der Applikationsperiode (Kontrollen) verglichen.

**Patentansprüche**

**Patentansprüche für folgende Vertragsstaaten : AT, BE, CH, DE, FR, GB, GR, IT, LI, LU, NL, SE**

**1.** Substituierte Pyrido(2,3-d)pyrimidine der allgemeinen Formel (I)

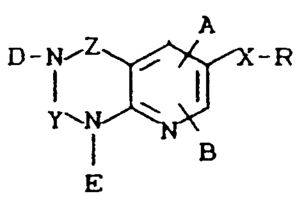

(I)

in welcher

| | |
|---|---|
| A | - für einen 5- bis 7-gliedrigen Heterocyclus steht, der bis zu 4 Heteroatome aus der Reihe Schwefel, Sauerstoff oder Stickstoff enthalten kann und der gegebenenfalls bis zu 3-fach gleich oder verschieden durch Halogen, Hydroxy, Trifluormethyl, geradkettiges oder verzweigtes Alkyl, Alkoxy oder Alkoxycarbonyl mit jeweils bis zu 8 Kohlenstoffatomen, Aryl mit 6 bis 10 Kohlenstoffatomen oder durch eine Gruppe der Formel $-NR^1R^2$ substituiert ist, worin |
| $R^1$ und $R^2$ | gleich oder verschieden sind und |

- Wasserstoff, Aryl oder Arylsulfonyl mit 6 bis 10 Kohlenstoffatomen, geradkettiges oder verzweigtes Alkyl oder Alkylsulfonyl mit bis zu 8 Kohlenstoffatomen bedeuten, wobei die letztgenannten Reste gegebenenfalls durch Aryl mit 6 bis 10 Kohlenstoffatomen substituiert sind, oder
- eine Gruppe der Formel $-COR^3$ bedeuten,
worin

$R^3$ - geradkettiges oder verzweigtes Alkyl oder Alkoxy mit bis zu 8 Kohlenstoffatomen oder Phenyl bedeutet,

- für Aryl mit 6 bis 10 Kohlenstoffatomen steht, das gegebenenfalls bis zu 5-fach gleich oder verschieden substituiert ist durch geradkettiges oder verzweigtes Alkyl, Alkylthio, Alkylsulfonyl, Alkoxy oder Alkoxycarbonyl mit jeweils bis zu 10 Kohlenstoffatomen, welche ihrerseits durch Trifluormethyl, Hydroxy, Alkoxy mit bis zu 6 Kohlenstoffatomen, Phenyl oder Phenoxy substituiert sein können, oder durch Aryl, Aryloxy, Arylthio oder Arylsulfonyl mit 6 bis 10 Kohlenstoffatomen, oder durch Halogen, Nitro, Cyano, Trifluormethyl, Benzyloxy oder eine Gruppe der Formel $-NR^1R^2$ substituiert ist,
oder
- für geradkettiges oder verzweigtes Alkyl mit jeweils bis zu 8 Kohlenstoffatomen steht,

B - für Cycloalkyl mit 3 bis 8 Kohlenstoffatomen steht,
- für Trifluormethyl oder geradkettiges oder verzweigtes Alkyl mit bis zu 12 Kohlenstoffatomen steht, das gegebenenfalls durch Halogen, Hydroxy, Cyano, Azido, Trifluormethyl, Alkylthio, Alkylsulfonyl oder Alkoxy mit jeweils bis zu 8 Kohlenstoffatomen oder durch Aryl, Aryloxy oder Arylthio mit 6 bis 10 Kohlenstoffatomen substituiert ist, wobei die Arylreste gegebenenfalls bis zu 3-fach gleich oder verschieden durch Halogen, Cyano, Trifluormethyl, Trifluormethoxy, geradkettiges oder verzweigtes Alkyl, Alkoxy, Alkylthio oder Alkylsulfonyl mit jeweils bis zu 8 Kohlenstoffatomen substituiert sein können, oder durch eine Gruppe der Formel $-NR^1R^2$ oder $-COR^3$ substituiert ist,
worin

$R^1$, $R^2$ und $R^3$ die oben angegebene Bedeutung haben,

- für Aryl mit 6 bis 10 Kohlenstoffatomen steht, das gegebenenfalls durch Halogen, Cyano, Nitro, Trifluormethyl, geradkettiges oder verzweigtes Alkyl, Alkoxy oder Alkoxycarbonyl mit jeweils bis zu 8 Kohlenstoffatomen oder Amino substituiert ist,

D - für Wasserstoff, Hydroxy oder
- Cycloalkyl mit 3 bis 8 Kohlenstoffatomen steht,
- für geradkettiges oder verzweigtes Alkyl mit jeweils bis zu 12 Kohlenstoffatomen steht, das gegebenenfalls durch Halogen, Hydroxy oder Alkoxy mit bis zu 8 Kohlenstoffatomen substituiert ist,

E - die oben angegebene Bedeutung von A hat und mit dieser gleich oder verschieden ist, oder
- für Wasserstoff oder
- Cycloalkyl mit 3 bis 8 Kohlenstoffatomen steht,
- für geradkettiges oder verzweigtes Alkyl mit bis 12 Kohlenstoffatomen steht, das gegebenenfalls durch Halogen, geradkettiges oder verzweigtes Alkenyl mit bis zu 8 Kohlenstoffatomen, Aryl mit 6 bis 10 Kohlenstoffatomen, durch einen 5- bis 7-gliedrigen Heterocyclus mit bis zu 4 Heteroatomen aus der Reihe Stickstoff, Sauerstoff oder Schwefel oder durch eine Gruppe der Formel $-NR^1R^2$, $-OR^4$, $-COR^5$ oder $S(O)_n-R^6$ substituiert ist,
worin

$R^1$ und $R^2$ die oben angegebene Bedeutung haben,

$R^4$ - Wasserstoff oder
- geradkettiges oder verzweigtes Alkyl mit bis zu 10 Kohlenstoffatomen bedeutet, das gegebenenfalls durch Hydroxy, Trialkylsilyl mit bis zu 10 Kohlenstoffatomen im Alkylteil, Halogen oder Aryl mit 6 bis 10 Kohlenstoffatomen substituiert ist,
- Trialkylsilyl mit bis zu 10 Kohlenstoffatomen oder Cycloalkyl mit 3 bis 8 Kohlenstoffatomen oder Aryl mit 6 bis 10 Kohlenstoffatomen bedeutet, welches seinerseits durch Halogen, Cyano, Nitro oder Amino substituiert sein kann, oder

- eine Gruppe der Formel $-COR^7$ bedeutet,
worin

$R^7$ - geradkettiges oder verzweigtes Alkyl mit bis zu 8 Kohlenstoffatomen, Aryl mit 6 bis 10 Kohlenstoffatomen oder die Gruppe $-NR^1R^2$ bedeutet,
worin

$R^1$ und $R^2$ die oben angegebene Bedeutung haben,

$R^5$ - Wasserstoff oder geradkettiges oder verzweigtes Alkyl mit bis zu 8 Kohlenstoffatomen bedeutet, das gegebenenfalls durch Hydroxy, Phenyl, Halogen oder Cyano substituiert ist,
- Aryl mit 6 bis 10 Kohlenstoffatomen bedeutet, welches durch Halogen, Amino, Hydroxy, Nitro oder Cyano substituiert sein kann, oder
- eine Gruppe der Formel $-NR^1R^2$ oder $-OR^4$ bedeutet,
worin

$R^1$, $R^2$ und $R^4$ die oben angegebene Bedeutung haben,

n - eine Zahl 0 oder 2 bedeutet,

$R^6$ - geradkettiges oder verzweigtes Alkyl mit bis zu 10 Kohlenstoffatomen bedeutet, das durch Halogen, Hydroxy, Phenyl oder eine Gruppe der Formel $-NR^1R^2$ substituiert sein kann,
worin

$R^1$ und $R^2$ die oben angegebene Bedeutung haben,
- Aryl mit 6 bis 10 Kohlenstoffatomen bedeutet, das durch Halogen, Hydroxy, Cyano, Nitro oder Amino substituiert sein kann, oder
- eine Gruppe der Formel $-NR^1R^2$ bedeutet, wenn n für die Zahl 2 steht,
worin

$R^1$ und $R^2$ die oben angegebene Bedeutung haben,
oder

E - für eine Gruppe der Formel $-NR^1R^2$ oder $-OR^4$ steht,
worin

$R^1$, $R^2$ und $R^4$ die oben angegebene Bedeutung haben,

Y und Z gleich oder verschieden sind und
- für eine Gruppe der Formel

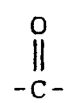

oder

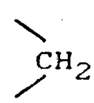

stehen,
oder

Y - für eine Thiocarbonylgruppe steht,

X - für eine Gruppe der Formel $-CH_2-CH_2-$ oder $-CH=CH-$steht,
und

R - für eine Gruppe der Formel

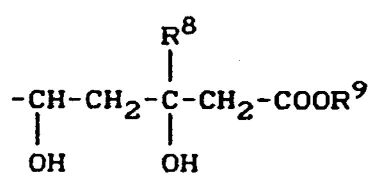

oder

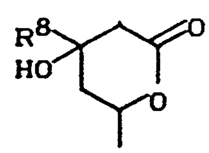

steht,
worin

$R^8$ - Wasserstoff oder geradkettiges oder verzweigtes Alkyl mit bis zu 10 Kohlenstoffatomen bedeutet
und

$R^9$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,

und deren Salze.

**2.** Substituierte Pyrido(2,3-d)pyrimidine nach Anspruch 1 der Formeln (Ia) und (Ib)

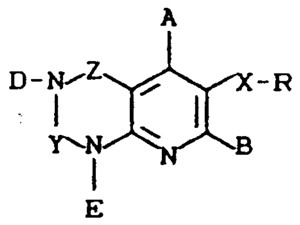

(Ia)

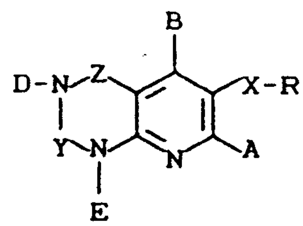

(Ib)

in welcher

A - für Thienyl, Furyl, Pyridyl oder Pyrimidyl steht, das gegebenenfalls bis zu 3-fach gleich oder verschieden durch Fluor, Chlor, Brom, Hydroxy, Trifluormethyl, geradkettiges oder verzweigtes Alkyl, Alkoxy oder Alkoxycarbonyl mit jeweils bis zu 6 Kohlenstoffatomen, Phenyl, oder durch eine Gruppe der Formel $-NR^1R^2$ substituiert ist,
worin

$R^1$ und $R^2$ gleich oder verschieden sind und
- Wasserstoff, Phenyl, Phenylsulfonyl, geradkettiges oder verzweigtes Alkyl oder Alkylsulfonyl mit bis zu 6 Kohlenstoffatomen, Benzyl oder Benzylsulfonyl bedeuten,
- eine Gruppe der Formel $-COR^3$ bedeuten,
worin

$R^3$ - geradkettiges oder verzweigtes Alkyl oder Alkoxy mit bis zu 6 Kohlenstoffatomen oder Phenyl bedeutet,
oder
- für Phenyl oder Naphthyl steht, das gegebenenfalls bis zu 4-fach gleich oder verschieden substituiert ist durch geradkettiges oder verzweigtes Alkyl, Alkoxy oder Alkoxycarbonyl mit jeweils bis zu 8 Kohlenstoffatomen, welche ihrerseits durch Trifluormethyl, Hydroxy, Alkoxy mit bis zu 4 Kohlenstoffatomen, Phenyl oder Phenoxy substituiert sein können, oder durch Phenyl, Phenoxy, Phenylthio, Phenylsulfonyl, Fluor, Chlor, Brom, Nitro, Cyano, Trifluormethyl, Benzyloxy oder durch eine Gruppe der Formel $-NR^1R^2$ substituiert ist,
oder
- für geradkettiges oder verzweigtes Alkyl mit bis zu 6 Kohlenstoffatomen steht,

B - für Cyclopropyl, Cyclobutyl, Cyclopentyl oder Cyclohexyl steht,
- für Trifluormethyl oder geradkettiges oder verzweigtes Alkyl mit bis zu 10 Kohlenstoffatomen steht, das gegebenenfalls durch Fluor, Chlor, Brom, Hydroxy, Cyano, Azido, Trifluormethyl, Methylthio, Methylsulfonyl, Alkoxy mit bis zu 6 Kohlenstoffatomen oder durch Phenyl, Phenyloxy oder Phenylthio substituiert ist, wobei die Phenylreste bis zu 2-fach gleich oder verschieden durch Fluor, Chlor, Brom, Cyano, Trifluormethyl, Trifluormethoxy, geradkettiges oder verzweigtes Alkyl, Alkoxy, Alkylthio oder Alkylsulfonyl mit jeweils bis zu 6 Kohlenstoffatomen substituiert sein können, oder
durch eine Gruppe der Formel $-NR^1R^2$ oder $-COR^3$ substituiert ist,
oder
- für Phenyl steht, das gegebenenfalls durch Fluor oder Chlor substituiert ist,

D - für Wasserstoff, Hydroxy oder
- Cyclopropyl, Cyclobutyl, Cyclopentyl oder Cyclohexyl steht,

- für geradkettiges oder verzweigtes Alkyl mit bis zu 10 Kohlenstoffatomen steht, das gegebenenfalls durch Fluor, Chlor, Brom, Hydroxy oder Alkoxy mit bis zu 6 Kohlenstoffatomen substituiert ist,

E die oben angegebene Bedeutung von A hat und mit dieser gleich oder verschieden ist, oder
- für Wasserstoff oder
- Cyclopropyl, Cyclobutyl, Cyclopentyl oder Cyclohexyl steht,
- für geradkettiges oder verzweigtes Alkyl mit bis zu 10 Kohlenstoffatomen steht, das gegebenenfalls durch Fluor, Chlor, Brom, geradkettiges oder verzweigtes Alkenyl mit bis zu 6 Kohlenstoffatomen, Phenyl, Pyrimidyl, Pyrrolyl, Pyrrolidinyl, Furyl oder Thiazolyl substituiert ist, oder durch eine Gruppe der Formel $-NR^1R^2$, $-OR^4$, $-COR^5$ oder $-S(O)_n-R^6$ substituiert ist,

worin

$R^1$ und $R^2$ die oben angegebene Bedeutung haben,

$R^4$
- Wasserstoff oder
- geradkettiges oder verzweigtes Alkyl mit bis zu 8 Kohlenstoffatomen bedeutet, das gegebenenfalls durch Hydroxy, Fluor, Chlor, Brom oder durch Phenyl substituiert ist, oder
- eine Gruppe der Formel $-COR^7$ bedeutet,

worin

$R^7$
- geradkettiges oder verzweigtes Alkyl mit bis zu 6 Kohlenstoffatomen, Phenyl oder eine Gruppe der Formel $-NR^1R^2$ bedeutet,

worin

$R^1$ und $R^2$ die oben angegebene Bedeutung haben,

$R^5$
- Wasserstoff, geradkettiges oder verzweigtes Alkyl mit bis zu 6 Kohlenstoffatomen bedeutet, das gegebenenfalls durch Hydroxy, Phenyl, Fluor, Chlor, Brom oder Cyano substituiert ist,
- Phenyl bedeutet, welches seinerseits durch Fluor, Chlor, Brom, Amino, Hydroxy, Nitro oder Cyano substituiert sein kann, oder
- eine Gruppe der Formel $-NR^1R^2$ oder $-OR^4$ bedeutet,

worin

$R^1$, $R^2$ und $R^4$ die oben angegebene Bedeutung haben,

n
- eine Zahl 0 oder 2 bedeutet,

$R^6$
- geradkettiges oder verzweigtes Alkyl mit bis zu 8 Kohlenstoffatomen, oder
- Phenyl bedeutet, das durch Fluor, Chlor, Brom, Hydroxy, Cyano, Nitro oder Amino substituiert sein kann, oder
- eine Gruppe der Formel $-NR^1R^2$ bedeutet, wenn n für die Zahl 2 steht,

worin

$R^1$ und $R^2$ die oben angegebene Bedeutung haben,

oder

E
- für eine Gruppe der Formel $-NR^1R^2$ oder $-OR^4$ steht,

worin

$R^1$, $R^2$ und $R^4$ die oben angegebene Bedeutung haben,

Y und Z gleich oder verschieden sind und
- für eine Gruppe der Formel

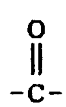

oder

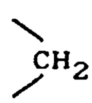

stehen,

oder

Y
- für eine Thiocarbonylgruppe steht,

X
- für eine Gruppe der Formel $-CH_2-CH_2-$ oder $-CH=CH-$steht

und

R - für eine Gruppe der Formel

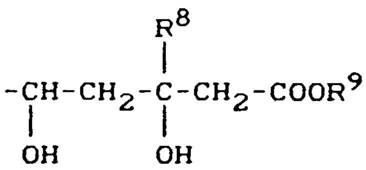

oder

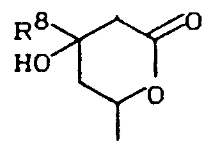

steht,
worin

$R^8$ - Wasserstoff oder geradkettiges oder verzweigtes Alkyl mit bis zu 8 Kohlenstoffatomen bedeutet
und

$R^9$ - Wasserstoff oder geradkettiges oder verzweigtes Alkyl mit bis zu 8 Kohlenstoffatomen oder Benzyl bedeutet, oder
- Phenyl oder ein Kation bedeutet

und deren Salze.

**3.** Substituierte Pyrido(2,3-d)pyrimidine nach den Ansprüchen 1 und 2,
worin

A - für Thienyl, Furyl oder Pyridyl steht, das gegebenenfalls bis zu 2-fach gleich oder verschieden durch Fluor, Chlor, Brom, Hydroxy, Trifluormethyl, Methyl, Ethyl, Methoxy, Ethoxy oder Phenyl, oder durch eine Gruppe der Formel $-NR^1R^2$ substituiert ist,
worin

$R^1$ und $R^2$ gleich oder verschieden sind und
- Wasserstoff, Phenyl, geradkettiges oder verzweigtes Alkyl mit bis zu 4 Kohlenstoffatomen bedeuten,
- für Phenyl steht, das gegebenenfalls bis zu 3-fach gleich oder verschieden substituiert ist durch geradkettiges oder verzweigtes Alkyl oder Alkoxy mit jeweils bis zu 6 Kohlenstoffatomen, welche ihrerseits durch Trifluormethyl, Hydroxy, Methoxy, Ethoxy, Propoxy, Phenyl oder Phenoxy substituiert sein können, oder durch Phenyl, Phenoxy, Fluor oder Chlor substituiert ist,
oder
- für geradkettiges oder verzweigtes Alkyl mit bis zu 4 Kohlenstoffatomen steht,

B - für Cyclopropyl, Cyclobutyl, Cyclopentyl oder Cyclohexyl steht,
- für Methyl, Ethyl, Propyl, Isopropyl, Butyl, tert.Butyl oder Trifluormethyl steht,
- für Phenyl steht, das gegebenenfalls durch Fluor substituiert ist,

D - für Wasserstoff oder
- für Cyclopropyl, Cyclopentyl oder Cyclohexyl steht,
- für Methyl, Ethyl, Propyl, Isopropyl, Butyl oder tert.Butyl steht,

E die oben angegebene Bedeutung von A hat und mit dieser gleich oder verschieden ist, oder
- für Wasserstoff, oder
- für Cyclopropyl, Cyclopentyl oder Cyclohexyl steht,
- für geradkettiges oder verzweigtes Alkyl mit bis zu 8 Kohlenstoffatomen steht, das gegebenenfalls durch Fluor, Chlor, geradkettiges oder verzweigtes Alkenyl mit bis zu 4 Kohlenstoffatomen, Phenyl oder Furyl substituiert ist, oder
- durch eine Gruppe der Formel $-NR^1R^2$ oder $-OR^4$ substituiert ist,
worin

$R^1$ und $R^2$ die oben angegebene Bedeutung haben,

$R^4$ - Wasserstoff oder
- geradkettiges oder verzweigtes Alkyl mit bis zu 6 Kohlenstoffatomen bedeutet, das gegebenenfalls durch Hydroxy oder durch Phenyl substituiert ist,
oder

E - für eine Gruppe der Formel $-NR^1R^2$ steht,
worin

| | |
|---|---|
| $R^1$ und $R^2$ | die oben angegebene Bedeutung haben, |
| Y und Z | gleich oder verschieden sind und |
| | - für eine Gruppe der Formel |

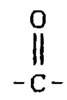 oder 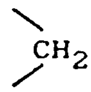

stehen,

oder

| | |
|---|---|
| Y | - für eine Thiocarbonylgruppe steht, |
| X | - für eine Gruppe -CH = CH- steht |
| | und |
| R | - für eine Gruppe der Formel |

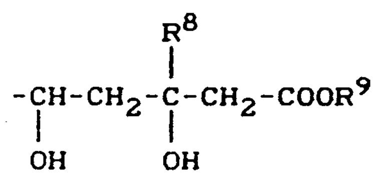 oder 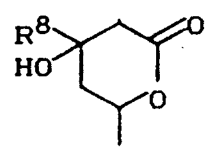

steht,

worin

| | |
|---|---|
| $R^8$ | - Wasserstoff, Methyl, Ethyl, Propyl, Isopropyl, Butyl, Isobutyl oder tert.Butyl bedeutet |
| | und |
| $R^9$ | - Wasserstoff, Methyl, Ethyl, Propyl, Isopropyl, Butyl, Isobutyl, tert.Butyl oder Benzyl bedeutet, oder |
| | - ein Natrium-, Kalium-, Calcium-, Magnesium- oder Ammoniumion bedeutet |

und deren Salze.

**4.** Verfahren zur Herstellung von substituierten Pyrido(2,3-d)pyrimidinen nach Anspruch 1 bis 3 dadurch gekennzeichnet, daß man Ketone der allgemeinen Formel (VIII)

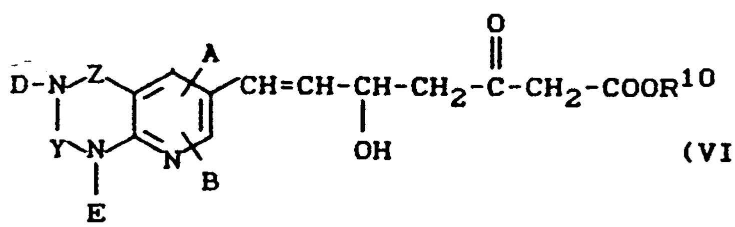

(VIII)

in welcher

A, B, D, E, Y und Z die oben angegebene Bedeutung haben,

und

$R^{10}$ - für Alkyl mit bis zu 6 Kohlenstoffatomen steht,

reduziert,

im Fall der Herstellung der Säuren die Ester verseift,

im Fall der Herstellung der Lactone die Carbonsäuren cyclisiert,

im Fall der Herstellung der Salze entweder die Ester oder die Lactone verseift,

im Fall der Herstellung der Ethylenverbindungen (X = $-CH_2-CH_2-$) die Ethenverbindungen (X =

-CH = CH-) nach üblichen Methoden hydriert, und gegebenenfalls Isomere trennt.

**5.** Substituierte Pyrido(2,3-d)pyrimidine nach Anspruch 1 zur Bekämpfung von Krankheiten.

**6.** Arzneimittel enthaltend mindestens ein substituiertes Pyrido(2,3-d)pyrimidin nach Anspruch 1.

**7.** Verfahren zur Herstellung eines Arzneimittels nach Anspruch 6, dadurch gekennzeichnet, daß man Stoffe nach Anspruch 1 gegebenenfalls mit Hilfe von üblichen Hilfs- und Trägerstoffen in eine geeignete Applikationsform bringt.

**8.** Verwendung der substituierten Pyrido(2,3-d)pyrimidine nach Anspruch 1 zur Herstellung von Arzneimitteln.

**9.** Verwendung der substituierten Pyrido(2,3-d)pyrimdine nach Anspruch 1 zur Herstellung von Arzneimitteln zur Behandlung von Hyperlipoproteinämie, Lipoproteinämie oder Arteriosklerose.

**Patentanspruch für folgenden Vertragsstaat : ES**

**1.** Verfahren zur Herstellung von substituierten Pyrido(2,3-d)pyrimidinen der Formel (I)

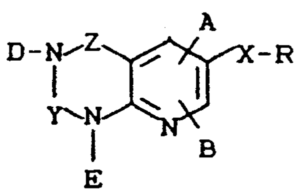

(I)

in welcher

| | |
|---|---|
| A | - für einen 5- bis 7-gliedrigen Heterocyclus steht, der bis zu 4 Heteroatome aus der Reihe Schwefel, Sauerstoff oder Stickstoff enthalten kann und der gegebenenfalls bis zu 3-fach gleich oder verschieden durch Halogen, Hydroxy, Trifluormethyl, geradkettiges oder verzweigtes Alkyl, Alkoxy oder Alkoxycarbonyl mit jeweils bis zu 8 Kohlenstoffatomen, Aryl mit 6 bis 10 Kohlenstoffatomen oder durch eine Gruppe der Formel $-NR^1R^2$ substituiert ist, worin |
| $R^1$ und $R^2$ | gleich oder verschieden sind und<br>- Wasserstoff, Aryl oder Arylsulfonyl mit 6 bis 10 Kohlenstoffatomen, geradkettiges oder verzweigtes Alkyl oder Alkylsulfonyl mit bis zu 8 Kohlenstoffatomen bedeuten, wobei die letztgenannten Reste gegebenenfalls durch Aryl mit 6 bis 10 Kohlenstoffatomen substituiert sind, oder<br>- eine Gruppe der Formel $-COR^3$ bedeutet, worin |
| $R^3$ | - geradkettiges oder verzweigtes Alkyl oder Alkoxy mit bis zu 8 Kohlenstoffatomen oder Phenyl bedeutet,<br>- für Aryl mit 6 bis 10 Kohlenstoffatomen steht, das gegebenenfalls bis zu 5-fach gleich oder verschieden substituiert ist durch geradkettiges oder verzweigtes Alkyl, Alkylthio, Alkylsulfonyl, Alkoxy oder Alkoxycarbonyl mit jeweils bis zu 10 Kohlenstoffatomen, welche ihrerseits durch Trifluormethyl, Hydroxy, Alkoxy mit bis zu 6 Kohlenstoffatomen, Phenyl oder Phenoxy substituiert sein können, oder durch Aryl, Aryloxy, Arylthio oder Arylsulfonyl mit 6 bis 10 Kohlenstoffatomen, oder durch Halogen, Nitro, Cyano, Trifluormethyl, Benzyloxy oder eine Gruppe der Formel $-NR^1R^2$ substituiert ist, worin |
| $R^1$ und $R^2$ | die oben angegebene Bedeutung haben, |
| B | - für Cycloalkyl mit 3 bis 8 Kohlenstoffatomen steht, |

- für Trifluormethyl oder geradkettiges oder verzweigtes Alkyl mit bis zu 12 Kohlenstoffatomen steht, das gegebenenfalls durch Halogen, Hydroxy, Cyano, Azido, Trifluormethyl, Alkylthio, Alkylsulfonyl oder Alkoxy mit jeweils bis zu 8 Kohlenstoffatomen oder durch Aryl, Aryloxy oder Arylthio mit 6 bis 10 Kohlenstoffatomen substituiert ist, wobei die Arylreste gegebenenfalls bis zu 3-fach gleich oder verschieden durch Halogen, Cyano, Trifluormethyl, Trifluormethoxy, geradkettiges oder verzweigtes Alkyl, Alkoxy, Alkylthio oder Alkylsulfonyl mit jeweils bis zu 8 Kohlenstoffatomen substituiert sein können, oder durch eine Gruppe der Formel $-NR^1R^2$ oder $-COR^3$ substituiert ist,

worin

$R^1$, $R^2$ und $R^3$ die oben angegebene Bedeutung haben,

- für Aryl mit 6 bis 10 Kohlenstoffatomen steht, das gegebenenfalls durch Halogen, Cyano, Nitro, Trifluormethyl, geradkettiges oder verzweigtes Alkyl, Alkoxy oder Alkoxycarbonyl mit jeweils bis zu 8 Kohlenstoffatomen oder Amino substituiert ist,

D - für Wasserstoff, Hydroxy oder
- Cycloalkyl mit 3 bis 8 Kohlenstoffatomen steht,
- für geradkettiges oder verzweigtes Alkyl mit jeweils bis zu 12 Kohlenstoffatomen steht, das gegebenenfalls durch Halogen, Hydroxy oder Alkoxy mit bis zu 8 Kohlenstoffatomen substituiert ist,

E - die oben angegebene Bedeutung von A hat und mit dieser gleich oder verschieden ist, oder
- für Wasserstoff oder
- Cycloalkyl mit 3 bis 8 Kohlenstoffatomen steht,
- für geradkettiges oder verzweigtes Alkyl mit bis 12 Kohlenstoffatomen steht, das gegebenenfalls durch Halogen, geradkettiges oder verzweigtes Alkenyl mit bis zu 8 Kohlenstoffatomen, Aryl mit 6 bis 10 Kohlenstoffatomen, durch einen 5- bis 7-gliedrigen Heterocyclus mit bis zu 4 Heteroatomen aus der Reihe Stickstoff, Sauerstoff oder Schwefel oder durch eine Gruppe der Formel $-NR^1R^2$, $-OR^4$, $-COR^5$ oder $S(O)_n-R^6$ substituiert ist,

worin

$R^1$ und $R^2$ die oben angegebene Bedeutung haben,

$R^4$ - Wasserstoff oder
- geradkettiges oder verzweigtes Alkyl mit bis zu 10 Kohlenstoffatomen bedeutet, das gegebenenfalls durch Hydroxy, Trialkylsilyl mit bis zu 10 Kohlenstoffatomen im Alkylteil, Halogen oder Aryl mit 6 bis 10 Kohlenstoffatomen substituiert ist,
- Trialkylsilyl mit bis zu 10 Kohlenstoffatomen oder Cycloalkyl mit 3 bis 8 Kohlenstoffatomen oder Aryl mit 6 bis 10 Kohlenstoffatomen bedeutet, welches seinerseits durch Halogen, Cyano, Nitro oder Amine substituiert sein kann, oder
- eine Gruppe der Formel $-COR^7$ bedeutet,

worin

$R^7$ - geradkettiges oder verzweigtes Alkyl mit bis zu 8 Kohlenstoffatomen, Aryl mit 6 bis 10 Kohlenstoffatomen oder die Gruppe $-NR^1R^2$ bedeutet,

worin

$R^1$ und $R^2$ die oben angegebene Bedeutung haben,

$R^5$ - Wasserstoff oder geradkettiges oder verzweigtes Alkyl mit bis zu 8 Kohlenstoffatomen bedeutet, das gegebenenfalls durch Hydroxy, Phenyl, Halogen oder Cyano substituiert ist,
- Aryl mit 6 bis 10 Kohlenstoffatomen bedeutet, welches durch Halogen, Amine, Hydroxy, Nitro oder Cyano substituiert sein kann, oder
- eine Gruppe der Formel $-NR^1R^2$ oder $-OR^4$ bedeutet,

worin

$R^1$, $R^2$ und $R^4$ die oben angegebene Bedeutung haben,

n - eine Zahl 0 oder 2 bedeutet,

$R^6$ - geradkettiges oder verzweigtes Alkyl mit bis zu 10 Kohlenstoffatomen bedeutet, das durch Halogen, Hydroxy, Phenyl oder eine Gruppe der Formel $-NR^1R^2$ substituiert sein kann,

worin

$R^1$ und $R^2$ die oben angegebene Bedeutung haben,

- Aryl mit 6 bis 10 Kohlenstoffatomen bedeutet, das durch Halogen, Hydroxy, Cyano, Nitro oder Amine substituiert sein kann, oder
- eine Gruppe der Formel $-NR^1R^2$ bedeutet, wenn n für die Zahl 2 steht, worin

$R^1$ und $R^2$ die oben angegebene Bedeutung haben,

oder

E - für eine Gruppe der Formel $-NR^1R^2$ oder $-OR^4$ steht, worin

$R^1$, $R^2$ und $R^4$ die oben angegebene Bedeutung haben,

Y und Z gleich oder verschieden sind und - für eine Gruppe der Formel

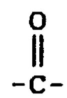

oder

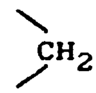

stehen,

oder

Y - für eine Thiocarbonylgruppe steht,

X - für eine Gruppe der Formel $-CH_2-CH_2-$ oder $-CH=CH-$steht,

und

R - für eine Gruppe der Formel

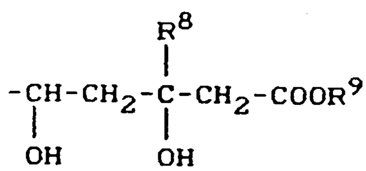

oder

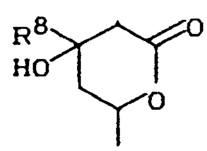

steht,

worin

$R^8$ - Wasserstoff oder geradkettiges oder verzweigtes Alkyl mit bis zu 10 Kohlenstoffatomen bedeutet

und

$R^9$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,

und deren Salze,

dadurch gekennzeichnet, daß man

Ketone der allgemeinen Formel (VIII)

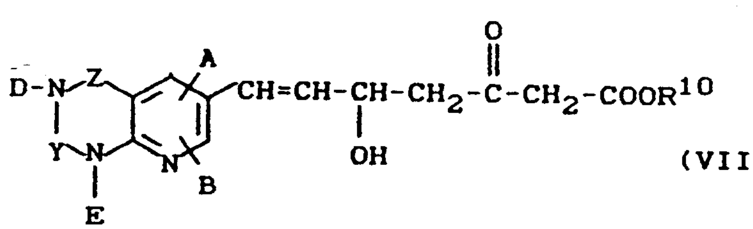

(VIII)

in welcher

A, B, D, E, Y und Z die oben angegebene Bedeutung haben,

und

R[10] - für Alkyl mit bis zu 6 Kohlenstoffatomen steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X = $-CH=CH-$) nach üblichen Methoden hydriert,
und gegebenenfalls Isomere trennt.

## Claims

**Claims for the following Contracting States : AT, BE, CH, DE, FR, GB, GR, IT, LI, LU, NL, SE**

**1.** Substituted pyrido(2,3-d)pyrimidines of the general formula (I)

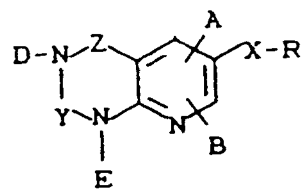

(I)

in which

A - represents a 5- to 7-membered heterocycle which may contain up to 4 heteroatoms from the series comprising sulphur, oxygen or nitrogen and which is optionally monosubstituted to trisubstituted by identical or different substituents from the series comprising halogen, hydroxyl, trifluoromethyl, straight-chain or branched alkyl, alkoxy or alkoxycarbonyl each having up to 8 carbon atoms, aryl having 6 to 10 carbon atoms or by a group of the formula $-NR^1R^2$,
in which

R' and $R^2$ are identical or different and
- denote hydrogen, aryl or arylsulphonyl having 6 to 10 carbon atoms or straight-chain or branched alkyl or alkylsulphonyl having up to 8 carbon atoms, where the last-mentioned radicals are optionally substituted by aryl having 6 to 10 carbon atoms, or
- denote a group of the formula $-COR^3$,
in which

$R^3$ - denotes straight-chain or branched alkyl or alkoxy having up to 8 carbon atoms or phenyl,
- represents aryl having 6 to 10 carbon atoms, which is optionally monosubstituted to pentasubstituted by identical or different substituents from the series comprising straight-chain or branched alkyl, alkylthio, alkylsulphonyl, alkoxy or alkoxycarbonyl each having up to 10 carbon atoms, which may in turn be substituted by trifluoromethyl, hydroxyl, alkoxy having up to 6 carbon atoms, phenyl or phenoxy,
or is substituted by aryl, aryloxy, arylthio or arylsulphonyl having 6 to 10 carbon atoms, or by halogen, nitro, cyano, trifluoromethyl, benzyloxy or a group of the formula $-NR^1R^2$,
or
- represents straight-chain or branched alkyl having up to 8 carbon atoms in each case,

B - represents cycloalkyl having 3 to 8 carbon atoms,
- represents trifluoromethyl or straight-chain or branched alkyl having up to 12 carbon atoms, which is optionally substituted by halogen, hydroxyl cyano, azido, trifluoromethyl, alkylthio, alkylsulphonyl or alkoxy each having up to 8 carbon atoms or by aryl, aryloxy or arylthio having 6 to 10 carbon atoms, where the aryl radicals may optionally be monosubstituted to trisubstituted by identical or different substituents from the series comprising halogen, cyano, trifluoromethyl,

trifluoromethoxy, straight-chain or branched alkyl, alkoxy, alkylthio or alkylsulphonyl each having up to 8 carbon atoms, or is substituted by a group of the formula $-NR^1R^2$ or $-COR^3$,
in which

$R^1$, $R^2$ and $R^3$ have the abovementioned meaning,
- represents aryl having 6 to 10 carbon atoms, which is optionally substituted by halogen, cyano, nitro, trifluoromethyl, straight-chain or branched alkyl, alkoxy or alkoxycarbonyl each having up to 8 carbon atoms or amino,

D - represents hydrogen, hydroxyl or
- cycloalkyl having 3 to 8 carbon atoms
- represents straight-chain or branched alkyl each having up to 12 carbon atoms, which is optionally substituted by halogen, hydroxyl or alkoxy having up to 8 carbon atoms,

E - has the meaning mentioned above for A and is identical or different to this, or
- represents hydrogen or
- represents cycloalkyl having 3 to 8 carbon atoms,
- represents straight-chain or branched alkyl having up to 12 carbon atoms, which is optionally substituted by halogen, straight-chain or branched alkenyl having up to 8 carbon atoms, aryl having 6 to 10 carbon atoms, by a 5- to 7-membered heterocycle having up to 4 heteroatoms from the series comprising nitrogen, oxygen or sulphur or by a group of the formula $-NR^1R^2$, $-OR^4$, $-COR^5$ or $-S(O)_n-R^6$,
in which

$R^1$ and $R^2$ have the abovementioned meaning,

$R^4$ - denotes hydrogen or
- straight-chain or branched alkyl having up to 10 carbon atoms, which is optionally substituted by hydroxyl, trialkylsilyl having up to 10 carbon atoms in the alkyl moiety, or halogen or aryl having 6 to 10 carbon atoms,
- denotes trialkylsilyl having up to 10 carbon atoms or cycloalkyl having 3 to 8 carbon atoms or aryl having 6 to 10 carbon atoms, which may in turn be substituted by halogen, cyano, nitro or amino, or
- denotes a group of the formula $-COR^7$,
in which

$R^7$ - denotes straight-chain or branched alkyl having up to 8 carbon atoms, aryl having 6 to 10 carbon atoms or the group $-NR^1R^2$,
in which

$R^1$ and $R^2$ have the abovementioned meaning,

$R^5$ - denotes hydrogen or straight-chain or branched alkyl having up to 8 carbon atoms, which is optionally substituted by hydroxyl, phenyl, halogen or cyano,
- denotes aryl having 6 to 10 carbon atoms, which may be substituted by halogen, amino, hydroxyl, nitro or cyano, or
- denotes a group of the formula $-NR^1R^2$ or $-OR^4$,
in which

$R^1$, $R^2$ and $R^4$ have the abovementioned meaning,

n - denotes a number 0 or 2,

$R^6$ - denotes straight-chain or branched alkyl having up to 10 carbon atoms, which may be substituted by halogen, hydroxyl, phenyl or a group of the formula $-NR^1R^2$,
in which

$R^1$ and $R^2$ have the abovementioned meaning,
- denotes aryl having 6 to 10 carbon atoms, which may be substituted by halogen, hydroxyl, cyano, nitro or amino, or
- denotes a group of the formula $-NR^1R^2$, if n represents the number 2,
in which

$R^1$ and $R^2$ have the abovementioned meaning,
or

E - represents a group of the formula $-NR^1R^2$ or $-OR^4$,
in which

$R^1$, $R^2$ and $R^4$ have the abovementioned meaning,

Y and Z are identical or different and
- represent a group of the formula

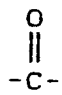 or 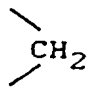 ,

or
Y - represents a thiocarbonyl group,
X - represents a group of the formula $-CH_2-CH_2-$ or $-CH=CH-$,
and
R - represents a group of the formula

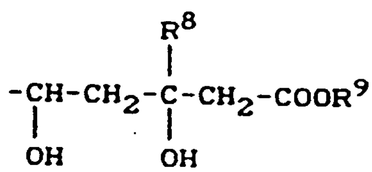 or 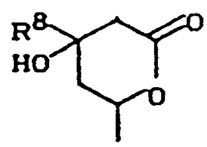 ,

in which
$R^8$ - denotes hydrogen or straight-chain or branched alkyl having up to 10 carbon atoms,
and
$R^9$ - denotes hydrogen or straight-chain or branched alkyl having up to 10 carbon atoms, which may be substituted by phenyl, or
- denotes aryl having 6 to 10 carbon atoms or a cation,

and their salts.

**2.** Substituted pyrido(2,3-d)pyrimidines according to claim 1 of the formulae (1a) and (Ib)

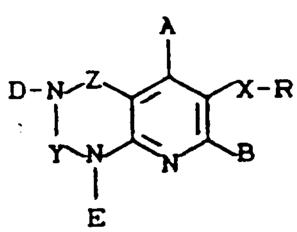

(Ia)

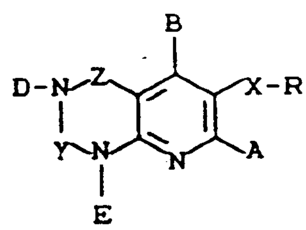

(Ib)

in which
A - represents thienyl, furyl, pyridyl or pyrimidyl, each of which is optionally monosubstituted to trisubstituted by identical or different substituents from the series comprising fluorine, chlorine, bromine, hydroxyl, trifluoromethyl, straight-chain or branched alkyl, alkoxy or alkoxycarbonyl each having up to 6 carbon atoms, phenyl, or by a group of the formula $-NR^1R^2$,
in which
$R^1$ and $R^2$ are identical or different and
- denote hydrogen, phenyl, phenylsulphonyl, straight-chain or branched alkyl or alkylsulphonyl having up to 6 carbon atoms, or benzyl or benzylsulphonyl,

- denote a group of the formula $-COR^3$,
in which

$R^3$ - denotes straight-chain or branched alkyl or alkoxy having up to 6 carbon atoms or phenyl,

or

- represents phenyl or naphthyl, each of which is optionally monosubstituted to tetrasubstituted by identical or different straight-chain or branched substituents from the series comprising alkyl, alkoxy or alkoxycarbonyl each having up to 8 carbon atoms, which may in turn be substituted by trifluoromethyl, hydroxyl, alkoxy having up to 4 carbon atoms, phenyl or phenoxy, or is substituted by phenyl, phenoxy, phenylthio, phenylsulphonyl, fluorine, chlorine, bromine, nitro, cyano, trifluoromethyl, benzyloxy or by a group of the formula $-NR^1R^2$,

or

- represents straight-chain or branched alkyl having up to 6 carbon atoms,

B - represents cyclopropyl, cyclobutyl, cyclopentyl or cyclohexyl,
- represents trifluoromethyl or straight-chain or branched alkyl having up to 10 carbon atoms, which is optionally substituted by fluorine, chlorine, bromine, hydroxyl, cyano, azido, trifluoromethyl, methylthio, methylsulphonyl, alkoxy having up to 6 carbon atoms or by phenyl, phenyloxy or phenylthio, where the phenyl radicals may be monosubstituted or disubstituted by identical or different substituents from the series comprising fluorine, chlorine, bromine, cyano, trifluoromethyl, trifluoromethoxy, straight-chain or branched alkyl, alkoxy, alkylthio or alkylsulphonyl each having up to 6 carbon atoms, or
is substituted by a group of the formula $-NR^1R^2$ or $-COR^3$,

or

- represents phenyl which is optionally substituted by fluorine or chlorine,

D - represents hydrogen, hydroxyl or
- represents cyclopropyl, cyclobutyl, cyclopentyl or cyclohexyl,
- represents straight-chain or branched alkyl having up to 10 carbon atoms, which is optionally substituted by fluorine, chlorine, bromine, hydroxyl or alkoxy having up to 6 carbon atoms,

E - has the meaning mentioned above for A and is identical or different to this, or
- represents hydrogen or
- represents cyclopropyl, cyclobutyl, cyclopentyl or cyclohexyl,
- represents straight-chain or branched alkyl having up to 10 carbon atoms, which is optionally substituted by fluorine, chlorine, bromine, straight-chain or branched alkenyl having up to 6 carbon atoms, phenyl, pyrimidyl, pyrrolyl, pyrrolidinyl, furyl or thiazolyl, or by a group of the formula $-NR^1R^2$, $-OR^4$, $-COR^5$ or $-S(O)_n-R^6$,
in which

$R^1$ and $R^2$ have the abovementioned meaning,

$R^4$ - denotes hydrogen or
- straight-chain or branched alkyl having up to 8 carbon atoms, which is optionally substituted by hydroxyl, fluorine, chorine, bromine or by phenyl, or
- denotes a group of the formula $-COR^7$,
in which

$R^7$ - denotes straight-chain or branched alkyl having up to 6 carbon atoms, phenyl or a group of the formula $-NR^1R^2$,
in which

$R^1$ and $R^2$ have the abovementioned meaning,

$R^5$ - denotes hydrogen, or straight-chain or branched alkyl having up to 6 carbon atoms, which is optionally substituted by hydroxyl, phenyl, fluorine, chlorine, bromine or cyano,
- denotes phenyl which may in turn be substituted by fluorine, chlorine, bromine, amino, hydroxyl, nitro or cyano, or
- denotes a group of the formula $-NR^1R^2$ or $-OR^4$,
in which

$R^1$, $R^2$ and $R^4$ have the abovementioned meaning,

n - denotes a number 0 or 2,

| | |
|---|---|
| $R^6$ | - denotes straight-chain or branched alkyl having up to 8 carbon atoms or,<br>- phenyl which may be substituted by fluorine, chlorine, bromine, hydroxyl, cyano, nitro or amino, or<br>- denotes a group of the formula $-NR^1R^2$, if n represents the number 2,<br>in which |
| $R^1$ and $R^2$ | have the abovementioned meaning,<br>or |
| E | - represents a group of the formula $-NR^1R^2$ or $-OR^4$,<br>in which |
| $R^1$, $R^2$ and $R^4$ | have the abovementioned meaning, |
| Y and Z | are identical or different and<br>- represent a group of the formula |

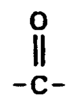 or 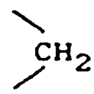 ,

or

| | |
|---|---|
| Y | - represents a thiocarbonyl group, |
| X | - represents a group of the formula $-CH_2-CH_2-$ or $-CH=CH-$<br>and |
| R | - represents a group of the formula |

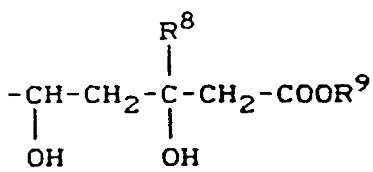 or 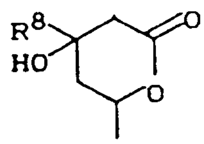

,

in which

| | |
|---|---|
| $R^8$ | - denotes hydrogen or straight-chain or branched alkyl having up to 8 carbon atoms,<br>and |
| $R^9$ | - denotes hydrogen or straight-chain or branched alkyl having up to 8 carbon atoms or benzyl, or<br>- denotes phenyl or a cation |

and their salts.

**3.** Substituted pyrido(2,3-d)pyrimidines according to claims 1 and 2,
in which

| | |
|---|---|
| A | - represents thienyl, furyl or pyridyl, which is optionally monosubstituted or disubstituted by identical or different substituents from the series comprising fluorine, chlorine, bromine, hydroxyl, trifluoromethyl, methyl, ethyl, methoxy, ethoxy or phenyl, or by a group of the formula $-NR^1R^2$,<br>in which |
| $R^1$ and $R^2$ | are identical or different and<br>- denote hydrogen, phenyl, or straight-chain or branched alkyl having up to 4 carbon atoms,<br>- represents phenyl which is optionally monosubstituted to trisubstituted by identical or different substituents from the series comprising straight-chain or branched alkyl or alkoxy each having up to 6 carbon atoms, which may in turn be substituted by trifluoromethyl, hydroxyl, methoxy, ethoxy, propoxy, phenyl or phenoxy, |

or is substituted by phenyl, phenoxy, fluorine or chlorine,
or
- represents straight-chain or branched alkyl having up to 4 carbon atoms,

B - represents cyclopropyl, cyclobutyl, cyclopentyl or cyclohexyl,
- represents methyl, ethyl, propyl, isopropyl, butyl, tert. butyl or trifluoromethyl,
- represents phenyl which is optionally substituted by fluorine,

D - represents hydrogen or
- represents cyclopropyl, cyclopentyl or cyclohexyl,
- represents methyl, ethyl, propyl, isopropyl, butyl or tert. butyl,

E - has the meaning mentioned above for A and is identical or different to this, or
- represents hydrogen, or
- represents cyclopropyl, cyclopentyl or cyclohexyl,
- represents straight-chain or branched alkyl having up to 8 carbon atoms, which is optionally substituted by fluorine, chlorine, straight-chain or branched alkenyl having up to 4 carbon atoms, phenyl or furyl, or
- is substituted by a group of the formula $-NR^1R^2$ or $-OR^4$,
in which

$R^1$ and $R^2$ have the abovementioned meaning,

$R^4$ - denotes hydrogen or
- straight-chain or branched alkyl having up to 6 carbon atoms, which is optionally substituted by hydroxyl or by phenyl,
or

E - represents a group of the formula $-NR^1R^2$, in which

$R^1$ and $R^2$ have the abovementioned meaning,

Y and Z are identical or different and
- represent a group of the formula

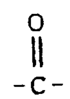 or 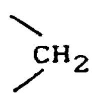 ,

or

Y - represents a thiocarbonyl group,

X - represents a group -CH=CH-
and

R - represents a group of the formula

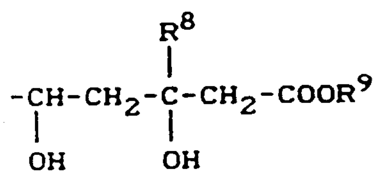 or 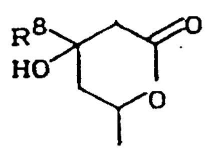 ,

in which

$R^8$ - denotes hydrogen, methyl, ethyl, propyl, isopropyl, butyl, isobutyl or tert. butyl
and

$R^9$ - denotes hydrogen, methyl, ethyl, propyl, isopropyl, butyl, isobutyl, tert. butyl or benzyl, or
- denotes a sodium, potassium, calcium, magnesium or ammonium ion

and their salts.

**4.** Process for the preparation of substituted pyrido(2,3-d)pyrimidines according to claims 1 to 3 characterized in that

ketones of the general formula (VIII)

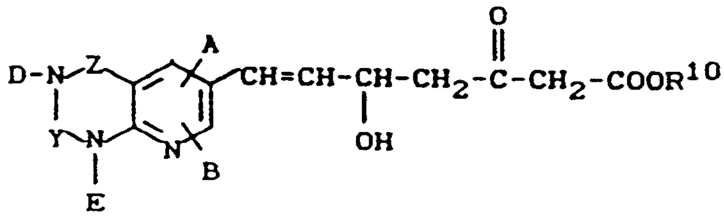

(VIII)

in which
A, B, D, E, Y and Z have the abovementioned meaning,
and

$R^{10}$ - represents alkyl having up to 6 carbon atoms,

are reduced,
in the case of the preparation of the acids the esters are hydrolyzed,
in the case of the preparation of the lactones the carboxylic acids are cyclized,
in the case of the preparation of the salts either the esters or the lactones are hydrolyzed,
in the case of the preparation of the ethylene compounds (X = $-CH_2-CH_2-$) the ethene compounds (X = -CH = CH-) are hydrogenated according to customary methods,
and, if appropriate, isomers are separated.

**5.** Substituted pyrido(2,3-d)pyrimidines according to Claim 1 for combating diseases.

**6.** Medicaments containing at least one substituted pyrido(2,3-d)pyrimidine according to Claim 1.

**7.** Process for the production of a medicament according to Claim 6, characterized in that substances according to Claim 1 are brought into a suitable form for administration, if appropriate with the aid of customary auxiliaries and excipients.

**8.** Use of the substituted pyrido(2,3-d)pyrimidines according to Claim 1 for the preparation of medicaments.

**9.** Use of the substituted pyrido(2,3-d)pyrimidines according to Claim 1 for the production of medicaments for the treatment of hyperlipoproteinaemia, lipoproteinaemia or arteriosclerosis.

**Claim for the following Contracting State : ES**

**1.** Process for the preparation of substituted pyrido(2,3-d)pyrimidines of the formula (I)

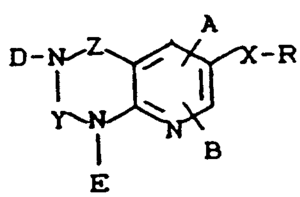

(I)

in which

A - represents a 5- to 7-membered heterocycle which may contain up to 4 heteroatoms from the series comprising sulphur, oxygen or nitrogen and which is optionally monosubstituted to trisubstituted by identical or different substituents from the series comprising halogen, hydroxyl, trifluoromethyl, straight-chain or branched alkyl, alkoxy or alkoxycarbonyl each having up to 8 carbon atoms, aryl having 6 to 10 carbon atoms or by a group of the formula $-NR^1R^2$,

in which

$R^1$ and $R^2$ are identical or different and
- denote hydrogen, aryl or arylsulphonyl having 6 to 10 carbon atoms or straight-chain or branched alkyl or alkylsulphonyl having up to 8 carbon atoms, where the last-mentioned radicals are optionally substituted by aryl having 6 to 10 carbon atoms, or
- denote a group of the formula $-COR^3$,

in which

$R^3$ - denotes straight-chain or branched alkyl or alkoxy having up to 8 carbon atoms or phenyl,
- represents aryl having 6 to 10 carbon atoms, which is optionally monosubstituted to pentasubstituted by identical or different substituents from the series comprising straight-chain or branched alkyl, alkylthio, alkylsulphonyl, alkoxy or alkoxycarbonyl each having up to 10 carbon atoms, which may in turn be substituted by trifluoromethyl, hydroxyl, alkoxy having up to 6 carbon atoms, phenyl or phenoxy, or is substituted by aryl, aryloxy, arylthio or arylsulphonyl having 6 to 10 carbon atoms, or by halogen, nitro, cyano, trifluoromethyl, benzyloxy or a group of the formula $-NR^1R^2$,

in which

$R^1$ and $R^2$ have the abovementioned meaning,

B - represents cycloalkyl having 3 to 8 carbon atoms,
- represents trifluoromethyl or straight-chain or branched alkyl having up to 12 carbon atoms, which is optionally substituted by halogen, hydroxyl, cyano, azido, trifluoromethyl, alkylthio, alkylsulphonyl or alkoxy each having up to 8 carbon atoms or by aryl, aryloxy or arylthio having 6 to 10 carbon atoms, where the aryl radicals may optionally be monosubstituted to trisubstituted by identical or different substituents from the series comprising halogen, cyano, trifluoromethyl, trifluoromethoxy, straight-chain or branched alkyl, alkoxy, alkylthio or alkylsulphonyl each having up to 8 carbon atoms, or is substituted by a group of the formula $-NR^1R^2$ or $-COR^3$,

in which

$R^1$, $R^2$ and $R^3$ have the abovementioned meaning,
- represents aryl having 6 to 10 carbon atoms, which is optionally substituted by halogen, cyano, nitro, trifluoromethyl, straight-chain or branched alkyl, alkoxy or alkoxycarbonyl each having up to 8 carbon atoms or amino,

D - represents hydrogen, hydroxyl or
- cycloalkyl having 3 to 8 carbon atoms
- represents straight-chain or branched alkyl each having up to 12 carbon atoms, which is optionally substituted by halogen, hydroxyl or alkoxy having up to 8 carbon atoms,

E - has the meaning mentioned above for A and is identical or different to this, or
- represents hydrogen or
- represents cycloalkyl having 3 to 8 carbon atoms,
- represents straight-chain or branched alkyl having up to 12 carbon atoms, which is optionally substituted by halogen, straight-chain or branched alkenyl having up to 8 carbon atoms, aryl having 6 to 10 carbon atoms, by a 5- to 7-membered heterocycle having up to 4 heteroatoms from the series comprising nitrogen, oxygen or sulphur or by a group of the formula $-NR^1R^2$, $-OR^4$, $-COR^5$ or $-S(O)_n-R^6$,

in which

$R^1$ and $R^2$ have the abovementioned meaning,

$R^4$ - denotes hydrogen or
- straight-chain or branched alkyl having up to 10 carbon atoms, which is optionally substituted by hydroxyl, trialkylsilyl having up to 10 carbon atoms in the alkyl moiety, or halogen or aryl having 6 to 10 carbon atoms,
- denotes trialkylsilyl having up to 10 carbon atoms or cycloalkyl having 3 to 8 carbon atoms or aryl having 6 to 10 carbon atoms, which may in turn be substituted by halogen, cyano, nitro or amino, or
- denotes a group of the formula $-COR^7$,

in which

$R^7$ - denotes straight-chain or branched alkyl having up to 8 carbon atoms, aryl having 6 to 10 carbon atoms or the group $-NR^1R^2$,

in which

$R^1$ and $R^2$ have the abovementioned meaning,

$R^5$ - denotes hydrogen or straight-chain or branched alkyl having up to 8 carbon atoms, which is optionally substituted by hydroxyl, phenyl, halogen or cyano,

- denotes aryl having 6 to 10 carbon atoms, which may be substituted by halogen, amino, hydroxyl, nitro or cyano, or

- denotes a group of the formula $-NR^1R^2$ or $-OR^4$,

in which

$R^1$, $R^2$ and $R^4$ have the abovementioned meaning,

n - denotes a number 0 or 2,

$R^6$ - denotes straight-chain or branched alkyl having up to 10 carbon atoms, which may be substituted by halogen, hydroxyl, phenyl or a group of the formula $-NR^1R^2$,

in which

$R^1$ and $R^2$ have the abovementioned meaning,

- denotes aryl having 6 to 10 carbon atoms, which may be substituted by halogen, hydroxyl, cyano, nitro or amino, or

- denotes a group of the formula $-NR^1R^2$, if n represents the number 2,

in which

$R^1$ and $R^2$ have the abovementioned meaning,

or

E - represents a group of the formula $-NR^1R^2$ or $-OR^4$,

in which

$R^1$, $R^2$ and $R^4$ have the abovementioned meaning,

Y and Z are identical or different and

- represent a group of the formula

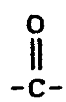 or 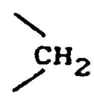 ,

or

Y - represents a thiocarbonyl group,

X - represents a group of the formula $-CH_2-CH_2-$ or $-CH=CH-$,

and

R - represents a group of the formula

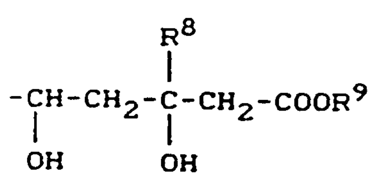 or 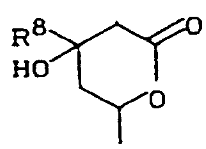 ,

in which

$R^8$ - denotes hydrogen or straight-chain or branched alkyl having up to 10 carbon atoms,

and

$R^9$ - denotes hydrogen or straight-chain or branched alkyl having up to 10 carbon atoms, which may be substituted by phenyl, or

- denotes aryl having 6 to 10 carbon atoms or a cation,

and their salts,

characterized in that

ketones of the general formula (VIII)

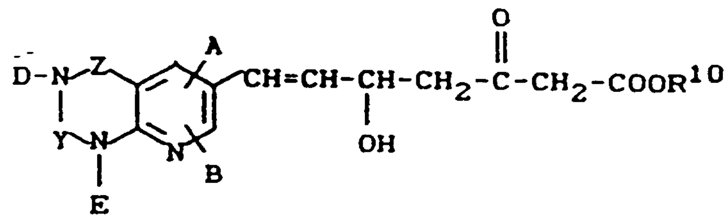

(VIII)

in which
A, B, D, E, Y and Z have the abovementioned meaning,
and
$R^{10}$ - represents alkyl having up to 6 carbon atoms,
are reduced,
in the case of the preparation of the acids the esters are hydrolyzed,
in the case of the preparation of the lactones the carboxylic acids are cyclized,
in the case of the preparation of the salts either the esters or the lactones are hydrolyzed,
in the case of the preparation of the ethylene compounds (X = $-CH_2-CH_2-$) the ethene compounds (X = -CH = CH-) are hydrogenated according to customary methods,
and, if appropriate, isomers are separated.

## Revendications

**Revendications pour les Etats contractants suivants : AT, BE, CH, DE, FR, GB, GR, IT, LI, LU, NL, SE**

**1.** Pyrido(2,3-d)pyrimidines substituées de formule générale (I)

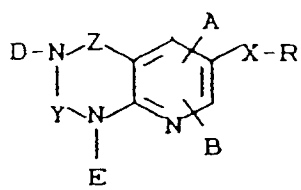

(I)

dans laquelle

A - représente un hétérocycle pentagonal à heptagonal qui peut contenir jusqu'à 4 hétéro-atomes de la série soufre, oxygène et azote et qui porte le cas échéant jusqu'à 3 substituants, identiques ou différents, halogéno, hydroxy, trifluorométhyle, alkyle, alkoxy ou alkoxycarbonyle à chaîne droite ou ramifiée ayant chacun jusqu'à 8 atomes de carbone, aryle de 6 à 10 atomes de carbone ou groupe de formule $-NR^1R^2$ dans laquelle

$R^1$ et $R^2$ sont identiques ou différents et représentent
- l'hydrogène, un reste aryle ou arylsulfonyle ayant 6 à 10 atomes de carbone, un reste alkyle ou alkylsulfonyle à chaîne droite ou ramifiée ayant jusqu'à 8 atomes de carbone, les restes mentionnés en dernier lieu étant substitués le cas échéant par un radical aryle ayant 6 à 10 atomes de carbone, ou bien
- un groupe de formule $-COR^3$,
dans laquelle

$R^3$ - est un reste alkyle ou alkoxy à chaîne droite ou ramifiée ayant jusqu'à 8 atomes de carbone ou un reste phényle,
- un groupe aryle de 6 à 10 atomes de carbone, qui est substitué, le cas échéant, jusqu'à 5 fois identiques ou différentes par un reste alkyle, alkylthio, alkylsulfonyle, alkoxy ou alkoxycarbonyle à chaîne droite ou ramifiée ayant chacun jusqu'à 10 atomes de carbone, ces restes pouvant être substitués quant à eux par un radical

trifluorométhyle, hydroxy, alkoxy ayant jusqu'à 6 atomes de carbone, phényle ou phénoxy, ou par un reste aryle, aryloxy, arylthio ou arylsulfonyle ayant 6 à 10 atomes de carbone, ou par un reste halogéno, nitro, cyano, trifluorométhyle, benzyloxy ou un groupe de formule $-NR^1R^2$,
ou bien
- un groupe alkyle à chaîne droite ou ramifiée, chacun ayant jusqu'à 8 atomes de carbone,

B - représente un groupe cycloalkyle de 3 à 8 atomes de carbone
- un groupe trifluorométhyle ou un groupe alkyle à chaîne droite ou ramifiée ayant jusqu'à 12 atomes de carbone, qui est substitué le cas échéant par un halogène, un radical hydroxy, cyano, azido, trifluorométhyle, alkylthio, alkylsulfonyle ou alkoxy ayant chacun jusqu'à 8 atomes de carbone ou par un radical aryle, aryloxy ou arylthio de 6 à 10 atomes de carbone, les restes aryle pouvant être substitués le cas échéant jusqu'à 3 fois identiques ou différentes par un radical halogéno, cyano, trifluorométhyle, trifluorométhoxy, alkyle, alkoxy, alkylthio ou alkylsulfonyle à chaîne droite ou ramifiée ayant chacun jusqu'à 8 atomes de carbone, ou par un groupe de formule $-NR^1R^2$ ou $-COR^3$,
dans lequel

$R^1$, $R^2$ et $R^3$ ont la définition indiquée ci-dessus,
- un groupe aryle ayant 6 à 10 atomes de carbone, qui est substitué le cas échéant par un radical halogéno, cyano, nitro, trifluorométhyle, un radical alkyle, alkoxy ou alkoxycarbonyle à chaîne droite ou ramifiée ayant chacun jusqu'à 8 atomes de carbone ou un radical amino,

D - représente l'hydrogène, un groupe hydroxy ou
- un groupe cycloalkyle ayant 3 à 8 atomes de carbone,
- un groupe alkyle linéaire ou ramifié ayant dans chaque cas jusqu'à 12 atomes de carbone, qui est substitué le cas échéant par un halogène, un radical hydroxy ou alkoxy ayant jusqu'à 8 atomes de carbone,

E - a la définition indiquée ci-dessus pour A et est identique à ce dernier ou en est différent, ou bien
- de l'hydrogène, ou bien
- un groupe cycloalkyle de 3 à 8 atomes de carbone,
- un groupe alkyle linéaire ou ramifié ayant jusqu'à 12 atomes de carbone, qui est substitué le cas échéant par un halogène, un radical alcényle à chaîne droite ou ramifiée ayant jusqu'à 8 atomes de carbone, aryle de 6 à 10 atomes de carbone, par un hétérocycle pentagonal à heptagonal ayant jusqu'à 4 hétéro-atomes de la série azote, oxygène ou soufre ou par un groupe de formule $-NR^1R^2$, $-OR^4$, $-COR^5$ ou $S(O)_n-R^6$,
où

$R^1$ et $R^2$ ont la définition indiquée ci-dessus

$R^4$ - est de l'hydrogène ou
- un groupe alkyle à chaîne droite ou ramifiée ayant jusqu'à 10 atomes de carbone, qui est substitué le cas échéant par un radical hydroxy, trialkylsilyle ayant jusqu'à 10 atomes de carbone dans la partie alkyle, halogéno ou aryle ayant 6 à 10 atomes de carbone,
- un groupe trialkylsilyle ayant jusqu'à 10 atomes de carbone ou cycloalkyle ayant 3 à 8 atomes de carbone ou aryle ayant 6 à 10 atomes de carbone, qui peut être substitué quant à lui par un halogène, un radical cyano, nitro ou amino, ou bien
- un groupe de formule $-COR^7$,
dans laquelle

$R^7$ - est un radical alkyle à chaîne droite ou ramifiée ayant jusqu'à 8 atomes de carbone, aryle de 6 à 10 atomes de carbone ou le groupe $-NR^1R^2$,
où

$R^1$ et $R^2$ ont la définition indiquée ci-dessus,

$R^5$ - représente de l'hydrogène ou un groupe alkyle à chaîne droite ou ramifiée ayant jusqu'à 8 atomes de carbone, qui est substitué le cas échéant par un radical hydroxy, phényle, halogéno ou cyano,
- un groupe aryle de 6 à 10 atomes de carbone, qui peut être substitué par un

radical halogéno, amino, hydroxy, nitro ou cyano, ou bien
- un groupe de formule $-NR^1R^2$ ou $-OR^4$ dans laquelle

$R^1$, $R^2$ et $R^4$ ont la définition indiquée ci-dessus,

n - est le nombre 0 ou 2,

$R^6$ - est un groupe alkyle à chaîne droite ou ramifiée ayant jusqu'à 10 atomes de carbone, qui peut être substitué par un radical halogéno, hydroxy, phényle ou un groupe de formule $-NR^1R^2$,
dans laquelle

$R^1$ et $R^2$ ont la définition indiquée ci-dessus,
- un groupe aryle de 6 à 10 atomes de carbone, qui peut être substitué par un radical halogéno, hydroxy, cyano, nitro ou amino, ou
- un groupe de formule $-NR^1R^2$, lorsque n est égal à 2,
- $R^1$ et $R^2$ ayant la définition indiquée ci-dessus,
ou bien

E - représente un groupe de formule $-NR^1R^2$ ou $-OR^4$ dans laquelle

$R^1$, $R^2$ et $R^4$ ont la définition indiquée ci-dessus,

Y et Z sont identiques ou différents et représentent
- un groupe de formule

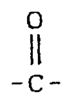

ou

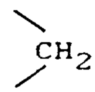

ou bien

Y - est un groupe thiocarbonyle,

X - est un groupe de formule $-CH_2-CH_2$ ou $-CH=CH-$,
et

R - est un groupe de formule

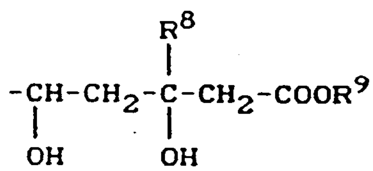

ou

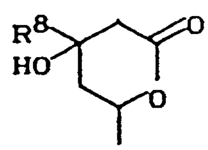

dans laquelle

$R^8$ - est de l'hydrogène ou un groupe alkyle à chaîne droite ou ramifiée ayant jusqu'à 10 atomes de carbone
et

$R^9$ - est de l'hydrogène ou un groupe alkyle à chaîne droite ou ramifiée ayant jusqu'à 10 atomes de carbone, qui peut être substitué par un radical phényle, ou bien
- un groupe aryle de 6 à 10 atomes de carbone ou un cation,

et leurs sels.

**2.** Pyrido(2,3-d)pyrimidines substituées suivant la revendication 1, de formules (Ia) et (Ib)

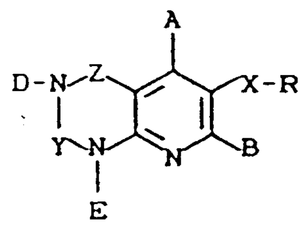

(Ia)

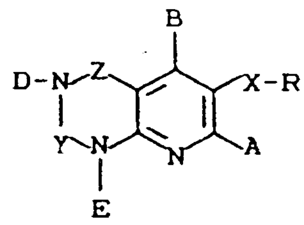

(Ib)

dans lesquelles

A - représente un groupe thiényle, furyle, pyridyle ou pyrimidyle qui est substitué le cas échéant jusqu'à 3 fois, identiques ou différentes, par du fluor, du chlore, du brome, un radical hydroxy, trifluorométhyle, alkyle, alkoxy ou alkoxycarbonyle à chaîne droite ou ramifiée ayant chacun jusqu'à 6 atomes de carbone, phényle, ou par un groupe de formule $-NR^1R^2$, dans laquelle

$R^1$ et $R^2$ sont identiques ou différents et représentent
- de l'hydrogène, un reste phényle, phénylsulfonyle, alkyle ou alkylsulfonyle à chaîne droite ou ramifiée ayant jusqu'à 6 atomes de carbone, benzyle ou benzylsulfonyle,
- un groupe de formule $-COR^3$,
dans laquelle

$R^3$ - est un radical alkyle ou alkoxy à chaîne droite ou ramifiée ayant jusqu'à 6 atomes de carbone, ou un radical phényle,
ou bien
- un groupe phényle ou naphtyle qui est substitué le cas échéant jusqu'à 4 fois identiques ou différentes par un reste alkyle, alkoxy ou alkoxycarbonyle à chaîne droite ou ramifiée ayant chacun jusqu'à 8 atomes de carbone, pouvant être substitués quant à eux par un radical trifluorométhyle, hydroxy, alkoxy ayant jusqu'à 4 atomes de carbone, phényle ou phénoxy, ou par un reste phényle, phénoxy, phénylthio, phénylsulfonyle, du fluor, du chlore, du brome, un reste nitro, cyano, trifluorométhyle, benzyloxy ou par un groupe de formule $-NR^1R^2$,
ou bien
- un groupe alkyle à chaîne droite ou ramifiée ayant jusqu'à 6 atomes de carbone,

B - est un groupe cyclopropyle, cyclobutyle, cyclopentyle ou cyclohexyle,
- un groupe trifluorométhyle ou un groupe alkyle à chaîne droite ou ramifiée ayant jusqu'à 10 atomes de carbone, qui est substitué le cas échéant par du fluor, du chlore, du brome, un radical hydroxy, cyano, azido, trifluorométhyle, méthylthio, méthylsulfonyle, alkoxy ayant jusqu'à 6 atomes de carbone ou par un radical phényle, phényloxy ou phénylthio, les restes phényle pouvant être substitués jusqu'à 2 fois identiques ou différentes par du fluor, du chlore, du brome, un radical cyano, trifluorométhyle, trifluorométhoxy, alkyle, alkoxy, alkylthio ou alkylsulfonyle à chaîne droite ou ramifiée ayant chacun jusqu'à 6 atomes de carbone, ou bien par un groupe de formule $-NR^1R^2$ ou $-COR^3$,
ou bien
- un groupe phényle qui est substitué le cas échéant par du fluor ou du chlore,

D - représente de l'hydrogène, un groupe hydroxy ou
- un groupe cyclopropyle, cyclobutyle, cyclopentyle ou cyclohexyle,
- un groupe alkyle linéaire ou ramifié ayant jusqu'à 10 atomes de carbone, qui est substitué le cas échéant par du fluor, du chlore, du brome, un radical hydroxy ou alkoxy ayant jusqu'à 6 atomes de carbone,

E - a la définition indiquée ci-dessus pour A et est identique à ce dernier ou en est différent, ou représente
- de l'hydrogène ou

- un groupe cyclopropyle, cyclobutyle, cyclopentyle ou cyclohexyle,
- un groupe alkyle à chaîne droite ou ramifiée ayant jusqu'à 10 atomes de carbone, qui est substitué le cas échéant par du fluor, du chlore, du brome, un radical alcényle à chaîne droite ou ramifiée ayant jusqu'à 6 atomes de carbone, phényle, pyrimidyle, pyrrolyle, pyrrolidinyle, furyle ou thiazolyle, ou par un groupe de formule $-NR^1R^2$, $-OR^4$, $-COR^5$ ou $-S(O)_n-R^6$,

où

$R^1$ et $R^2$ ont la définition indiquée ci-dessus,

$R^4$ - représente de l'hydrogène ou
- un groupe alkyle linéaire ou ramifié ayant jusqu'à 8 atomes de carbone, qui est substitué le cas échéant par un radical hydroxy, fluoro, chloro, bromo ou par un radical phényle, ou bien
- un groupe de formule $-COR^7$,

dans laquelle

$R^7$ - est un radical alkyle à chaîne droite ou ramifiée ayant jusqu'à 6 atomes de carbone, un radical phényle ou un groupe de formule $-NR^1R^2$,

dans laquelle

$R^1$ et $R^2$ ont la définition indiquée ci-dessus,

$R^5$ - représente l'hydrogène, un groupe alkyle à chaîne droite ou ramifiée ayant jusqu'à 6 atomes de carbone, qui est substitué le cas échéant par un radical hydroxy, phényle, fluoro, chloro, bromo ou cyano,
- un groupe phényle qui peut être substitué quant à lui par un radical fluoro, chloro, bromo, amino, hydroxy, nitro ou cyano, ou bien
- un groupe de formule $-NR^1R^2$ ou $-OR^4$, dans laquelle

$R^1$, $R^2$ et $R^4$ ont la définition indiquée ci-dessus,

n - est le nombre 0 ou 2,

$R^6$ - est un groupe alkyle à chaîne droite ou ramifiée ayant jusqu'à 8 atomes de carbone, ou bien
- un groupe phényle qui peut être substitué par un radical fluoro, chloro, bromo, hydroxy, cyano, nitro ou amino, ou bien
- un groupe de formule $-NR^1R^2$ lorsque n est le nombre 2,

où

$R^1$ et $R^2$ ont la définition indiquée ci-dessus,

ou bien

E - est un groupe de formule $-NR^1R^2$ ou $-OR^4$,

dans laquelle

$R^1$, $R^2$ et $R^4$ ont la définition indiquée ci-dessus,

Y et Z sont identiques ou différents et représentent
- un groupe de formule

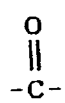

ou

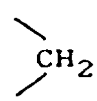

ou bien

Y - est un groupe thiocarbonyle,

X - est un groupe de formule $-CH_2-CH_2-$ ou $-CH=CH-$

et

R - est un groupe de formule

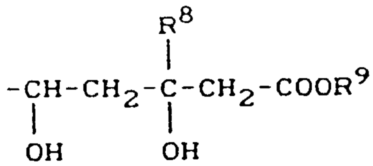

ou

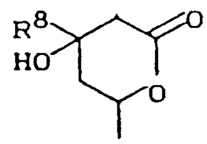

dans laquelle

$R^8$ - représente l'hydrogène ou un groupe alkyle à chaîne droite ou ramifiée ayant jusqu'à 8 atomes de carbone

et

$R^9$ - représente de l'hydrogène ou un groupe alkyle à chaîne droite ou ramifiée ayant jusqu'à 8 atomes de carbone ou le groupe benzyle, ou bien

- un groupe phényle ou un cation

et leurs sels.

**3.** Pyrido(2,3-d)pyrimidines substituées suivant les revendications 1 et 2,

dans lesquelles

A - est un groupe thiényle, furyle ou pyridyle qui est substitué le cas échéant jusqu'à 2 fois identiques ou différentes par du fluor, du chlore, du brome, un radical hydroxy, trifluorométhyle, méthyle, éthyle, méthoxy, éthoxy ou phényle ou par un groupe de formule $-NR^1R^2$,

dans laquelle

$R^1$ et $R^2$ sont identiques ou différents et représentent

- de l'hydrogène, un radical phényle, alkyle à chaîne droite ou ramifiée ayant jusqu'à 4 atomes de carbone,
- un groupe phényle qui est substitué le cas échéant jusqu'à 3 fois identiques ou différentes par un reste alkyle ou alkoxy linéaire ou ramifié ayant chacun jusqu'à 6 atomes de carbone, qui peuvent être substitués quant à eux par un radical trifluorométhyle, hydroxy, méthoxy, éthoxy, propoxy, phényle ou phénoxy, ou par un reste phényle, phénoxy, fluoro ou chloro,

ou bien

- un groupe alkyle à chaîne droite ou ramifiée ayant jusqu'à 4 atomes de carbone,

B - est un groupe cyclopropyle, cyclobutyle, cyclopentyle ou cyclohexyle,

- un groupe méthyle, éthyle, propyle, isopropyle, butyle, tertio-butyle ou trifluorométhyle,
- un groupe phényle qui est substitué le cas échéant par du fluor,

D - représente de l'hydrogène ou

- un groupe cyclopropyle, cyclopentyle ou cyclohexyle,
- un groupe méthyle, éthyle, propyle, isopropyle, butyle ou tertio-butyle,

E - a la définition indiquée ci-dessus pour A et est identique à ce dernier ou en est différent, ou représente

- de l'hydrogène ou
- un groupe cyclopropyle, cyclopentyle ou cyclohexyle,
- un groupe alkyle linéaire ou ramifié ayant jusqu'à 8 atomes de carbone, qui est substitué le cas échéant par du fluor, du chlore, un radical alcényle linéaire ou ramifié ayant jusqu'à 4 atomes de carbone, phényle ou furyle, ou bien

est substitué par un groupe de formule $-NR^1R^2$ ou $-OR^4$,

dans laquelle

$R^1$ et $R^2$ ont la définition indiquée ci-dessus,

$R^4$ - est de l'hydrogène ou

- un groupe alkyle linéaire ou ramifié ayant jusqu'à 6 atomes de carbone, qui est substitué le cas échéant par un radical hydroxy ou phényle,

ou bien

E - est un groupe de formule $-NR^1R^2$,

dans laquelle

$R^1$ et $R^2$ ont la définition indiquée ci-dessus,

Y et Z sont identiques ou différents et représentent
- un groupe de formule

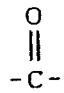

ou

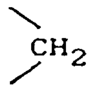

ou bien
Y - est un groupe thiocarbonyle,
X - est un groupe -CH = CH-
et
R - est un groupe de formule

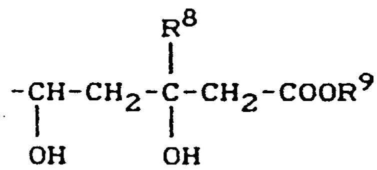

ou

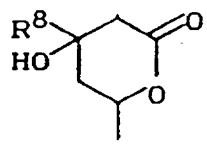

dans laquelle
$R^8$ - représente de l'hydrogène, un groupe méthyle, éthyle, propyle, isopropyle, butyle, isobutyle ou tertio-butyle
et
$R^9$ - représente de l'hydrogène, un groupe méthyle, éthyle, propyle, isopropyle, butyle, isobutyle, tertio-butyle ou benzyle, ou bien
- un ion sodium, potassium, calcium, magnésium ou ammonium,
et leurs sels.

**4.** Procédé de production de pyrido(2,3-d)pyrimidines substituées suivant les revendications 1 à 3, caractérisé en ce qu'on réduit des cétones de formule générale (VIII)

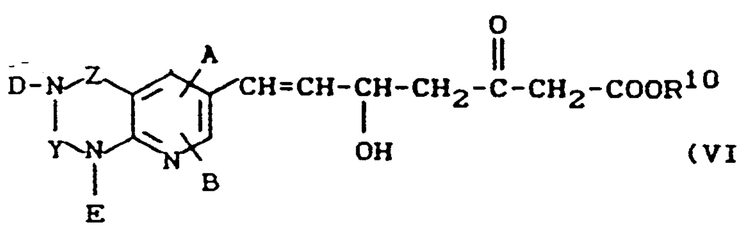

(VIII)

dans laquelle
A, B, D, E, Y et Z ont la définition indiquée ci-dessus, et
$R^{10}$ - est un groupe alkyle ayant jusqu'à 6 atomes de carbone,
dans le cas de la production des acides, on saponifie les esters,
dans le cas de la production des lactones, on cyclise les acides carboxyliques,
dans le cas de la production des sels, on saponifie ou bien les esters ou bien les lactones,
dans le cas de la production des composés éthyléniques (X = $-CH_2-CH_2-$), on hydrogène par des procédés classiques les composés éthéniques (X = -CH = CH-),
et on sépare éventuellement les isomères.

**5.** Pyrido(2,3-d)pyrimidines substituées suivant la revendication 1, destinées à combattre des maladies.

**6.** Médicament contenant au moins une pyrido(2,3-d)pyrimidine substituée suivant la revendication 1.

7. Procédé de préparation d'un médicament suivant la revendication 6, caractérisé en ce qu'on donne une forme qui convient pour l'administration à des substances suivant la revendication 1, le cas échéant à l'aide de substances auxiliaires et de supports.

8. Utilisation des pyrido(2,3-d)pyrimidines substituées suivant la revendication 1 pour la préparation de médicaments.

9. Utilisation des pyrido(2,3-d)pyrimidines substituées suivant la revendication 1 pour la préparation de médicaments destinés au traitement de l'hyperlipoproteinémie, de la lipoprotéinémie ou de l'artériosclérose.

**Revendication pour l'Etat contractant suivant : ES**

1. Procédé de production de pyrido(2,3-d)pyrimidines substituées de formule (I)

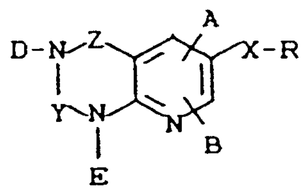

(I)

dans laquelle

A - représente un hétérocycle pentagonal à heptagonal qui peut contenir jusqu'à 4 hétéro-atomes de la série soufre, oxygène et azote et qui porte le cas échéant jusqu'à 3 substituants, identiques ou différents, halogéno, hydroxy, trifluorométhyle, alkyle, alkoxy ou alkoxycarbonyle à chaîne droite ou ramifiée ayant chacun jusqu'à 8 atomes de carbone, aryle de 6 à 10 atomes de carbone ou groupe de formule $-NR^1R^2$ dans laquelle

$R^1$ et $R^2$ sont identiques ou différents et représentent
- l'hydrogène, un reste aryle ou arylsulfonyle ayant 6 à 10 atomes de carbone, un reste alkyle ou alkylsulfonyle à chaîne droite ou ramifiée ayant jusqu'à 8 atomes de carbone, les restes mentionnés en dernier lieu étant substitués le cas échéant par un radical aryle ayant 6 à 10 atomes de carbone, ou bien
- un groupe de formule $-COR^3$,
dans laquelle

$R^3$ - est un reste alkyle ou alkoxy à chaîne droite ou ramifiée ayant jusqu'à 8 atomes de carbone ou un reste phényle,
- un groupe aryle de 6 à 10 atomes de carbone, qui est substitué, le cas échéant, jusqu'à 5 fois identiques ou différentes par un reste alkyle, alkylthio, alkylsulfonyle, alkoxy ou alkoxycarbonyle à chaîne droite ou ramifiée ayant chacun jusqu'à 10 atomes de carbone, ces restes pouvant être substitués quant à eux par un radical trifluorométhyle, hydroxy, alkoxy ayant jusqu'à 6 atomes de carbone, phényle ou phénoxy, ou par un reste aryle, aryloxy, arylthio ou arylsulfonyle ayant 6 à 10 atomes de carbone, ou par un reste halogéno, nitro, cyano, trifluorométhyle, benzyloxy ou un groupe de formule $-NR^1R^2$,
dans laquelle
- $R^1$ et $R^2$ ont la définition indiquée ci-dessus

B - représente un groupe cycloalkyle de 3 à 8 atomes de carbone
- un groupe trifluorométhyle ou un groupe alkyle à chaîne droite ou ramifiée ayant jusqu'à 12 atomes de carbone, qui est substitué le cas échéant par un halogène, un radical hydroxy, cyano, azido, trifluorométhyle, alkylthio, alkylsulfonyle ou alkoxy ayant chacun jusqu'à 8 atomes de carbone ou par un radical aryle, aryloxy ou arylthio de 6 à 10 atomes de carbone, les restes aryle pouvant être substitués le cas échéant jusqu'à 3 fois identiques ou différentes par un radical halogéno, cyano, trifluorométhyle, trifluorométhoxy, alkyle, alkoxy, alkylthio ou alkylsulfonyle à chaîne

droite ou ramifiée ayant chacun jusqu'à 8 atomes de carbone, ou par un groupe de formule $-NR^1R^2$ ou $-COR^3$,
dans lequel
$R^1$, $R^2$ et $R^3$ ont la définition indiquée ci-dessus,
- un groupe aryle ayant 6 à 10 atomes de carbone, qui est substitué le cas échéant par un radical halogéno, cyano, nitro, trifluorométhyle, un radical alkyle, alkoxy ou alkoxycarbonyle à chaîne droite ou ramifiée ayant chacun jusqu'à 8 atomes de carbone ou un radical amino,
D - représente l'hydrogène, un groupe hydroxy ou
- un groupe cycloalkyle ayant 3 à 8 atomes de carbone,
- un groupe alkyle linéaire ou ramifié ayant dans chaque cas jusqu'à 12 atomes de carbone, qui est substitué le cas échéant par un halogène, un radical hydroxy ou alkoxy ayant jusqu'à 8 atomes de carbone,
E - a la définition indiquée ci-dessus pour A et est identique à ce dernier ou en est différent, ou bien
- de l'hydrogène, ou bien
- un groupe cycloalkyle de 3 à 8 atomes de carbone,
- un groupe alkyle linéaire ou ramifié ayant jusqu'à 12 atomes de carbone, qui est substitué le cas échéant par un halogène, un radical alcényle à chaîne droite ou ramifiée ayant jusqu'à 8 atomes de carbone, aryle de 6 à 10 atomes de carbone, par un hétérocycle pentagonal à heptagonal ayant jusqu'à 4 hétéro-atomes de la série azote, oxygène ou soufre ou par un groupe de formule $-NR^1R^2$, $-OR^4$, $-COR^5$ ou $S(O)_n-R^6$,
où
$R^1$ et $R^2$ ont la définition indiquée ci-dessus,
$R^4$ - est de l'hydrogène ou
- un groupe alkyle à chaîne droite ou ramifiée ayant jusqu'à 10 atomes de carbone, qui est substitué le cas échéant par un radical hydroxy, trialkylsilyle ayant jusqu'à 10 atomes de carbone dans la partie alkyle, halogéno ou aryle ayant 6 à 10 atomes de carbone,
- un groupe trialkylsilyle ayant jusqu'à 10 atomes de carbone ou cycloalkyle ayant 3 à 8 atomes de carbone ou aryle ayant 6 à 10 atomes de carbone, qui peut être substitué quant à lui par un halogène, un radical cyano, nitro ou amino, ou bien
- un groupe de formule $-COR^7$,
dans laquelle
$R^7$ - est un radical alkyle à chaîne droite ou ramifiée ayant jusqu'à 8 atomes de carbone, aryle de 6 à 10 atomes de carbone ou le groupe $-NR^1R^2$,
où
$R^1$ et $R^2$ ont la définition indiquée ci-dessus,
$R^5$ - représente de l'hydrogène ou un groupe alkyle à chaîne droite ou ramifiée ayant jusqu'à 8 atomes de carbone, qui est substitué le cas échéant par un radical hydroxy, phényle, halogéno ou cyano,
- un groupe aryle de 6 à 10 atomes de carbone, qui peut être substitué par un radical halogéno, amino, hydroxy, nitro ou cyano, ou bien
- un groupe de formule $-NR^1R^2$ ou $-OR^4$ dans laquelle
$R^1$, $R^2$ et $R^4$ ont la définition indiquée ci-dessus,
n - est le nombre 0 ou 2,
$R^6$ - est un groupe alkyle à chaîne droite ou ramifiée ayant jusqu'à 10 atomes de carbone, qui peut être substitué par un radical halogéno, hydroxy, phényle ou un groupe de formule $-NR^1R^2$,
dans laquelle
$R^1$ et $R^2$ ont la définition indiquée ci-dessus,
- un groupe aryle de 6 à 10 atomes de carbone, qui peut être substitué par un radical halogéno, hydroxy, cyano, nitro ou amino, ou
- un groupe de formule $-NR^1R^2$, lorsque n est égal à 2,
- $R^1$ et $R^2$ ayant la définition indiquée ci-dessus,
ou bien
E - représente un groupe de formule $-NR^1R^2$ ou $-OR^4$ dans laquelle

| | |
|---|---|
| $R^1$, $R^2$ et $R^4$ | ont la définition indiquée ci-dessus, |
| Y et Z | sont identiques ou différents et représentent |
| | - un groupe de formule |

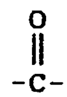 ou 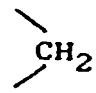

| | |
|---|---|
| | ou bien |
| Y | - est un groupe thiocarbonyle, |
| X | - est un groupe de formule $-CH_2-CH_2$ ou $-CH=CH-$, |
| | et |
| R | - est un groupe de formule |

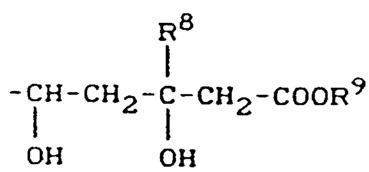 ou 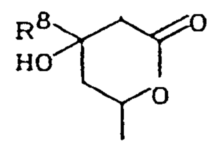

| | |
|---|---|
| | dans laquelle |
| $R^8$ | - est de l'hydrogène ou un groupe alkyle à chaîne droite ou ramifiée ayant jusqu'à 10 atomes de carbone |
| | et |
| $R^9$ | - est de l'hydrogène ou un groupe alkyle à chaîne droite ou ramifiée ayant jusqu'à 10 atomes de carbone, qui peut être substitué par un radical phényle, ou bien |
| | - un groupe aryle de 6 à 10 atomes de carbone ou un cation, |

et de leurs sels,
caractérise en ce qu'on réduit des cétones de formule générale (VIII)

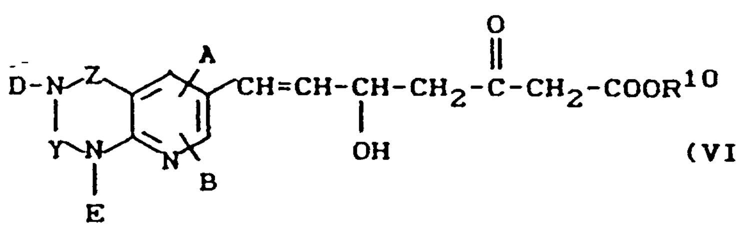

(VIII)

dans laquelle
A, B, D, E, Y et Z ont la définition indiquée ci-dessus, et
$R^{10}$ - est un groupe alkyle ayant jusqu'à 6 atomes de carbone,
dans le cas de la production des acides, on saponifie les esters,

dans le cas de la production des lactones, on cyclise les acides carboxyliques,

dans le cas de la production des sels, on saponifie ou bien les esters ou bien les lactones,

dans le cas de la production des composés éthyléniques ($X = -CH_2-CH_2-$), on hydrogène les composés éthéniques ($X = -CH=CH-$) par des procédés classiques,

et on sépare éventuellement les isomères.